(19) Europäisches Patentamt / European Patent Office / Office européen des brevets

(11) EP 2 515 837 B1

(12) # FASCICULE DE BREVET EUROPEEN

(45) Date de publication et mention de la délivrance du brevet:
**07.11.2018 Bulletin 2018/45**

(21) Numéro de dépôt: **10810856.4**

(22) Date de dépôt: **21.12.2010**

(51) Int Cl.:
***A61K 8/34*** (2006.01) ***A61K 8/49*** (2006.01)
***A61K 8/97*** (2017.01) ***A61K 8/19*** (2006.01)
***A61K 8/25*** (2006.01) ***A61K 8/29*** (2006.01)
***A61K 8/28*** (2006.01) ***A61K 8/26*** (2006.01)
***A61Q 5/10*** (2006.01)

(86) Numéro de dépôt international:
**PCT/FR2010/052841**

(87) Numéro de publication internationale:
**WO 2011/086282 (21.07.2011 Gazette 2011/29)**

(54) **COMPOSITION COMPRENANT AU MOINS UN DERIVE D'ORTHODIPHENOL UN DERIVE METALLIQUE PARTICULIER ET UN AGENT ALCALINISANT, POUR COLORER LES FIBRES KERATINIQUES**

ZUSAMMENSETZUNG ZUM FÄRBEN VON KERATINFASERN MIT MINDESTENS EINEM ORTHODIPHENOLDERIVAT, EINEM BESTIMMTEN METALLDERIVAT UND EINEM ALKALINISIERUNGSMITTEL

COMPOSITION FOR DYEING KERATIN FIBERS, INCLUDING AT LEAST ONE ORTHO-DIPHENOL DERIVATIVE, ONE PARTICULAR METAL DERIVATIVE, AND ONE ALKALINIZING AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.02.2010 US 300117 P**
**23.12.2009 FR 0959515**

(43) Date de publication de la demande:
**31.10.2012 Bulletin 2012/44**

(73) Titulaire: **L'Oréal**
**75008 Paris (FR)**

(72) Inventeurs:
- **RONDOT, Christophe**
  **F-77290 Mitry-Mory (FR)**
- **SABELLE, Stéphane**
  **F-75005 Paris (FR)**
- **CAVEZZA, Alexandre**
  **F-93320 Les Pavillons sous Bois (FR)**
- **MANFRE, Franco**
  **F-94170 Le Perreux sur Marne (FR)**

(74) Mandataire: **Wattremez, Catherine**
**L'Oréal**
**Service DIPI**
**9 Rue Pierre Dreyfus**
**92110 Clichy (FR)**

(56) Documents cités:
**EP-A1- 0 348 280** **EP-A1- 0 664 114**
**EP-A2- 0 335 477** **DE-A1- 4 208 297**
**DE-A1-102004 049 093** **DE-A1-102005 062 830**
**FR-A1- 2 598 318** **FR-A1- 2 700 266**
**FR-A1- 2 814 943** **GB-A- 2 132 642**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 515 837 B1

## Description

L'invention a pour objet une composition comprenant **a)** au moins un dérivé d'orthodiphénol différent des dérivés à motif indoles , **b)** au moins un dérivé métallique particulier, **c)** au moins un agent alcalinisant choisi parmi les alcanolamines et les (bi)carbonates; un procédé de coloration de fibres kératiniques par traitement desdites fibres mettant en oeuvre les ingrédients **a)**, **b)** et **c)** et leur utilisation pour colorer les fibres kératiniques.

Il est connu d'obtenir des colorations dites « permanentes » avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques. Ce procédé de coloration d'oxydation consiste à appliquer sur les fibres kératiniques, des bases ou un mélange de bases et de coupleurs avec du peroxyde d'hydrogène ($H_2O_2$ ou eau oxygénée) à titre d'agent oxydant, à laisser diffuser, puis à rincer les fibres. Les colorations qui en résultent sont permanentes, puissantes, et résistantes aux agents extérieurs, notamment à la lumière, aux intempéries, aux lavages, à la transpiration et aux frottements,

Cependant les colorations capillaires commerciales qui les contiennent peuvent présenter des inconvénients comme le tachage, les problèmes d'odeur, de confort et de dégradation des fibres kératiniques. C'est particulièrement le cas avec les colorations d'oxydation.

Dans le domaine de la coloration, il est également connu de colorer des matières kératiniques telles que les cheveux ou la peau à partir de composés orthodiphénols en présence d'un sel métallique notamment de Mn et/ou de Zn. En particulier les demandes de brevets FR 2 814 943, FR 2 814 945, FR 2 814 946, FR 2 814 947, proposent des compositions pour la coloration de la peau ou des fibres kératiniques, comprenant un précurseur de colorant qui contient au moins un orthodiphénol, des sels et oxydes de Mn et/ou Zn, des alcalins de type hydrogénocarbonate dans un ratio particulier Mn, Zn / hydrogénocarbonate et éventuellement une enzyme. Selon ces documents il est possible d'obtenir des colorations des matières kératiniques avec l'oxygène de l'air. Cependant les colorations obtenues sont insuffisamment intenses notamment dans le cas des fibres capillaires.

Il est également connu d'utiliser des métaux pour améliorer la coloration des cheveux dans des quantités de métaux du même ordre de grandeur que celle des colorants en utilisant un procédé de mordençage. Néanmoins ce procédé présente en générale l'inconvénient de ne pas toujours respecter la cosmétique de la fibre kératinique.

En outre il est connu d'utiliser un orthodiphénol tel que la 4-méthycathéchol et un sel de cuivre de type acétate de cuivre pour colorer les cheveux avec du persulfate (EP 0 664 114). Néanmoins les colorations obtenues ne sont pas tout à fait satisfaisantes notamment en termes d'homogénéité de la coloration, de la chromaticité et/ou de la montée de la couleur.

Il existe donc un réel besoin de développer des procédés de colorations qui permettent d'obtenir des colorations puissantes à partir d'orthodiphénols, notamment à partir d'extrait naturel riche en orthodiphénols, tout en limitant la décoloration des fibres kératiniques. En particulier, il existe un besoin d'obtenir des colorations moins agressives pour les cheveux et dans un même temps qui résistent aux agents extérieurs (lumière, intempéries, shampooings), qui soient tenaces et/ou homogènes, faible sélectivité de la coloration entre la racine et la pointe, tout en restant puissantes et/ou chromatiques. Il est également recherché que les colorations obtenues restent naturelles, notamment dans les reflets dorés.

Ce but est atteint par la présente invention qui a pour objet un procédé de coloration des fibres kératiniques, dans lequel lesdites fibres sont traitées par :

- **a)** un ou plusieurs dérivé(s) d'orthodiphénol(s) différent(s) des dérivés à motif indoles,
- **b)** un ou plusieurs dérivé(s) métallique(s) choisi(s) parmi les sels métalliques, complexes métalliques, oxydes métalliques, oxanions métalliques, leurs formes supportées, leurs hydrates et leur mélanges pour lesquels le ou les métal(aux) est (sont) choisi(s) parmi :
    - **i)** l'or (Au),
    - **ii)** le molybdène (Mo),
    - **iii)** les oxydes d'argent (Ag) I et II, les sels d'Ag I et II choisis parmi les halogénures d'argent, le sulfate d'Ag, **$[R^1\text{-}C(O)O]_nAg$** avec n= 1 ou 2, $R^1$ représentant un groupe $(C_1\text{-}C_6)$alkyle tel que l'acétate d'Ag, le lactate d'Ag, les complexes d'argent tels que les métalloporphyrines d'Ag I, les phtalocyanines d'Ag I ou les chlorophyllines Ag I,
    - **iv)** le tungstène (W),

**v)** le vanadium (V),
**vi)** le Ruthénium (Ru),
**vii)** les métalloporphyrines de Mg II, les phtalocyanines de Mg II, les chlorophyllines de Mg II, les chlorophylles de Mg II,
**ix)** le rhénium (Re),
**x) le** titane (Ti),
**xi)** le silicium (Si),
**xii)** les oxydes d'étain,
**xiii)** le zirconium (Zr),
**xiv)** le niobium (Nb),
**xv)** l'indium (In),
**xvi)** le sélénium (Se), et

**c)** un ou plusieurs agent(s) alcalinisant(s) choisi(s) parmi les alcanolamines et les (bi)carbonates;

étant entendu que le pH à la fin du procédé est alcalin, soit supérieur à 7 et de préférence compris entre 8 et 12. Particulièrement compris entre 8 et 10,5.

Un autre objet de l'invention concerne une composition cosmétique pour la coloration des fibres kératinique comprenant :

- un ou plusieurs ingrédient(s) **a)** tel(s) que défini(s) précédemment ;
- un ou plusieurs ingrédient(s) **b)** tel(s) que défini(s) précédemment et
- un ou plusieurs ingrédient(s) **c)** tel(s) que défini(s) précédemment ;

ou une composition cosmétique telle qu'utilisée dans le procédé de coloration qui comprend :

- un ou plusieurs ingrédient(s) **a)** tel(s) que défini(s) précédemment ;
- un ou plusieurs dérivé(s) d'or tel que défini précédemment

étant entendu que le pH des compositions est supérieur à 7 et de préférence compris entre 8 et 12. Particulièrement compris entre 8 et 10,5.

Un autre objet de l'invention concerne un dispositif à plusieurs compartiments comprenant les ingrédients **a)** à **c)** tels que définis précédemment.

Un autre objet de l'invention concerne l'utilisation pour la coloration des fibres kératiniques tels que les cheveux de l'association de **a)** à **c)** tels que définis précédemment ou de l'association **a)** tel que défini précédemment et **b)** un ou plusieurs dérivé(s) d'or.

Le procédé selon l'invention présente l'avantage de colorer les fibres kératiniques humaines, avec des résultats de colorations puissantes, chromatiques, résistantes aux lavages, la transpiration, le sébum et à la lumière et de plus durables sans altération desdites fibres. De plus les colorations obtenues à partir du procédé donnent des couleurs homogènes de la racine à la pointe d'une fibre (faible sélectivité de coloration).

*a) dérivé d'orthodiphénol :*

Un mode particulier de l'invention concerne des dérivés d'orthodiphénols ou mélanges de composés comprenant au moins un cycle aromatique, de préférence un cycle benzénique, comportant au moins deux groupes hydroxy (OH) portés par deux atomes de carbone adjacents du cycle aromatique qui ne sont pas des dérivés autoxydables à motif indole. Plus particulièrement ils sont différents du 5,6-dihydroxyindole.

Le cycle aromatique peut être plus particulièrement un cycle aryle condensé ou hétéroaromatique condensé i.e. contenant éventuellement un ou plusieurs hétéroatomes, tel que le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatique comportant au moins deux groupes hydroxy portés par deux atomes de carbone adjacents du cycle aromatique. Préférentiellement le cycle aromatique des dérivés orthodiphénols selon l'invention est un cycle benzénique.

Par cycle condensé on entend qu'au moins deux cycles saturés ou insaturés, hétérocycliques ou non, présentent une liaison commune i.e. qu'au moins un cycle soit accolé à un autre cycle.

Les orthodiphénols selon l'invention peuvent être salifiés ou non. Ils peuvent également se trouver sous forme d'aglygone (sans sucre liés) ou sous forme de composés glycosylés.

Plus particulièrement le dérivé d'orthodiphénol a) représente un composé de formule (I), ou l'un de ces oligomères, sous forme salifiée ou non :

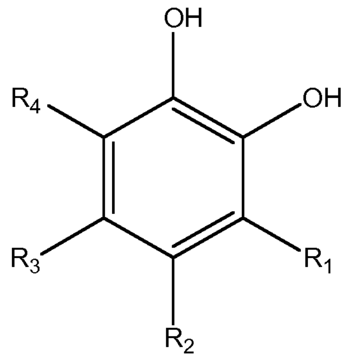

(I)

formule (I) dans laquelle les substituants :

- $R_1$ à $R_4$, identiques ou différents, représentent :
  - un atome d'hydrogène,
  - un atome halogène,
  - un radical hydroxy,
  - un radical carboxyle,
  - un radical carboxylate d'alkyle ou alcoxycarbonyle,
  - un radical amino éventuellement substitué,
  - un radical alkyle linéaire ou ramifié éventuellement substitué,
  - un radical alcényle linéaire ou ramifié éventuellement substitué,
  - un radical cycloalkyle éventuellement substitué,
  - un radical alcoxy,
  - un radical alcoxyalkyle,
  - un radical alcoxyaryle, le groupe aryle pouvant être éventuellement substitué,
  - un radical aryle,
  - un radical aryle substitué,
  - un radical hétérocyclique, saturé ou non, porteur ou non d'une charge cationique ou anionique, éventuellement substitué et/ou éventuellement condensé avec un cycle aromatique de préférence benzénique, ledit cycle aromatique étant éventuellement substitué particulièrement par un ou plusieurs groupements hydroxy ou glycosyloxy,
  - un radical contenant un ou plusieurs atomes de silicium,

- où deux des substituants portés par deux atome de carbone adjacents $R_1$-$R_2$, $R_2$-$R_3$ ou $R_3$- $R_4$ forment conjointement avec les atomes de carbone les portant un cycle saturé ou insaturé, aromatique ou non, contenant éventuellement un ou plusieurs hétéroatomes et éventuellement condensé avec un ou plusieurs cycles saturés ou insaturés contenant éventuellement un ou plusieurs hétéroatomes. Particulièrement $R_1$ à $R_4$ forment conjointement de un à quatre cycles.

Un mode de réalisation particulier de l'invention concerne des dérivés d'orthodiphénols de formule (I) dont deux substituants adjacents $R_1$ - $R_2$, $R_2$- $R_3$ ou $R_3$- $R_4$ ne peuvent former avec les atomes de carbone qui les portent un radical pyrrolyle. Plus particulièrement $R_2$ et $R_3$ ne peuvent former un radical pyrrolyle condensé au cycle benzénique portant les deux hydroxy.

Les cycles saturés ou insaturés, éventuellement condensés, peuvent être aussi éventuellement substitués.

Les radicaux alkyles sont des radicaux hydrocarbonés, saturés, linéaires ou ramifiés, généralement en $C_1$-$C_{20}$, particulièrement en $C_1$-$C_{10}$, de préférence les radicaux alkyles en $C_1$-$C_6$, tels que méthyle, éthyle, propyle, butyle, pentyle et hexyle.

Les radicaux alcényles sont des radicaux hydrocarbonés, linéaires ou ramifiés, insaturés en $C_2$-$C_{20}$; de préférence comprenant au moins une double liaison tels que éthylène, propylène, butylène, pentylène, méthyl-2-propylène et décylène.

Les radicaux aryles sont des radicaux carbonés mono ou polycycliques, condensés ou non, comprenant préférentiellement de 6 à 30 atomes de carbone et dont au moins un cycle est aromatique ; préférentiellement choisis parmi le radical aryle est un phényle, biphényle, naphtyle, indényle, anthracényle, et tétrahydronaphtyle.

Les radicaux alcoxy sont des radicaux alkyle-oxy avec alkyle tel que défini précédemment, de préférence en $C_1$-$C_{10}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxy, amine et cétone.

Les radicaux alkyles ou alcényles lorsqu'ils sont éventuellement substitués, peuvent être substitués par au moins un substituant porté par au moins un atome de carbone, choisi parmi :

- un atome d'halogène;
- un groupement hydroxy ;
- un radical alkoxy en $C_1$-$C_2$ ;
- un radical alcoxycarbonyle en $C_1$-$C_{10}$;
- un radical (poly)-hydroxyalkoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

    * un groupement hydroxy,
    * un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
    * un groupement ammonium quaternaire $-N^+R'R''R'''$, $M^-$ pour lequel R', R'', R''', identiques ou différents représentent un atome d'hydrogène, ou un groupement alkyle en $C_1$-$C_4$; et $M^-$ représente le contre-ion de l'acide organique, minéral ou de l'halogénure correspondant,
    * ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement nitro ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Les radicaux aryles ou hétérocycliques ou la partie aryle ou hétérocycliques des radicaux lorsqu'ils sont éventuellement substitués peuvent être substitués par au moins un substituant porté par au moins un atome de carbone, choisi parmi :

- un radical alkyle en $C_1$-$C_{10}$, de préférence en $C_1$-$C_8$, éventuellement substitué par un ou plusieurs radicaux choisis parmi les radicaux hydroxyle, alkoxy en $C_1$-$C_2$, (poly)-hydroxyalkoxy en $C_2$-$C_4$, acylamino, amino substitué par deux

radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;

- un atome d'halogène ;
- un groupement hydroxy ;
- un radical alkoxy en $C_1$-$C_2$ ;
- un radical alcoxycarbonyle en $C_1$-$C_{10}$;
- un radical (poly)-hydroxyalkoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  * un groupement hydroxy,
  * un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
  * un groupement ammonium quaternaire $-N^+R'R''R'''$, $M^-$ pour lequel R', R", R'", identiques ou différents représentent un atome d'hydrogène, ou un groupement alkyle en $C_1$-$C_4$; et $M^-$ représente le contre-ion de l'acide organique, minéral ou de l'halogénure correspondant,
  * ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino ($R'SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué ou non) ;
- un groupement cyano ;
- un groupement nitro ;
- un groupement polyhalogénoalkyle, préférentiellement le trifluorométhyle ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Par radical glycosyle on entend un radical issu d'un mono ou polysacharride.

Les radicaux contenant un ou plusieurs atomes de silicium sont de préférence des radicaux poly-diméthylsiloxane, poly-diphénylsiloxane, poly-diméthylphénylsiloxane, stéaroxy-diméthicone.

Les radicaux hétérocycliques sont en général des radicaux comprenant dans au moins un cycle un ou plusieurs hétéroatomes choisis parmi O, N et S, de préférence O ou N, éventuellement substitués par notamment un ou plusieurs groupes alkyles, alcoxy, acide carboxylique, hydroxy, amine ou cétone. Ces cycles peuvent contenir un ou plusieurs groupements oxo sur les atomes de carbone de l'hétérocycle.

Parmi les radicaux hétérocycliques utilisables, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les orthodiphénols utiles dans le procédé de l'invention peuvent être naturels ou synthétiques. Parmi les orthodiphénols naturels on inclut les composés qui peuvent être présents dans la nature et qui sont reproduits par (hémi)synthèse chimique.

Les sels des orthodiphénols de l'invention peuvent être des sels d'acides ou de bases. Les acides peuvent être minéraux ou organiques. De préférence l'acide est l'acide chlorhydrique qui conduit aux chlorures.

Les bases peuvent être minérales ou organiques. Particulièrement les bases sont des hydroxydes alcalins tels que la soude qui conduit à des sels de sodium.

Selon un mode de réalisation particulier de l'invention, la composition comprend comme ingrédient i) un ou plusieurs dérivé(s) d'orthodiphénol(s) synthétique(s) qui n'existe pas dans la nature.

Selon un autre mode de réalisation préféré de l'invention, le procédé de coloration des fibres kératiniques met en oeuvre comme ingrédient a) un ou plusieurs dérivé(s) d'orthodiphénol(s) naturel(s).

Plus particulièrement, les orthodiphénols utilisables dans le procédé de l'invention selon i) sont en particulier :

- les flavanols comme la catéchine et le gallate d'épichatéchine,
- les flavonols comme la quercétine,
- les anthocyanidines comme la cyanidine, la delphinidine, la pétunidine,
- les anthocyanines ou les anthocyanes comme la myrtilline ,
- les orthohydroxybenzoates, par exemple les sels d'acide gallique,
- les flavones comme la lutéoline,
- les hydroxystilbènes, par exemple le tétrahydroxy-3,3',4,5'-stilbène, éventuellement oxylés (par exemple glucosylés),
- la 3,4-dihydroxyphénylalanine et ses dérivés,
- la 2,3-dihydroxyphénylalanine et ses dérivés,
- la 4,5-dihydroxyphénylalanine et ses dérivés,
- les dihydroxycinnamates tels que l'acide caféique et l'acide chlorogénique,
- les orthopolyhydroxycoumarines,
- les orthopolyhydroxyisocoumarines,
- les orthopolyhydroxycoumarones,
- les orthopolyhydroxyisocoumarones,
- les orthopolyhydroxychalcones,
- les orthopolyhydroxychromones,
- les orthopolyhydroxyquinones,
- les orthopolyhydroxyxanthones,
- le 1,2 dihydroxybenzène et ses dérivés,
- le 1,2,4 trihydroxybenzène et ses dérivés,
- le 1,2,3 trihydroxybenzène et ses dérivés,
- le 2,4,5 trihydroxytoluène et ses dérivés,
- les proanthocyanidines et notamment les proanthocyanidines A1, A2, B1, B2, B3 et C1,
- les proanthocyanines,
- l'acide tannique,
- l'acide ellagique,
- et les mélanges des composés précédents.

Lorsque les précurseurs de coloration présentent des formes D et L, les deux formes peuvent être utilisées dans les compositions selon l'invention, ainsi que les racémiques.

Selon un mode de réalisation les orthodiphénols naturels sont issus d'extraits d'animaux, de bactéries, de champignons, d'algues, de plantes utilisés dans leur totalité ou partiellement. En particulier concernant les plantes les extraits sont issus de plante ou de partie de plantes telles que les fruits dont les agrumes, les légumes, les arbres, les arbustes. On peut aussi utiliser des mélanges de ces extraits, riches en othodiphénols tels que définis précédemment.

De préférence le ou les orthodiphénols naturels de l'invention sont issus d'extraits de plantes ou de parties de plantes

Au sens de l'invention on assimilera ces dits extraits dans leur ensemble en tant que composé a).

Les extraits sont obtenus par extraction de diverses parties de plantes telles que par exemple la racine, le bois, l'écorce, la feuille, la fleur, le fruit, le pépin, la gousse, la pelure.

Parmi les extraits de plantes, on peut citer les extraits de feuilles de thé, de rose.

Parmi les extraits de fruits, on peut citer les extraits de pomme, de raisin (en particulier de pépins de raisin), ou les extraits de fèves et/ou cabosses de cacaoyer.

Parmi les extraits de légumes, on peut citer les extraits de pomme de terre, de pelures d'oignon.

Parmi les extraits de bois d'arbres, on peut citer les extraits d'écorce de pin, les extraits de bois de campêche.

On peut également utiliser des mélanges d'extraits végétaux.

Selon un mode de réalisation particulier de l'invention le ou les dérivés d'orthodiphénols sont des extraits naturels, riches en orthodiphénols. Selon un mode préféré, le ou les dérivés d'orthodiphénols sont uniquement des extraits naturels.

Les extraits naturels selon l'invention peuvent se présenter sous forme de poudres ou de liquides. De préférence les extraits de l'invention se présentent sous forme des poudres.

Selon l'invention, le ou les dérivé(s) d'orthodiphénol(s) synthétique(s), naturel(s), et/ou le ou les extrait(s) naturel(s) utilisé(s) comme ingrédient i) dans une ou plusieurs composition(s) utile(s) dans le procédé selon l'invention représente(nt) de préférence, de 0.001% à 20% en poids du poids total de la ou des compositions contenant le ou les orthodiphénols ou le ou les extraits.

En ce qui concerne les orthodiphénols purs, la teneur dans la ou les compositions les contenant est comprise de préférence entre 0,001 et 5 % en poids de chacune de ces compositions.

En ce qui concerne les extraits, la teneur dans la ou les compositions contenant les extraits tels quels est comprise de préférence entre 0,5 et 20 % en poids de chacune de ces compositions.

*b) dérivé(s) métallique(s)*

Le procédé de l'invention utilise un ou plusieurs dérivé(s) métallique(s) b) qui catalysent l'oxydation par l'oxygène de l'air. Ces dérivés sont choisis parmi les sels métalliques, complexes métalliques, oxydes métalliques, oxanions métalliques, les hydrates et leurs formes supportées pour lesquels les métaux sont i) Au ; ii) Mo ; iii) Ag ; iv) W ; v) V ; vi) Ru ; vii) Mg ; ix) Re ; x) Ti ; xi) Si ; xii) Sn ; xiii) Zr ; xiv) Nb ; xv) In ; xvi) Se ; xvii) Al tels que définis précédemment, particulièrement ces dérivés ne pouvant représenter ni l'halogénure de Sn, ni l'halogénure d'Al,; ni le nitrate d'Ag..

Par « *sel métallique* » on entend un composé différent des alliages i.e. le sel est constitué d'un métal combiné avec certains éléments non métalliques.

La formation des sels métalliques découle d'une attaque oxydante. Le métal est oxydé en une espèce cationique et se combine alors avec une espèce anionique pour donner un sel. Cette formation a lieu en appliquant les principes et réaction d'oxydoréduction (réaction chimique au cours de laquelle se produit un transfert d'électrons dans laquelle l'atome qui capte les électrons est appelé « oxydant » ; celui qui les cède, « réducteur ») ; ou par réactions d'échange chimique entre un sel et un autre sel ou un acide, en présence ou pas d'oxygène de l'air. Ces réactions sont connues par l'homme du métier.

Préférentiellement les sels selon l'invention sont solubles dans l'eau à une proportion d'au moins 0,0001 g/L.

Les sels de métaux selon l'invention peuvent être introduits sous forme solide dans les compositions ou bien provenir d'une eau naturelle, minérale ou thermale, riche en ces ions ou encore d'eau de mer (mer morte notamment). Ils peuvent aussi provenir de composés minéraux comme les extrait végétaux les contenant (cf. par exemple brevet le document FR 2 814 943).

Par « *complexe métallique* » ou « *coordination compounds* » on entend des systèmes dans lesquels l'ion métallique, l'atome central, est lié chimiquement à un ou plusieurs donneurs d'électron (ligands). Un ligand comprenant différents groupes coordinants (capable de coordination avec un métal) donne des composés métalliques répondant à des principes de sphère de coordination à nombre d'électrons déterminés (complexes internes ou chelates) - voir Ullmann's Encyclopedia of Industrial Chemistry, « Metal complex dyes », 2005, p. 1-42 - Plus particulièrement par complexe métallique on entend :

i) des colorants métalliques ou « *metal-complex dyes* » qui sont des colorants complexés issus de colorants azoïques, azométhiniques, hydrazono, formazans (libres, bidendates, tridendates, quadridendates) tels que ceux décrits dans Ullmann's Encyclopedia of Industrial Chemistry, « Metal complex dyes », 2005, p. 1-42, qui comprennent préférentiellement du Cu et Mg ;

ii) les composés de type « *aza[18]annulènes* » ou également appelées *« (métallo)porphyrines* » et « *phtalocyanines* » qui contiennent 4 et 8 atomes d'azote respectivement compris dans le périmètre du macrocycle - voir l'ouvrage « Color Chemistry, » H. Zollinger, 3th Ed., Wiley-VCH, 2003, chap.5. Aza[18]annulenes, p. 123-160. L'ion métallique se trouve alors au centre dudit macrocycle lié par coordination à 2 atomes d'hydrogènes aux atomes d'azote de pyrroles, le métal pouvant également être stabilisé par un ou plusieurs ligands bidendates ou non; l'ion métallique étant préférentiellement du $Mg^{2+}$ ou $Cu^{2+}$;

particulièrement le complexe métallique est :

- une « *métalloporphyrine* » constitué d'un squelette à 4 groupes pyrroles qui sont reliés à leur position $\alpha,\alpha'$ par 4 groupes méthines et contiennent 16 atomes hybridés $sp^2$, complexant un métal tel que Cu, Mg.ou
- une « *chlorine* » (correspondant à une porphyrine dont une double liaison C=C externe d'un groupe pyrrole a

été réduite) complexé à un métal préférentiellement $Mg^{2+}$ tel que le chromophore de la chlorophylle : la chlorophylline ;

iii) les « *phtalocyanines* » analogues tétraaza de tétrabenzoporphyrines, telles que le Monastral Fast Blue B (C.I. Pigment Blue 15); Monastral Fast Blue G (C.I. Pigment Blue 16) (voir « Color Chemistry, » H. Zollinger, 3th Ed., Wiley-VCH, 2003, chap.5. Aza[18]annulenes, p. 140) ; les dérivés sulfonylés Sirius light Turquoise Blue G (C.I. Direct Blue 86, acide phtalocyanine tétrasulfonique de Cuivre) et les « phtalocyanines » telles décrites dans Ullmann's Encyclopedia of Industrial Chemistry, « Phtalocyanines », 2005, p. 1-34 qui comprennent du Cu et Si.

Par « *oxyde(s) métallique(s)* » on entend les composés de formule générique $\mathbf{A_xO_y}$ avec **A** représentant un élément métallique et $1 \leq x \leq 4$ et $1 \leq y \leq 12$

Par « *oxanion(s) métallique(s)* » on entend les composés de formule générique $\mathbf{Z_zA_xO_y}$ avec **A** représentant un élément métallique, **Z** représentant un métal alcalin tel que Li, Na, K ou un atome d'hydrogène ou un ion ammonium, et $1 \leq z \leq 6$, $1 \leq x \leq 4$ et $1 \leq y \leq 12$

Par « *forme(s) supportée(s)* » on entend les formes où le dérivé métallique b) est imprégné sur un matériau appelé « support ». Les supports éventuels de ces dérivés métalliques peuvent être choisis parmi le charbon, la silice, l'alumine, des polymères éventuellement chargés comprenant des contre-anions ou contre-cations (contre-cation ou contre-anion de l'espèce métallique). A titre d'exemple les polymères peuvent être le polyéthylène glycol (PEG) et le polystyrène.

1) Selon un autre mode de réalisation hors invention le ou les dérivé(s) métallique(s) peut (peuvent) également être choisis parmi le ou les dérivé(s) de cuivre suivant(s) :

*i)* les oxyde(s) de cuivre (Cu) de degré d'oxydation I ou II (Cu I ou II) ;
*ii)* les complexes métalliques de cuivre tels que les métalloporphyrine(s) de Cu I et II, les phtalocyanines et les chlorophyllines de cuivre ;
*iii)* les sel(s) de Cu I et II choisis parmi :

a) les halogénures de Cu II de formule $\mathbf{CuHal(R^2)}$ avec Hal représentant un atome d'halogène, et $R^2$ représentant un groupe hydroxy, $(C_1\text{-}C_6)$alkoxy, ou $R^1$-C(O)O-avec $R^1$ représentant un groupe $(C_1\text{-}C_6)$alkyle ,
b) les alkyl$(C_1\text{-}C_{16})$carboxylates de Cu II tels que l'acétate de Cu,
c) les $((C_1\text{-}C_{16})$alkyl)sulfates de Cu tels que le laurylsulfate de Cu ammoniacal,
d) les (bi)carbonate de Cu tel que le carbonate de Cu,
e) les alkyl$(C_1\text{-}C_{16})$polycarboxylates de Cu II tels que le citrate de Cu $Cu_3(C_6H_5O_7)_2$, le succinate de Cu,
f) les alkyl$(C_1\text{-}C_{16})$polycarboxylates de Cu II avec le groupe alkyle éventuellement interrompu par un ou plusieurs hétéroatomes tel que l'atome d'azote comme l'édétate de Cu,
g) les (poly)hydroxyalkyl$(C_1\text{-}C_{16})$carboxyates de Cu II tels que gluconate de Cu, le glycocollate de Cu, le lactate de Cu,
h) les hétérocycloalkylcarboxylates tels que le pidolate de Cu,
i) le désoxyribonucléate de Cu,
j) l'oxalate de Cu,
k) les $[(poly)(C_1\text{-}C_{16})$alkyl](poly)phosphates de Cu tels que le diphosphate de Cu,

*iv)* les complexes métalliques du Cu (I) ou (II) comprenant des ligands particulièrement ceux à ligands mono, di, tri ou tétra fonctionnalisés tels que :

a) les complexes de Cu(II) à ligand bisazométhine de formule (a) suivante :

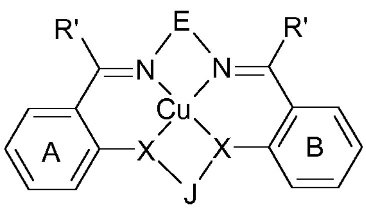

(α)

ainsi que ces hydrates, formule (a) dans laquelle :

◦ E représente une chaine divalente $(C_1-C_6)$alkylène linéaire ou ramifiée éventuellement substitué par des groupes oxo, ou $(C_2-C_6)$alkylène linéaire ou ramifiée,
◦ soit J est présent et représente un groupe tel que défini pour E et dans ce cas X représente un hétéroatome choisi parmi N et P ;
◦ soit J est absent et X représente un hétéroatome tel que O, S, N(R'), ou P(R') avec R' représentant un atome d'hydrogène ou un groupe $(C_1-C_6)$alkyle linéaire ou ramifié,
◦ A et B, identiques ou différents, sont des groupes aryle éventuellement substitués, ou hétéroaryle éventuellement substitués; et
◦ R' est tel que défini pour N(R') et P(R') ;

b) les complexes de Cu(II) à ligand azométhine tels que ceux de formule (β) suivante :

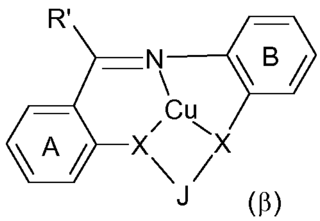

(β)

ainsi que ces hydrates, formule (β) dans laquelle : X, J, A, B, R' et J sont tels que définis précédemment dans la formule (a) ;
c) les dérivés de colorants directs azoïques à complexe de cuivre tels que ceux de formule (γ) suivante :

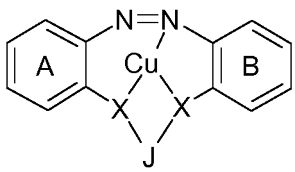

(γ)

avec X, J, A et B tels que définis précédemment dans la formule (α),
d) les complexes de Cu(II) issus de colorants sont particulièrement les complexes de Cu du : 2,2'-dihydroxyazo ; 2,2'-hydroxyaminoazo ; 2,2'-dihydroxyazométhine, 2,2'-dihydroxycarboxyazo ; 2,2'-dihydroxycarboxyazométhine ; les ligands tridendates dérivés des formazans ;

plus particulièrement ;

i) les oxyde(s) de cuivre (Cu) de degré d'oxydation I ou II (Cu I ou II),
ii) les complexes métalliques du cuivre tels que les métalloporphyrines de Cu I et II, les phtalocyanines telle que décrites dans US 3,931,249 et les chlorophyllines de cuivre,
iii) les sels de Cu I et II choisis parmi :

a) les halogénures de Cu II de formule **CuHal($R^2$)** avec Hal représentant un atome d'halogène, et $R_2$ représentant un groupe hydroxy, $(C_1-C_6)$alkoxy, ou $R^1$-C(O)O- avec $R^1$ tel que défini précédemment,
b) les alkyl$(C_1-C_{16})$carboxyates de Cu II tels que l'acétate de Cu,
c) les $((C_1-C_{16})$alkyl)sulfates de Cu tels que le laurylsulfate de Cu ammoniacal,
d) les (bi)carbonates de Cu tel que le carbonate de Cu,
e) les alkyl$(C_1-C_{16})$polycarboxylates de Cu II tels que le citrate de Cu $Cu_3(C_6H_5O_7)_2$, le succinate de Cu,
f) les alkyl$(C_1-C_{16})$polycarboxylates de Cu II avec le groupe alkyle éventuellement interrompu par un ou plusieurs hétéroatomes tel que l'atome d'azote comme l'édétate de Cu,
g) les (poly)hydroxyalkyl$(C_1-C_{16})$carboxyates de Cu II tels que gluconate de Cu, le glycocollate de Cu, le lactate de Cu,
h) les hétérocycloalkylcarboxylate tel que le pidolate de Cu,
i) le désoxyribonucléate de Cu,

j) l'oxalate de Cu,

k) les [(poly)($C_1$-$C_{16}$)alkyl](poly)phosphates de Cu tel que le diphosphate de Cu, ,.

iv) les complexes métalliques du Cu (I) ou (II) comprenant des ligands tels que les *metal complex dyes* décrits dans Ullmann's Encyclopedia, 2005 Wiley-VCH Verlag GmbH & Co, KgA, Weinheim, 10.1002/14356007.a16_299, pp. 1-42, particulièrement ceux à ligands mono, di, tri ou tétra fonctionnalisés tels que :

a) les complexes de Cu(II) à ligand bisazométhine tels que ceux de formule (a) suivante :

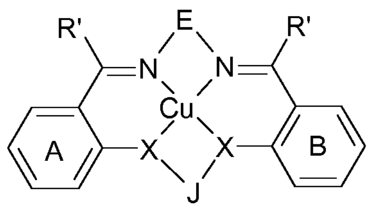

(α)

ainsi que ces hydrates, fo
rmule (α) dans laquelle :

- E représente une chaine divalente ($C_1$-$C_6$)alkylène linéaire ou ramifiée éventuellement substitué par des groupes oxo, ou ($C_2$-$C_6$)alkylène linéaire ou ramifiée telle que éthylène $-CH_2-CH_2-$, arylène tel que ortho phénylène, hétéroarylène,
- Soit J est présent et représente un groupe tel que défini pour E et dans ce cas X représente un hétéroatome choisi parmi N et P ;
- Soit J est absent et X représente un hétéroatome tel que O, S, N(R'), ou P(R') avec R' représentant un atome d'hydrogène ou un groupe ($C_1$-$C_6$)alkyle linéaire ou ramifié, préférentiellement X = O,
- A et B, identiques ou différents, particulièrement identiques, sont des groupes aryle éventuellement substitués, ou hétéroaryle éventuellement substitués; préférentiellement A et B sont des aryles tels que phényle et
- R' est tel que défini pour N(R') et P(R'), préférentielle R' est un atome d'hydrogène ;

plus particulièrement le complexe de Cu est tel que X = O, E = éthylène, J est absente, R' = H, A et B = phényle susbtitué en para de l'atome d'oxygène par un groupe sulfate de métal alcalin tel que le sodium ou tel que le Brilliant Red [76683-16-4] ;

b) les complexes de Cu(II) à ligand azométhine tels que ceux de formule (β) suivante :

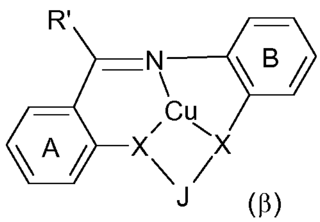

(β)

ainsi que ces hydrates,

formule (β) dans laquelle : X, J, A, B, R' et J sont tels que définis précédemment dans la formule (α), particulièrement R' représente un atome d'hydrogène, B représente un phényle, X représente un atome d'oxygène, A représente un phényle éventuellement substitué en para de l'oxygène par un phényle ou un naphtyle tels que le Pigment Yellow 117 [21405-81-2] et le Pigment Yellow 129 [68859-61-0] ;

c) les dérivés de colorants directs azoïques à complexe de cuivre tels que ceux de formule (γ) suivante :

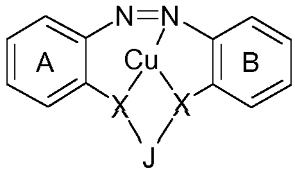

(γ)

avec X, J, A et B tels que définis précédemment dans la formule (α), particulièrement les composés de formule (γ) sont choisis parmi les Acid Dyes tels que le Sirius Light Blue 3 RL [13217-74-8], C.I. Direct Blue 93, Benzo Fast Red CGL, C.I. Direct Red 180, et ceux [92341-30-5], [119103-25-2], [116932-38-8], [113989-79-0] ;
les dérivés de colorants directs Bisazoïques à complexe de cuivre tels que le Direct Blue 80 [12222-003] ;
les dérivés des colorants Formazans ou formazan dyes tels que décrits dans Ullmann's Encyclopedia, 2005 Wiley-VCH Verlag GmbH & Co, KgA, Weinheim, 10.1002/14356007.a16_299, p. 27, point 6 et 6.1.2 : Bidendates tel que [53708-91-1], Tridendates ou Quadridendates tels que [36090-18-3] et [109973-79] ;
d) les complexes de Cu(II) issus de colorants sont particulièrement les complexes de Cu du : 2,2'-dihydroxyazo ; 2,2'-hydroxyaminoazo ; 2,2'-dihydroxyazométhine, 2,2'-dihydroxycarboxyazo ; 2,2'-dihydroxycarboxyazométhine, les ligands tridendates dérivés des formazanes.

Plus particulièrement le ou les dérivé(s) métallique(s) peut (peuvent) être choisi(s) parmi :

i) les oxyde(s) de cuivre (Cu) de degré d'oxydation I ou II (Cu (I) ou (II)), plus particulièrement Cu de degré d'oxydation I ;
ii) les complexes métalliques du cuivre tels que les métalloporphyrines de Cu (I) et (II), les phtalocyanines telle que décrites précédemment et les chlorophyllines de cuivre a et b :

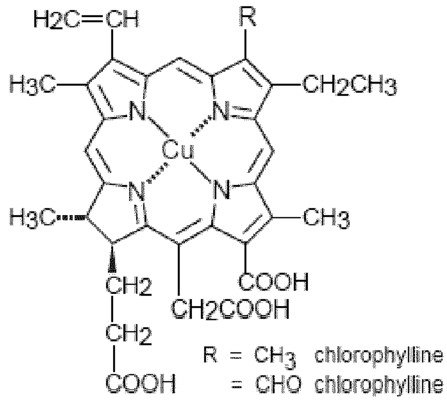

R = $CH_3$ chlorophylline a
= CHO chlorophylline b

et
iv) les complexes métalliques du Cu (I) ou (II) comprenant des ligands tels que définis précédemment.

Plus particulièrement le dérivé métallique de cuivre est choisi parmi le gluconate de cuivre, la chlorophylline a ou b cuivrique, et CuHal(OH) avec Hal représentant un atome d'halogène tel que CuCl(OH).
**i)** Selon un mode de réalisation préféré de l'invention le ou les dérivé(s) métallique(s) est (sont) de l'or (Au). Plus particulièrement :

a) des oxydes d'or I et III tel que $Au_2O_3$,
b) les hydroxydes d'Au I et III tels que $Au(OH)_3$, AuOH, Au(O)OH,
c) des sels d'or I particulièrement de formule AuHal avec Hal représentant un atome d'halogène (F, Cl, Br, I) tel que AuCl ou AuI, ,
d) des sels d'or III particulièrement choisi parmi les formules suivantes :

- **$Au(Hal)_3$** avec Hal, identiques ou différents, tels que définis précédemment, comme AuCl3, et $AuBr_3$,
- **$ZAu(Hal)_4$,** hydraté ou non, avec Z représentant un atome d'hydrogène, un métal alcalin tel que Li, Na, K ou un ammonium $NH_4^+$, et Hal, identiques ou différents, tels que définis précédemment, comme le KAuCl4 ou

HAuCl4,

- **$Au(R)_3$** avec R, identiques ou différents, représentant :
  - un groupe ($C_1$-$C_6$)alkylcarbonyloxo, où le groupe alkyle est linéaire ou ramifié, tel que méthyle ou terbutyle tel que $Au(OAc)_3$,
  - ou alors un ou deux des groupes R représentant un ligand L portant au moins un groupe électrodonneur tel que amino, phosphino, hydroxy, thiol, ou le ligand est un carbène « persistant » particulièrement de type « *Arduengo* » (Imidazol-2-ylidenes) ; préférentiellement le ligand est une phosphine telle que triphényl phosphine par exemple $(Ph_3P)AuOC(O)^tBu$,

v) des complexes métalliques de l'Au tels que des métalloporphyrines d'Au I et III des phtalocyanines d'or I et III ou des chlorophyllines a ou b d'or I et III ;

Préférentiellement, le ou les dérivé(s) métallique(s) est (sont) choisi(s) parmi les oxydes d'or I et III tels que $Au_2O_3$, les hydroxydes d'or et les sels d'or III tels que $ZAu(Hal)_4$, et $Au(R)_3$. Plus préférentiellement le dérivé métallique est choisi parmi les oxydes et hydroxydes tels que $Au_2O_3$, $Au(OH)_3$, AuOH.

**ii)** Selon un mode de réalisation particulier de l'invention, le ou les dérivé(s) métallique(s) est (sont) de l'argent (Ag). Plus particulièrement :

a) des oxydes d'argent I et II tels que $Ag_2O$ et AgO,

b) des sels d'Ag I particulièrement choisis parmi les formules suivantes :

- AgHal avec Hal représentant un atome d'halogène (F, Cl, Br, I) tel que AgCl, AgBr, AgI ;
- $Ag_xR^3_z$, avec $R^3$ identiques ou différents représentant :
  - un groupe sulfate tels que $Ag_2SO_4$
  - un groupe ($C_1$-$C_6$)alkylcarbonyloxo où le groupe alkyle est linéaire ou ramifié et peut éventuellement être substitué par un groupement hydroxyle, tel que l'acétate d'Ag, le propionate d'Ag, le lactate d'Ag
  - x et z tels que définis précédemment
  - à l'exception du nitrate d'argent $Ag(NO_3)$,:

c) des métalloporphyrines d'Ag I ;

d) des phtalocyanines d'Ag I telles que décrites dans US 3,931,249 ; et

e) des chlorophyllines a ou b d'Ag I.

**iii)** Selon un mode de réalisation particulier de l'invention, le ou les dérivé(s) métallique(s) est du molybdène (Mo). Particulièrement les dérivé(s) métalliques de nombre d'oxydation (II) à (VI) et sont tels que décrits *dans* Kirk-Othmer Encyclopedia of Chemical Technology Copyright© 2001 by John Wiley & Sons, Inc. Last updated: 17 Sep 2009, « Molybdenium compounds », Edward I. Stieffel, pp. 871-895 ou Ullmann's Encyclopedia; WILEY-VCH Verlag GmbH & Co. KGaA, 2000-2005, « Molybdenum and Molybdenum Compounds ».

Plus particulièrement :

a) des oxydes de Mo particulièrement VI tels que :

- l'oxyde de molybdène(VI),de formule **$MoO_3$**;
- les oxydes de Mo (IV) à ligand $\beta$-dicétones **$MoO_2L_2$** avec L ligand, identiques ou différents, préférentiellement identiques représentent une $\beta$-dicétone de type **R-C(X)-C(R')-C(X)R"** avec R et R", identiques ou différents, représentent un groupe ($C_1$-$C_6$)alkyle, linéaire ou ramifié, et R' représente un atome d'hydrogène ou un groupe ($C_1$-$C_6$)alkyle, linéaire ou ramifié, X représente un atome d'oxygène, de soufre ou un groupe N(R) avec R représentant un atome d'hydrogène ou un groupe ($C_1$-$C_6$)alkyle, linéaire ou ramifié, plus particulièrement le dioxide de Mo est de formule $MoO_2[CH_3C(O)CHC(O)CH_3]_2$ [*17524-05-9*] ;
- les complexes d'oxyde de Mo(VI) provenant du $MoO_3$ et de ligand hydroxyacide carboxylique en $C_2$-$C_{10}$ notamment d'acide citrique, maléique, tels que décrits dans l'article C. B. Knobler et al., J. Chem. Soc. Dalton Trans. 1983, 1299) ou de polyols en $C_2$-$C_{10}$ comprenant de 2 à 5 groupes hydroxy notamment l'éthylène glycol, glycérol, tels que décrits dans l'article F. A. Schroder, J. Scherle, Z. Naturforsch. B: Anorg. Chem. Org. Chem. 28B (1973) 46; voir également C. B. Knobler, B.R. Penfold, G. T. Wilkins, J. Chem. Soc. Dalton Trans. 1980, 248)

- le monohydrate, $\mathbf{MoO_3 \cdot H_2O}$ [*39082-25-2*], le dihydrate,$\mathbf{MoO_3 \cdot 2H_2O}$ [25942-34-1], l'acide molybdique ($\mathbf{H_2MoO_4 \cdot H_2O}$);
- le dioxide de Molybdène ;
- dihalogénodioxomolybdène $\mathbf{(Hal)_2MoO_2}$ avec Hal identique ou différent est tel que défini précédemment, particulièrement Hal sont identiques et représentent un atome de chlore ;
- les molybdènes bleus ou « molybdenum blues » [66771-43-5], mélange d'oxyde de Mo / hydroxydes de Mo(VI) et Mo(V) tels que décrits dans l'article V. K. Rudenko, Koord. Khim. 5 (1979) 307; *(*Sov. J. Coord. Chem. (Engl. Transl.) 5 (1979) 231 ; particulièrement $Mo^{6+}{}_3$ $Mo^{5+}{}_3$ $O_{18}H$, et les dérivés de condensation d'avec les ions phosphates ;
- les mélanges d'oxydes de Mo à valences différentes Mo(VI)-Mo(V) (les bronzes d'oxyde de Mo) tels que décrit dans l'article M. Greenblat, Chem. Rev. 88 (1988) 31; plus particulièrement les bronzes binaires et les bronzes tertiaires,$\mathbf{A_{0.33}MoO_3}$ (A = Li, K, Rb, Cs, Tl), ; $\mathbf{A_{0.3}MoO_3}$ (A = K, Rb, Tl) ; $\mathbf{A_{0.9}Mo_8O_{17}}$ (A = Li, Na, K, Tl), et les bronzes à terre rares $La_2Mo_2O_7$;.

b) des oxanions de Mo choisi parmi les molybdates $\mathbf{Z_2MoO_4}$ avec Z, identiques ou différents, tels que définis précédemment tel que, le molybdate de sodium $Na_2MoO_4$, le molybdate d'ammonium $(NH_4)2MoO_4$ ;

c) des polyoxométallates tels que : $\mathbf{[XY_uMo_{12-u}O_{40}]^{(3+u)-}(Z)_{(3+u)}}$ avec X et Y choisis parmi P, Si, V ; $0 \leq u \leq 6$, et Z est tel que défini précédemment où Z représente un atome d'hydrogène, particulièrement le polyoxométallate est de formule $H_5PV_2MO_{10}O_{40}$,

d) les halogénures de Mo binaires d'états d'oxydation (II) à (VI), hexacoordiné par 6 halogènes de Mo(V), (IV) et (III), les atomes de Mo étant lié par des liaison halogène tel que l'halogénure de Mo(II) contenant des clusters $[Mo_6Hal_8]^{4+}$ liés à des atomes d'halogène pour donner $\mathbf{Mo_6Hal_{12}}$ avec Hal, identiques ou différents, tels que défini précédemment et plus particulièrement Hal représente Cl ;

e) les tétrahalogénures de molybdène $\mathbf{(Hal)_4Mo}$ avec Hal', identiques ou différents, tels que définis précédemment, tel que $MOCl_4$;

f) les dérivés soufrés du Mo choisis parmi :

- les disulfures de Molybdenum [1317-33-5], sulfures de molybdenum(IV), $MoS_2$ les molybdates de formule $\mathbf{(Z)_2MoS_4}$, avec Z, identiques ou différents, tels que défini précédemment, particulièrement Z représentent un ammonium tels que tétrathiomolybdate $(NH_4)_2[MoS_4]$[*15060-55-6*] ;
- les sesquisulfure de Mo [12033-33-9] ; les trisulfures de dimolybdène (III), $Mo_2S_3$ ;
- les sels de tétrasulfures $\mathbf{Z\ MoS_{24}^{-}}$ avec Z tel que défini précédemment, préférentiellement représente l'ammonium ;

g) les dérivés d'Oxomolybdène (VI) choisi parmi :

- $\mathbf{Mo(O)Hal_4}$ avec Hal, identiques ou différents, sont tels que définis précédemment, particulièrement Hal représente l'atome de F et le Cl ;
- ,$\mathbf{Mo(O)_2Hal_2}$ avec Hal, identiques ou différents, sont tels que définis précédemment, particulièrement Hal représente l'atome de F, le Cl et le Br ;

h) les dérivés trihalogénooxomolybdène(V) et ces adduits avec des ligands organiques L tels que définis précédemment, L représente préférentiellement :

- un groupe bidendate R-C(X)-CR'R"-C(X)-R'" avec R et R"", identiques ou différents, représentant un groupe $(C_1-C_6)$alkyle, linéaire ou ramifié et R' et R", identiques ou différentes représentent un atome d'hydrogène ou un groupe $(C_1-C_6)$alkyle, linéaire ou ramifié, préférentiellement R' et R" représentent un atome d'hydrogène, X représente un atome d'oxygène, de soufre, ou un groupe N(R) avec R représentant un atome d'hydrogène ou groupe $(C_1-C_6)$alkyle, linéaire ou ramifié, tel que l'acétylacétone ;
- un groupe bidendate de type 2,2-bipyridyle ;

i) le dérivé d'Oxomolybdène de formule $\mathbf{Mo(O)Hal_3 \cdot 2\ L}$ avec L et Hal tels que définis précédemment, préférentiellement L représente un R"-O-R' avec R et R' tels que définis précédemment, tel que diéthyléther et un groupe hétéroaryle tel que pyridine ;

j) les molybdates, isopolymolybdates, et hétéropolymolybdates contenant un anion tétraédrique $[MoO4]^{2-}$ tels que l'ammonium heptamolybdate (isopolymolybdate), $(NR'_4)_6Mo_7O_{24}$ hydraté, avec R' identiques ou différents sont tels que définis précédemment, particulièrement R' est un atome d'hydrogène ;

k) les molybdates de cations divalents notamment ceux solubles dans l'eau tels que les molybdates du $Mg^{2+}$ et les

molybdates de cations trivalents notamment de formules $\mathbf{A_2(MoO_4)_3}$ ou $\mathbf{A_2Mo_3O_{12}}$, avec A représentant un atome choisi parmi Al, Cr, Bi et Lanthanide ;

l) les hétéropolymolybdates à octaèdre [$\mathbf{MoO_6}$] incorporant d'autres hétéroatomes que l'atome d'oxygène choisi parmi notamment S, N et P, Plus spécifiquement les hétéromolybdates sont de formule $\mathbf{[X^+{}_nMo_{12}O_{40}]^{(8-n)-}}$, à hétéroatomes (X) tétracoordinés tel que [*12026-57-2*], $H_3[PMo_{12}O_{40}]\cdot 28H_2O$,

m) les complexes des Mo avec des ligands organosoufrés tels que les phosphorodithioates ou dithiophosphates et dithiocarbamates, $\mathbf{[Mo_2O_3L_4]}$ et $\mathbf{[Mo_2O_2S_2L_2]}$ avec L tel que défini précédemment, particulièrement L représente $(RO)_2PS^-{}_2$ , $R_2NCS^-$ avec R, identiques ou différents, sont tels que définis précédemment ;

n) l'hexacarbonyl de molybdène [13939-06-5], $Mo(CO)_6$;

o) les pigments organiques dérivés de molybdate de métal alcalin et alcalino-terreux tel que dérivés du molybdate de sodium tels que dérivés de i) Diarylméthane (Auramine C.I. 655), ii) Triarylméthane (Malachite Green C.I. 657, Brilliant Green C.I. 662 ; Rhoduline Blue 6G C.I. 658, Acronol Brilliant Blue C.I. 664, Methyl Violet B C.I. 680; Victoria Pure Blue BO C.I. Pr198); iii) Xanthène (Rhodamine B C.I. 749, Rhodamine 6G) ; et

p) les molybdates de Ca et de Sr et

q) les porphyrines de Mo telles que décrites dans l'article de T. Ma, K. Inoue, E. Abe, J. Yu, X. Wang, B. Zhang, J Electroanal. Chem. 537 (2002) 31, et phtalocyanines de Mo telles que décrites dans US 3,931,249.

Préférentiellement, le dérivé métallique est choisi parmi les composés de formule $Z_2$MoO4 tel que Na2MoO4,
iv) Selon une variante particulière de l'invention, le ou les dérivé(s) métallique(s) est (sont) du tungstène (W). Plus particulièrement a) des oxydes de tungstène VI, b) des oxanions de tungstène préférentiellement les tungstates de métal alcalin $\mathbf{Z_2WO_4}$, hydraté ou non, avec Z, identiques ou différents, tels que définis précédemment, c) des polyoxométallates tels que $\mathbf{[XY_uW_{12-u}O_{40]}{}^{(4+1)-};(Z)_{(4+u)}}$ avec X et Y choisis parmi P, Si, V ; $0 \le u \le 6$, particulièrement le polyoxométallate est de formule $H_4SiW_{12}O_{40}$.
Préférentiellement, le dérivé métallique est choisi les tungstates de métal alcalin tels que le tungstate de sodium $Na_2WO_4$.
v) Selon une variante particulière de l'invention, le ou les dérivé(s) métallique(s) est (sont) du vanadium (V). Plus particulièrement a) des oxydes de vanadium tel que le $V_2O_5$, b) des oxanions de vanadium choisis parmi les vanadates et métavanadates tels que l'acéthylacétonate d'oxyde de vanadium $VO(acac)_2$, $VOSO_4$, ammonium vanadate et c) des polyoxométallates tels que $\mathbf{[XV_uM_{12-u}O_{40}]^{q-};(Z)_q}$ avec M = W, Mo ; X = P, Si ; $0 \le u \le 6$ et q=3+x si M=Mo ou q=4+x si M=W et Z est tel que défini précédemment, particulièrement le polyoxométallate est de formule $H_5PV_2MO_{10}O_{40}$ et d) des complexes de vanadium tels que ceux décrits dans US 3,931,249.
Préférentiellement, le dérivé métallique est choisi parmi ceux de formule $\mathbf{[XV_uMO_{12-u}O_{40}]^{(3+u)};(Z)_{(3+u)}}$ telle que définie précédemment et particulièrement $H_5PV_2Mo_{10}O_{40}$.
vi) Selon une mode de réalisation particulier de l'invention, le ou les dérivé(s) métallique(s) est (sont) du ruthénium (Ru). Plus particulièrement i) des oxydes de ruthénium, ii) des oxanions de ruthénium tels que les perruthénates de métal alcalin, et iii) des complexes de ruthénium tels que $\mathbf{(Hal)_2RuL_4}$ avec Hal identiques ou différents, tels que définis précédemment et L, identiques ou différents, sont des ligands tels que définis précédemment.
Préférentiellement, le dérivé métallique est choisi parmi $RuCl_2(PPh_3)_4$, le perruthénate de potassium.
vii) Selon une mode de réalisation particulier de l'invention, le ou les dérivé(s) métallique(s) est (sont) choisi(s) parmi les métalloporphyrines de magnésium II, d) les phtalocyanines de magnésium II, e) les chlorophyllines de magnésium II, f) les chlorophylles de magnésium II, les colorants à complexes métalliques ou *metal complex dyes* tels que décrits dans Ullmann's Encyclopedia, 2005 Wiley-VCH Verlag GmbH & Co, KgA, Weinheim, 10.1002/14356007.a16_299, pp. 1-42) en particuliers ceux dérivés des formazanes tels que décrits dans Ullmann's Encyclopedia, 2005 Wiley-VCH Verlag GmbH & Co, KgA, Weinheim, 10.1002/14356007.a16_299, p. 27, point 6.1.2.
Préférentiellement, le dérivé métallique est choisi parmi la chlorophylline a ou b magnésique et la chlorophylle a ou b magnésique :

| 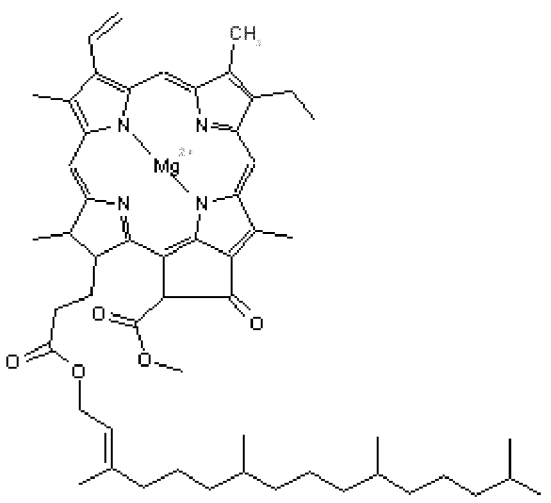 | 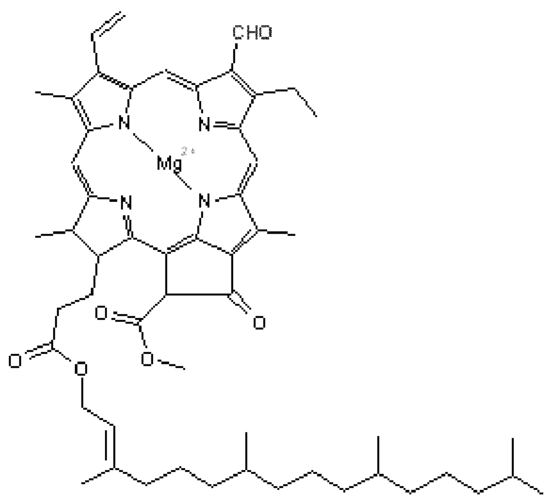 |
|---|---|
| Chlorophylle a | Chlorophylle b |

**ix)** Selon une mode de réalisation particulier de l'invention, le ou les dérivé(s) métallique(s) est (sont) du Rhénium (Re). Plus particulièrement du $R'ReO_3$ avec R' représentant un atome d'hydrogène ou un groupe ($C_1$-$C_6$)alkyle, linéaire ou ramifié, tel que $CH_3ReO_3$ ou les complexes de Re tels que les phtalocyanines décrites par exemple dans US 3,931,249.
**x)** Selon une mode de réalisation particulier de l'invention, le ou les dérivé(s) métallique(s) est (sont) du titane (Ti). Particulièrement

a) des sels de titane IV tels que le $Ti(SO_4)_2$,
b) des oxydes de titane notamment choisi(s) parmi les oxydes de titane ainsi que leurs sels, leurs hydrates et leurs formes supportées. A titre d'exemple on peut citer les oxydes et hydroxydes de Ti sont tels que ceux décrits dans Ullmann's encyclopedia « Titanium, Titanium Alloys, and Titanium Compounds », 2005 Wiley-VCH Verlag GmbH & Co. KGaA, Weinheim, 10.1002/14356007.a27 095, pp. 1-33. Plus particulièrement le ou les dérivé(s) métallique(s) est (sont) du titane (Ti) et, choisi(s) parmi l'hydroxide et d'hydroxide de Titane (III) $Ti(OH)_3$ et $TiO_3$, le trioxide de dititane $Ti_2O_3$, les trioxydes de titane de métal alcalinoterreux, les pentoxides de titane de métal alcalinoterreux, les titanates de formule générale $\mathbf{M^{II}TiO_4}$ dans laquelle $M^{II}$ représente un métal Mg, Zn, Mn ou Co, l'acide peroxytitanique et les peroxytitanates $H_4TiO_5$, le dioxyde de titane (II) $TiO_2$, le disulfure de titane $TiS_2$, Les oxides peuvent provenir de minéraux tels que anatase et la rutile qui contiennent du $TiO_2$; la pérovskite qui contient du trioxyde de calcium $CaTiO_3$, a sphène ou titanite qui contient du $CaTi(SiO_4)O$ ;
c) des complexes de Ti tels que les phtalocyanines décrites par exemple dans US 3,931,249.

Préférentiellement le ou les dérivé(s) métallique(s) est (sont) $TiO_2$.
**xi)** Selon une mode de réalisation particulier de l'invention, le ou les dérivé(s) métallique(s) est (sont) du silicium (Si), plus particulièrement de l'oxyde de silicium ainsi que leurs sels, leurs hydrates et leurs formes supportées.tel que $SiO_2$.
**xii)** Selon une mode de réalisation particulier de l'invention, le dérivé métallique est (sont) des oxydes d'étain ainsi que leurs sels, leurs hydrates et leurs formes supportées. A titre d'exemple on peut citer les oxydes d'étain sont tels que ceux décrits dans le point 10 et 11 de Ullmann's encyclopedia « Tin, Tin Alloys, and Tin Compounds », 2005 Wiley-VCH Verlag GmbH & Co. KGaA, Weinheim10.1002/14356007.a27 049, pp. 27-29. Particulièrement l'oxyde d'étain (II) Hydraté tel que $5\ SnO{\cdot}2H_2O$ et l'oxyde d'étain (II), l'oxyde d'étain (IV) hydraté $SnO_2{\cdot}nH_2O$ et l'oxyde d'étain (IV) $SnO_2$, les sels de métal alcalin tel que le sodium et potassium d'hydroxyde d'étain de formule $\mathbf{M_2\ [Sn(OH)_6]}$ avec M représentant un métal alcalin, les hydroxydes d'étain de formule $\mathbf{R_3SnOH}$, $\mathbf{R_2SnOH_2}$, ou $\mathbf{RSnOH_3}$ avec, R qui représente un groupe hydrocarboné tel que ($C_1$-$C_6$)alkyle, linéaire ou ramifié, ou ($C_1$-$C_6$)alkoxy, linéaire ou ramifié, (di) ($C_1$-$C_6$)alkylamino. Préférentiellement le ou les oxyde(s) métallique(s) est(sont), l'oxyde d'étain (IV) $SnO_2$.
**xiii)** Selon une mode de réalisation particulier de l'invention, le ou les dérivé(s) métallique(s) est (sont) du zirconium (Zr). Particulièrement le ou les dérivé(s) métallique(s) est (sont) du zirconium (Zr) choisi(s) parmi :

a) les oxyde de titane ainsi que leurs sels, leurs hydrates et leurs formes supportées. A titre d'exemple on peut citer les oxydes et hydroxydes de Ti sont tels que ceux décrits dans les points 2.2, 2.3 et 2.5 et de Ullmann's encyclopedia

« Zirconium and Zirconium Compounds », 2005 Wiley-VCH Verlag GmbH & Co. KGaA, Weinheim, 10.1002/14356007.a28 543, pp. 15-18. Plus particulièrement le ou les oxyde(s) de zirconium est(sont), choisi(s) parmi l'oxyde de Zirconium [1314-23-4], $ZrO_2$, l'hydrate d'oxyde de zirconium $[Zr_4(OH)_8 \cdot 16H_2O]_8$, le dihalogénure d' Hydroxide de zirconium tel que le dichloride d'hydroxyde de Zirconium [*22196-48-1*], ainsi que les composés de formule $Zr(OH)_2Hal_2 \cdot 7H_2O$, avec Hal représentant un atome d'halogène tel que le chlore, les oxyhalogénure de zirconium zirconium tel que l'oxychlorure de zirconium,les oxides d'halogénure de zirconium tel que dichlorure d'oxyde de zirconium, $ZrOCl_2 \cdot 8H_2O$, $[Zr_4(OH)_8 \cdot 16H_2O]Cl_8 \cdot 12H_2O$ et le monohalogénure de zirconium tel que le monochlorure d'hydroxide de zirconium $[Zr_4(OH)_{12} \cdot 16H_2O]Cl_4$ ;

b) et les complexes de Zr tels que les phtalocyanines décrites par exemple dans US 3,931,249.

Préférentiellement le ou les dérivés(s) métallique(s) est(sont), l'oxyde de zirconium (II) le $ZrO_2$.
**xiv)** Selon une mode de réalisation particulier de l'invention, le ou les dérivé(s) métallique(s) est (sont) du niobium (Nb). Particulièrement le ou les oxyde(s) métallique(s) est(sont), choisi(s) parmi les oxydes de niobium ainsi que leurs sels, leurs hydrates et leurs formes supportées. A titre d'exemple on peut citer les oxydes et hydroxydes de Nb sont tels que ceux décrits dans les points 5.1 et 5.2 et de Ullmann's encyclopedia « Niobium and Niobium Compounds », 2005 Wiley-VCH Verlag GmbH & Co. KGaA, Weinheim, 10.1002/14356007.a17251, pp. 5-6. Plus particulièrement le ou les oxyde(s) de Nb est(sont), choisi(s) parmi le pentoxyde de niobium $Nb_2O_5$ [*1313-96-8*], le trioxyde de niobium de métal alcalin tel que le trioxyde de niobium de lithium $LiNbO_3$ [12031-63-9] ou $KNbO_3$ [12030-85-2] et les oxyhalogénures de Niobium tel que l'oxychlorure de chlore [*13597-20-1*], $NbOCl_3$.
Préférentiellement le ou les dérivés(s) métallique(s) est(sont) le pentoxyde de niobium $Nb_2O_5$.
**xv)** Selon une mode de réalisation particulier de l'invention, le ou les dérivé(s) métallique(s) est (sont) de l'indium (In). Particulièrement le ou les dérivé(s) de l'indium (In) est(sont) choisi(s) parmi les oxyde d'indium ainsi que leurs sels, leurs hydrates et leurs formes supportées. A titre d'exemple on peut citer les oxydes d'indium sont tels que ceux décrits dans le point 7 de Ullmann's encyclopedia « Indium and Indium Compounds », 2005 Wiley-VCH Verlag GmbH & Co. KGaA, Weinheim10.1002/14356007.a14 157, pp. 7.
Plus particulièrement l'oxyde de Indium(III) de formule $In_2O_3$, l'oxyde d'Indium(II) [12136-26-4] de formule InO, et l'oxyde d'indium(I) [12030-22-7] de formule $In_2O$, l'hydroxyde d'Indium [56108-30-6], de formule $In(OH)_3$, Préférentiellement l'oxyde d'indium (III) $In_2O_3$. Préférentiellement $In_20_3$.
**xvi)** Selon une mode de réalisation particulier de l'invention, le ou les dérivé(s) métallique(s) est (sont) du sélénium (Se). Plus particulièrement du $SeO_2$.

Un mode de réalisation préféré de l'invention concerne l'ingrédient b) qui se trouve dans une seule espèce métallique choisie parmi **i)** à xvi) tels que définis précédemment.

Préférentiellement le ou les dérivé(s) métallique(s) b) est(sont) choisi(s) parmi les sels métalliques, complexes métalliques, oxydes métalliques, oxanions métalliques, leurs formes supportées, leurs hydrates et leur mélanges pour lesquels le ou les métal(aux) est (sont) choisi(s) parmi **i)** l'or (Au), **ii)** le molybdène (Mo), **iv)** le tungstène (W), **xi)** le silicium (Si), **xii)** les oxydes d'étain, **xiii)** le zirconium (Zr), et **xv)** l'indium (In).

Plus préférentiellement le ou les dérivé(s) métallique(s) b) est (sont) choisi(s) parmi **i)** l'or (Au), **ii)** l'argent (Ag), **iii)** le molybdène (Mo), **iv)** le tungstène (W), **v)** le vanadium (V), **vi)** le ruthénium (Ru), **vii)** le magnésium (Mg), **xi)** le silicium (Si). Particulièrement le ou les dérivé(s) métallique(s) b) est (sont) choisi(s) parmi les composés de formule $ZAu(Hal)_4$ tel que $KAuCl_4$; $Au(R)_3$ $Au_2O_3$ ; $Z_2MoO_4$; $Z_2WO_4$; $[XY_uMo_{12-u}O_{40}]^{q-}(Z)_q$ et $H_5(PV_2Mo_{10}O_{40})$, $(Hal)_2RuL_4$ tels que définis dans la revendication précédente ; chlorophylline de magnésium ; $KRuO_4$, $H_4SiW_{12}O_{40}$ et $Ag_2O$.

Selon une autre variante b) est choisi parmi les composés de formule $ZAu(Hal)_4$; $Au(R)_3$ $Au_2O_3$ ; $Z_2MoO_4$ ; $Z_2WO_4$ ; $[XY_xMo_{12-x}O_{40}]^{(3+x)-}(Z)_{(3+x)}$ ; $TiO_2$; $SiO_2$ ; $SnO_2$ ; $ZrO_2$ ou $In_2O_3$.

Selon un mode réalisation préféré de l'invention le ou les dérivé(s) métallique(s) b) est (sont) de l'or (Au). Plus particulièrement, le ou les dérivé(s) métallique(s) b) est (sont) de l'or (Au) à l'exception des sels d'or.

Selon un mode de réalisation préféré de l'invention, le ou les dérivés métalliques utilisés représentent de 0,0001% à 10% en poids environ du poids total de la ou des composition(s) contenant ce ou ces sels métalliques et encore plus préférentiellement de 0,0001% à 0,1% en poids environ.

Selon un mode de réalisation préféré de l'invention, le ou les oxyde(s) métallique(s), leurs sels, leurs hydrates et leurs formes supportées utilisés représentent de 0,0001% à 10% en poids environ du poids total de la ou des composition(s) contenant ce ou ces sels métalliques et encore plus préférentiellement de 0,0001% à 0,1% en poids environ.

*c) agents) alcalinisant(s)*

L'agent alcalinisant utilisé dans le procédé de coloration selon l'invention en tant que troisième ingrédient **c)** est choisi parmi i) les (bi)carbonates, et iii) les alcanolamines telles que les mono-, di- et triéthanolamines ainsi que leurs dérivés iv) les éthylènediamines oxyéthylénées et/ou oxypropylénées,

Par (bi)carbonates i) est sous entendu :

a) les carbonates de métal alcalins (Mét$_2^+$, $CO_3^{2-}$), de métal alcalino-terreux (Mét'$^{2+}$, $CO_3^{2-}$) d'ammonium ((R"$_4$N$^+$)$_2$,$CO_3^{2-}$) ou de phosphonium ((R"$_a$P$^+$)$_2$,$CO_3^{2-}$ avec Mét' représentant un métal alcalino-terreux et Mét représentant un métal alcalin, et R", identiques ou différents, représentent un atome d'hydrogène, un groupement ($C_1$-$C_6$)alkyle éventuellement substitué tel que hydroxyéthyle),
et
b) les bicarbonates, également appelés hydrogénocarbonates, de formules suivantes :

- R'$^+$, $HCO_3^-$ avec R' représentant un atome d'hydrogène, un métal alcalin, un groupement ammonium R"$_4$N$^+$- ou phosphonium R"$_4$P$^+$- où R", identiques ou différents, représentent un atome d'hydrogène, un groupement ($C_1$-$C_6$)alkyle éventuellement substitué tel qu'hydroxyéthyle et lorsque R' représente un atome d'hydrogène l'hydrogénocarbonate est alors appelé dihydrogénocarbonate ($CO_2$, $H_2O$) ; et
- Mét'$^{2+}$ ($HCO_3^-$)$_2$ avec Mét' représentant un métal alcalino-terreux.

Plus particulièrement, l'agent alcalinisant est choisi parmi les (bi)carbonates de métal alcalin ou alcalino-terreux ; préférentiellement les (bi)carbonates de métal alcalin.

On peut citer les carbonates ou hydrogénocarbonates de Na, K, Mg, Ca et leurs mélanges, et en particulier l'hydrogénocarbonate de Na. Ces hydrogénocarbonates peuvent provenir d'une eau naturelle, par exemple eau de source du bassin de Vichy, de La Roche Posay, eau de Badoit (cf. brevet par exemple le document FR 2 814 943). Particulièrement on peut citer le carbonate de sodium [497-19-8] = $Na_2CO_3$, l'hydrogénocarbonate de sodium ou bicarbonate de soude [144-55-8] = $NaHCO_3$, et le dihydrogénocarbonate de sodium = $Na(HCO_3)_2$.

Selon un mode de réalisation particulièrement avantageux le ou les agent(s) alcalinisant(s) c) est (sont) choisi(s) parmi les alcanolamines et les (bi)carbonates alcalins ou alcalino-terreux. De plus ils se trouvent préférentiellement ensemble lors du procédé de coloration.

Le ou les agent(s) alcalinisant(s) tels que définis précédemment représentent de préférence de 0,001% à 10 % en poids du poids de la ou des compositions les contenant. Plus particulièrement de 0,005 % à 8 % en poids de la composition.

*Les agents oxydants chimiques* :

Dans le cadre de la présente invention, le procédé de l'invention peut être réalisé en l'absence d'agent oxydant chimique c'est-à-dire en laissant agir l'oxygène de l'air.

Selon un autre procédé de coloration de l'invention, ledit procédé met en oeuvre un agent d'oxydation chimique.

*Par agent oxydant chimique on entend :*

a) l'ozone ;
b) les persels de métal alcalin ou d'ammonium quaternaire tels que les perborates, les persulfates, les percarbonates, les peroxodiphosphates, l'Oxone® particulièrement l'oxydant est choisi parmi le perborate de sodium, le persulfate de sodium, le persulfate de potassium, le persulfate d'ammonium, le percarbonate de sodium, le percarbonate de potassium ;
c) les peracides organiques aliphatiques en $C_1$-$C_6$ et aromatiques en $C_6$-$C_{20}$ et leurs formes percarboxylates, tels que l'acide performique, l'acide peracétique, les dérivés d'acides perbenzoïques, l'acide trifluoroacétique, l'acide peroxyphtalique, l'acide peroxymaléique, l'acide peroxypropionique particulièrement l'oxydant est l'acide peracétique ;
d) les peroxydes organiques, tels que le dioxirane, les peroxydes d'alkyles en $C_1$-$C_6$, le peroxyde de benzoyle, les carboxylates de peroxo($C_1$-$C_6$)alkyles, les peroxydes de bis(tri)($C_1$-$C_6$)alkylsilyle tel que le peroxyde bis(triméthylsilyle), les péroxydicarbonates d'alkyles en $C_1$-$C_6$, le nonanoyloxybenzene sulfonate de sodium tels que décrits dans les documents WO1995000625 et US4412934
e) les anions oxydants tels que les nitrites, les nitrates, les hypochlorites, hypobromites, hypoiodites, les chlorites, bromites, iodites, les chlorates, bromates, iodates, les periodates, particulier l'oxydant est choisi parmi l'hypochlorite ou le periodate de métal alcalin tel que l'hypochlorite de sodium ou le periodate de sodium ;
f) les radicaux stables N-oxy (NO·), tels que le radical 2,2,6,6-tétra($C_1$-$C_6$)alkylpipéridino-oxy, 2,2,6,6-té-

tra($C_1$-$C_6$)alkylmorpholino-oxy les sels de Frémy nitrosodisulfonates, la N-oxyde morpholine ; particulièrement l'oxydant est choisi parmi le radical 2,2,6,6-tétraméthylpipéridinyloxy

g) les dérivés hypervalents de l'iode, tels que l'iodotriacétate, l'iodosobenzène, l'iodobenzènetriacétate, les dérives d'acide iodoperbenzoiques, les périodinanes, les alkyls et benzoylhypoiodites

Plus préférentiellement l'oxydant est choisi parmi l'iodotriacétate, l'iodosobenzène, l'iodobenzènetriacétate, l'acide iodoperbenzoique, le periodinane de Dess-Martin

h) les composés organiques suivants : N-halogénosuccinimides, l'acide trichloroisocyanurique, la N-hydroxyphtalimide, les nitrites d'alkyles

Les supports éventuels de ces oxydants a) à h) peuvent être choisis parmi la silice, l'alumine, le charbon, des polymères chargés ou neutres

i) les peroxyde d'hydrogène ou système(s) générateur(s) de peroxyde d'hydrogène tel(s) que :

- i-1) le peroxyde d'urée ;
- i2) les complexes polymériques pouvant libérer du peroxyde d'hydrogène tels que le polyvinylpyrrolidone/$H_2O_2$ en particulier se présentant sous forme de poudres et les autres complexes polymériques décrits dans US 5,008,093 ; US 3,376,110 ; US 5,183,901 ;
- i-3) les oxydases produisant du peroxyde d'hydrogène en présence d'un substrat adéquat (par exemple le glucose dans le cas de glucose oxydase ou l'acide urique avec l'uricase) ;
- i-4) les peroxydes de métaux générant dans l'eau du peroxyde d'hydrogène comme le peroxyde de calcium, peroxyde de magnésium;
  - i-v) les perborates ; ou
  - i-vi) les percarbonates.

Particulièrement le ou les agents oxydants chimiques sont choisis parmi i) les peroxyde d'hydrogène ou systèmes générateurs de peroxyde d'hydrogène. Plus particulièrement $H_2O_2$

Selon un mode de réalisation préféré de l'invention le procédé met en oeuvre un ou plusieurs système(s) générateur(s) de peroxyde d'hydrogène choisi(s) parmi i) le peroxyde d'urée, i-2) les complexes polymériques pouvant libérer du peroxyde d'hydrogène choisis parmi le polyvinylpyrrolidone/$H_2O_2$ ; i-3) les oxydases; i-v) les perborates et i-vi) les percarbonates.

Par ailleurs la ou les compositions comprenant le peroxyde d'hydrogène ou le ou les générateur(s) de peroxyde d'hydrogène peut ou peuvent également renfermer divers adjuvants utilisés classiquement dans les compositions pour la teinture des cheveux et tels que définis ci-après au point intitulé « *composition cosmétique* ».

Selon un mode particulier de l'invention, le peroxyde d'hydrogène ou le ou les système(s) générateur(s) de peroxyde d'hydrogène utilisé(s) représente(nt) de préférence, de 0.001% à 12% en poids exprimé en peroxyde d'hydrogène par rapport au poids total de la composition ou des compositions le ou les contenant et encore plus préférentiellement de 0,2% à 2,7% en poids.

*Les photoirradiations par des ondes électromagnétiques de longueurs d'onde comprises dans l'UVjusqu'à l'IR ;*

Le procédé de coloration selon l'invention ou la composition selon l'invention peuvent être mis en oeuvre ou être appliqués sur des fibres kératinques en présence d'une ou plusieurs photoirradiation(s) par une ou plusieurs onde(s) électromagnétique(s) de longueur d'onde comprise entre 10 nm dans le domaine de l'ultra-violet (UV) et 100 $\mu$m dans le domaine de l'infrarouge (IR). Par photoirradiation avec une onde électromagnétique on entend toute exposition de la composition ou partie de la composition à une onde lumineuse lors du procédé de coloration capillaire. Le spectre lumineux pouvant comprendre les longueurs d'ondes comprises dans la région de l'UV (10-400nm), le visible (400-745nm), les infrarouges (745nm à 100$\mu$m).

Selon un mode particulier de l'invention le procédé de coloration a lieu à la lumière naturelle du Soleil ou du jour.

Selon un autre procédé de coloration des fibres kératinique la source de la photoirradiation est artificielle. Pour les lampes émettant dans l'UV, on peut citer celles décrites dans Ullmann's Encyclopedia « Ultraviolet and Visible Spectroscopy » 2008 Wiley-VCH Verlag GmbH & Co. KGaA, Weinheim, 10.1002/14356007.b05 383.pub2, point 3.2. Pour les lampes en général on peut citer celles mentionnées dans Ullmann's Encyclopedia « Lamps » 2005 Wiley-VCH Verlag GmbH & Co. KGaA, Weinheim 10.1002/14356007.a15 115 et Ullmann's Encyclopedia « Photochemistry » 2005 Wiley-VCH Verlag GmbH & Co. KGaA, Weinheim 10.1002/14356007.a19573, point 3.2 « light sources ».

Les lampes utilisée dans le procédé de l'invention sont notamment des lampes à incandescences, à halogène, fluorescentes, à mercure, à faible pression, des lampes à faible pression par exemple à sodium ou à néon, des lampes à forte pression par exemple à mercure, des lampes à halogénures, des lampes flash par exemple au xénon, des lampes à excimer fluorescent telle que celle à xénon, les Light Emitting Diodes ou LED de 50 à 1000mW, les lampes émettant

la lumière noire ou lumière de Wood, et les lasers.

Préférentiellement les sources artificielles proviennent de lampes à mercures, à halogène et tungstène, de tube néon blanc, de lampe UV émettant à 254 nm, ou à 365 nm.

*l'eau:*

Selon un mode réalisation de l'invention, de l'eau est de préférence incluse dans le procédé de l'invention. Elle peut provenir de l'humidification des fibres kératiniques et/ou de la ou des compositions comprenant les composés a) à c) tels que définis précédemment ou d'une ou plusieurs autres compositions. De préférence l'eau provient au moins d'une composition comprenant au moins un composé choisi parmi a) à c) tels que définis précédemment.

*compositions cosmétiques:*

Les compositions cosmétiques selon l'invention sont cosmétiquement acceptable i.e. elles comprennent un support de coloration qui contient généralement de l'eau ou un mélange d'eau et d'un ou plusieurs solvants organiques ou un mélange de solvants organiques.

Par solvant organique, on entend une substance organique capable de dissoudre ou disperser une autre substance sans la modifier chimiquement.

*Les Solvants organiques:*

A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, l'hexylène glycol, ainsi que les alcools aromatiques comme l'alcool benzylique ou le phénoxyéthanol.

Les solvants organiques sont présents dans des proportions de préférence comprises entre 1 et 60 % en poids environ par rapport au poids total de la composition tinctoriale, et encore plus préférentiellement entre 5 et 30 % en poids environ.

*Les Adjuvants:*

La ou les compositions du procédé de coloration conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Lesdits adjuvants sont choisis de préférence parmi des agents tensioactifs tels que des tensioactifs anioniques, non ioniques ou leurs mélanges et des agents épaississants minéraux ou organiques.

Les adjuvants ci-dessus sont en général présents en quantité comprise pour chacun d'eux entre 0,01 et 40 % en poids par rapport au poids de la composition, de préférence entre 0,1 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

*Les Colorants additionnels:*

Le procédé mettant en oeuvre les ingrédients **a)** à **c)** tels que définis précédemment et éventuellement un ou plusieurs agent(s) oxydant(s) chimique(s), l'eau ; ou la composition cosmétique selon l'invention comprenant les ingrédients **a)** à **c)** tels que définis précédemment et éventuellement un ou plusieurs agent(s) oxydant(s) chimique(s), l'eau ; peut en outre mettre en oeuvre ou comprendre un ou plusieurs colorants directs additionnels.

Ces colorants directs sont par exemple choisis parmi ceux classiquement utilisés en coloration directe, et parmi lesquels on peut citer tous les colorants aromatiques et/ou non aromatiques d'utilisation courante tels que les colorants directs nitrés benzéniques neutres, acides ou cationiques, les colorants directs azoïques différents des « *metal complex*

*dyes* » neutres, acides ou cationiques, les colorants directs naturels différents des orthodiphénols, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents. Tous ces colorants additionnels sont différents des dérivés d'orthodiphénols selon l'invention et des « metal-complex dyes » ou des porphyrines, métalloporphyrines, les phtalocyanines, appartenant à a) selon l'invention.

Parmi les colorants directs naturels, on peut citer la lawsone, la juglone, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs additionnels utilisés dans la ou les compositions représentent de préférence, de 0.001% à 10% en poids environ du poids total de la ou des compositions les contenant et encore plus préférentiellement de 0.05% à 5% en poids environ.

Le procédé mettant en oeuvre les ingrédients **a)** à **c)** tels que définis précédemment et éventuellement le peroxyde d'hydrogène ou système générateur de peroxyde d'hydrogène, et de l'eau ou la composition cosmétique selon l'invention comprenant les ingrédients **a)** à **c)** tels que définis précédemment et éventuellement le peroxyde d'hydrogène ou système générateur de peroxyde d'hydrogène et de l'eau peut également mettre en oeuvre ou comprendre une ou plusieurs bases d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

La ou les bases d'oxydation présentes dans la ou les compositions sont en général présentes chacune en quantité comprise entre 0,001 à 10 % en poids du poids total de la ou des compositions correspondantes.

La ou les composition(s) cosmétique(s) de l'invention peuvent se présenter sous des formes galéniques diverses, telles qu'une poudre, une lotion, une mousse, une crème, un gel ou sous tout autre forme appropriée pour réaliser une teinture des fibres kératiniques. Elles peuvent également être conditionnées en flacon pompe sans propulseur ou sous pression en flacon aérosol en présence d'un agent propulseur et former une mousse.

*pH de la ou des composition(s)*

Le procédé selon l'invention met en oeuvre les ingrédients **a)**, **b)** et **c)**, avec le pH final qui est basique ou alcalin soit supérieur à 7 et de préférence compris entre 8 et 12. Particulièrement compris entre 8 et 10,5. Il en est de même pour les compositions selon l'invention qui sont basiques ou alcalines et qui présentent un pH supérieur à 7, de préférence compris entre 8 et 12. Particulièrement compris entre 8 et 10,5.

Le pH de ces compositions peut être ajusté à la valeur désirée au moyen d'agents alcalinisants tels que définis précédemment dans **c)** ou d'agents acidifiants habituellement utilisés en teinture des fibres kératiniques ou bien encore à l'aide de systèmes tampons classiques.

Parmi les agents acidifiants des compositions utilisées dans l'invention, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants il s'agit des agents tels que définis précédemment dans « *c) agent(s) alcalinisant(s)* ».

*Procédé de coloration en une ou plusieurs étapes*

Selon un mode de réalisation particulier de l'invention le procédé de coloration est réalisé, en une ou plusieurs étapes, par application sur les fibres kératiniques d'une ou de plusieurs compositions cosmétiques contenant, pris ensemble ou séparément dans la ou les dites compositions, les ingrédients suivants:

- **a)** un ou plusieurs dérivé(s) d'orthodiphénol(s) tels que définis précédemment ;
- **b)** un ou plusieurs dérivé(s) métallique(s) tels que définis précédemment ;
- **c)** un ou plusieurs agent(s) alcalinisant(s) tels que définis précédemment ;

étant entendu que le pH à la fin du procédé est alcalin i.e. que le pH de la composition comprenant l'ingrédient **c)** est alcalin, soit supérieur à 7, de préférence compris entre 8 et 12. Particulièrement compris entre 8 et 10,5.

Le temps de pause entre les étapes d'application des compositions comprenant le ou les ingrédients a), b),

et/ou c) est fixé entre 3 et 120 minutes, préférentiellement entre 10 et 60 minutes et plus particulièrement entre 15 et 45 minutes.

Les fibres kératiniques peuvent être ou non préalablement humidifiées.

Plus particulièrement, dans le procédé de l'invention le ou les composé(s) c) se trouve(nt) soit en mélange avec a) et b) ; soit appliqué séparément après application d'une composition cosmétique comprenant les ingrédients a) et b) ; ou alors - soit appliqué ensemble avec l'ingrédient a) après application d'une composition cosmétique comprenant le ou les ingrédients b).

Un mode de réalisation particulier de l'invention concerne les procédés de coloration en une ou deux étapes.

Selon un mode de réalisation particulier de l'invention, le procédé de coloration de fibres kératiniques se fait en deux étapes.

Dans une variante de procédé en deux étapes la première étape consiste à appliquer sur les dites fibres une composition cosmétique comprenant les ingrédients a) et b), tels que définis précédemment, puis dans une deuxième étape une composition cosmétique comprenant l'ingrédient c) tel que défini précédemment est appliquée sur lesdites fibres, étant entendu qu'au moins une des deux compositions cosmétiques est aqueuse.

Selon un procédé particulièrement avantageux de l'invention l'ingrédient c) qui est appliqué sur les fibres comprend au moins un (bi)carbonate tel que défini précédemment. Plus particulièrement le (bi)carbonate se trouve dans une composition en présence d'une alcanolamine telle que la monoéthanolamine.

Préférentiellement, le procédé de coloration selon l'invention est réalisé en deux étapes dont la première étape est d'appliquer sur les fibres kératiniques les ingrédients a) et b) ensemble puis dans une deuxième étape d'appliquer ensemble les ingrédients c) puis éventuellement suivis d'étapes de post- traitement tel que le rinçage par exemple à l'eau, le shampoouinage avec un shampoing classique et/ou le séchage des fibres kératiniques.

Pour ces derniers procédés le temps de pause après application de la composition cosmétique pour la première étape est généralement fixé entre 3 et 120 minutes et préférentiellement entre 10 et 60 minutes et plus préférentiellement entre 15 et 45 minutes. Le temps de pause après application de la deuxième composition cosmétique pour la deuxième étape est généralement fixé entre 3 et 120 minutes et préférentiellement entre 3 et 60 minutes et plus préférentiellement entre 5 et 30 minutes.

Quelque soit le mode d'application, la température d'application est généralement comprise entre la température ambiante (15 à 25°C) et 80°C et plus particulièrement entre 15 et 45°C. Ainsi, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de lissage de la chevelure, un fer chauffant à une température comprise entre 60 et 220°C et de préférence entre 120 et 200°C.

Un mode particulier de l'invention concerne un procédé de coloration qui est réalisé à température ambiante (25 °C).

Dans tous les modes particuliers et variantes des procédés précédemment décrits les compositions évoquées sont des compositions prêtes à l'emploi qui peuvent résulter du mélange extemporané de deux ou plusieurs compositions et notamment de compositions présentes dans des kits de teintures.

*Dispositif ou « kit » de teinture :*

Un autre objet de l'invention est un dispositif à plusieurs compartiments ou « kit » de teinture. De façon avantageuse, ce kit comporte de 2 à 5 compartiments contenant de 2 à 5 compositions dans lesquels sont répartis les ingrédients **a)** un ou plusieurs dérivé(s) d'orthodiphénol(s), **b)** un ou plusieurs dérivés(s) métallique(s), c) un ou plusieurs agent(s) alcalinisant(s), et éventuellement un compartiment contient du peroxyde d'hydrogène ou un ou plusieurs système(s) générateur(s) de peroxyde d'hydrogène et/ou de l'eau lesdites compositions comprenant **a)**, **b)** et/ou **c)** étant aqueuses ou pulvérulentes, avec particulièrement au moins une de ces compositions étant aqueuse.

Les compositions du dispositif selon l'invention sont conditionnées dans des compartiments distincts, accompagnés, éventuellement, de moyens d'application appropriés, identiques ou différents, tels que les pinceaux, les brosses ou les éponges.

Le dispositif mentionné ci-dessus peut également être équipé d'un moyen permettant de délivrer sur les cheveux le mélange souhaité, par exemple tel que les dispositifs décrits dans le brevet FR 2 586 913.

## EXEMPLES DE COLORATION

### A) Résultats colorimétriques :

La coloration des cheveux est évaluée visuellement et lue au spectrocolorimètre Minolta (CM3600d, illuminant

D65, angle 10°, valeurs SCI) pour les mesures colorimétriques L*, a*, b*.

Dans ce système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est jaune.

La variation de coloration entre les mèches colorées de cheveux blancs permanentés non traités (témoin) et après traitement sont définis par ($\Delta E^*_{montée}$) selon l'équation suivante :

$$\Delta E^* montée = \sqrt{(L^* - L_o^*)^2 + (a^* - a_o^*)^2 + (b^* - b_o^*)^2}$$

Dans cette équation, L*, a* et b* représentent les valeurs mesurées après coloration des cheveux permanentés à 90% de blancs et $L^*_0$, $a_0^*$ et $b_0^*$ représentent les valeurs mesurées des cheveux permanentés à 90% de blancs non traités.

Plus la valeur de ΔE est importante, plus la différence de couleur entre les mèches témoins et les mèches colorées est importante.

**Protocole A** :

1. Phase de traitement avec les orthodiphénols/extraits :

Composition pour 100 g :

32 g eau
32 g éthanol
32 g propylène glycol
0,168 g de molécule ou d'extrait
$1{,}37.10^{-4}$ mol de dérivé métallique

Le traitement des fibres kératiniques est réalisé par application avec un rapport de bain 1 g de mèche pour 12 g de composition sur cheveux permanentés 90% blancs. Le temps de pause après application est de 30 minutes à l'étuve 50 °C sous papier aluminium.

Phase de révélation de la couleur :

Composition alcaline (pH 9,5) pour 100g

2,6 g de bicarbonate d'ammonium
2 g de mono éthanol amine
Qsp 100 g eau

Le traitement des fibres kératiniques est ensuite réalisé par application sur cheveux avec un rapport de bain de 1 g de cheveux pour 2,5 g de composition révélatrice. Le temps de pause après application est de 10 minutes à température ambiante.

2. Rinçage, shampooing et séchage.

Le traitement est terminé par un rinçage à l'eau, un shampouinage à l'aide d'un shampoing classique et un séchage au sèche cheveux.

**Protocole B** :

1. Phase de traitement avec les orthodiphénols/extraits :

Composition pour 100 g :

32 g eau
32 g éthanol

32 g propylène glycol
0,42 g de molécule ou d'extrait
1,37.$10^{-4}$ mol d'additif métallique

Le traitement des fibres kératiniques est réalisé par application avec un rapport de bain 1 g de mèche pour 12 g de composition sur cheveux permanentés 90% blancs. Le temps de pause après application est de 30 minutes à l'étuve 50 °C sous papier aluminium.

2. Phase de révélation :

Composition alcaline (pH 9,5) pour 100 g

2,6 g de bicarbonate d'ammonium
2 g de mono éthanol amine
Qsp 100 g eau

Le traitement des fibres kératiniques est ensuite réalisé par application sur cheveux avec un rapport de bain de 1 g de cheveux pour 2,5 g de composition révélatrice. Le temps de pause après application est de 10 minutes à température ambiante.

3. Rinçage, shampooing et séchage.

Le traitement est terminé par un rinçage à l'eau, un shampouinage à l'aide d'un shampoing classique et un séchage au sèche cheveux.

Résultats de ΔE :

i) avec comme ingrédient a) orthodiphénol la quercétine :

| Exemples | Dérivé Métallique b) | Protocole | $\Delta E_{montée}$ |
|---|---|---|---|
| 1 | $Na_2MoO_4$ - $2H_2O$ | A | 23,1 |
| 2 | $TiO_2$ | A | 18,0 |
| 3 | $ZrO_2$ | A | 19,3 |
| 4 | $Au_2O_3$ | B | 21,8 |
| 5 | $Au(OH)_3$ | B | 22,4 |
| 6 | AuOH | B | 22,1 |
| 7 | $SnO_2$ | B | 24,3 |
| 8 | $In_2O_3$ | B | 23,0 |
| 9 | $SiO_2$ | B | 22,8 |

ii) avec comme ingrédient a) orthodiphénol l'Hématéine :

| Exemples | Dérivé Métallique b) | Protocole | $\Delta E_{montée}$ |
|---|---|---|---|
| 11 | $Au_2O_3$ | B | 22,4 |
| 12 | $Au(OH)_3$ | B | 23,7 |
| 13 | AuOH | B | 23,7 |
| 14 | $SnO_2$ | B | 25,0 |
| 15 | $In_2O_3$ | B | 25,7 |
| 16 | $SiO_2$ | B | 25,3 |

iii) avec comme ingrédient a) orthodiphénol l'Hématoxyline :

| Exemples | Dérivé Métallique b) | Protocole | $\Delta E_{montée}$ |
|---|---|---|---|
| 18 | $KAuCl_4$ | B | 22,0 |

iv) avec comme ingrédient a) un extrait : l'extrait d'oignon :

| Exemples | Dérivé Métallique b) | Protocole | $\Delta E_{montée}$ |
|---|---|---|---|
| 19 | $Na_2MoO_4$ - $2H_2O$ | A | 18,0 |
| 20 | Tungstate de sodium - $2H_2O$ | A | 19,0 |

D'après les résultats supra des tableaux, il apparaît clairement que le procédé de coloration et la composition selon l'invention comprenant a) un orthodiphénol et extrait associé à b) un dérivé métallique en c) milieu alcalin permettent de colorer efficacement que ce soit sur les fibres kératiniques caractérisé notamment par une importante différence de couleur entre les mèches témoins avant traitement (BN) et les mèches colorées ($\Delta E_{montée}$ élevé).

**B) Essais comparatifs par rapport à EP0 664 114**

Protocole comparatif décrit dans EP 0 664 114 avec 4-méthylcatéchol (0,8 mmol) + résorcinol (0,4 mmol) + persulfate de sodium (0,8 mmol) + tampon bicarbonate + sel métallique (0,025 mmol), selon l'exemple 5 de EP 0 664 114 (p. 7 et 8) :
La coloration par couple de compositions a été évaluée. La première composition correspondant à celle de l'état de la technique dans les même conditions expérimentales (l'exemple 5 de EP 0 664 114) et la composition selon l'invention ne se distingue de celle de l'état de la technique que par le remplacement du sel de cuivre (acétate de cuivre) par le type de sel métallique selon l'invention (même nombre de mole que le comparatif = 0,025 mmol).
Le traitement des fibres kératiniques est réalisé par application avec un rapport de bain 1 g de mèche pour 5 g de composition sur cheveux sensibilisé avec une sensibilité alcaline de 25,6 (SA25,6). Le temps de pause après application est de 30 minutes à température ambiante.

**Résultats colorimétrique sur la chromaticité et l'homogénéité :**

Les valeurs de L, a, b sont mesurées par le spéctrocolorimètre Minolta (CM3600d, illuminant D65, angle 10°, valeurs SCI) pour les mesures colorimétriques L*, a*, b*.

Chromaticité : C*

La chromaticité dans le système CIE L*, a*, b* est calculée selon l'équation suivante :

$$C^* = \sqrt{a^{*2} + b^{*2}}$$

Plus la valeur de C* est élevée plus la coloration obtenue est chromatique

Homogénéité de la coloration ou sélectivité : $\Delta E^*_{sélect}$

La variation de coloration entre les mèches colorées de cheveux blancs naturels et les mèches permanentés ou sensibilisés (SA25,6) sont définis par ($\Delta E^*_{sélect}$) selon l'équation suivante :

$$\Delta E^* sélect = \sqrt{(L^* - L_o^*)^2 + (a^* - a_o^*)^2 + (b^* - b_o^*)^2}$$

Dans cette équation, L*, a* et b* représentent les valeurs mesurées après coloration des mèches permanentés ou sensibilisés (SA25,6) à 90% de blancs et $L^*_0$, $a_0^*$ et $b_0^*$ représentent les valeurs mesurées des cheveux naturels à 90% après coloration.

Plus la valeur de $\Delta E$ est importante, plus la différence de couleur entre les mèches naturelles et permanentées ou sensibilisées est visible reflétant ainsi l'effet de sélectivité de coloration entre la racine et la pointe. Moins, la valeur de $\Delta E_{select}$ est importante moins la coloration est sélective et plus la couleur est homogène. On parle également de coloration à « l'unison ».

*Cas avec sel métallique : Ag*

*Résultat avec l'acétate de cuivre selon l'état de la technique :*

- sur mèche grise à 90% blanc nature (90BN) L*a*b* : 44,48 / 10,17 / 28,85, $\Delta E$*montée = 23,6, chromaticité C* = 30.6
- sur mèche SA25,6 L*a*b* : 37,42 / 18,56 / 34,80, $\Delta E$* montée = 13,7, chromaticité C* = 39,4

*Résultats avec l'argent $Ag_2O$ : plus chromatique*

- sur mèche 90BN L*a*b* : 46,15 / 8,73 / 33,80, montée DE* = 25,6, chromaticité C* = 34,9
- sur mèche SA25,6 L*a*b* : 40,83 / 15,9 / 38,09, montée DE* = 12,3, chromaticité C* = 41,3

*Homogénéité ou sélectivité :*

$\Delta E_{sélect}$ = 12,47 (état de la technique)
$\Delta E_{sélect}$ = 9,90 (*$Ag_2O$*)

*Cas avec sels métalliques : W et Si*

Résultats avec $H_4SiW_{12}O_{40}$ : moins sélectif vs. $\Delta E_{sélect}$ = 12,47 (état de la technique)

- sur mèche 90BN L*a*b* : 49,76 / 6,04 / 23,14
- sur mèche SA25.6 L*a*b* : 45,12 / 11,72 / 31,75

soit $\Delta E_{sélect}$ = 11,30 ($H_4SiW_{12}O_{40}$)

*Cas avec sels métalliques : Au*

Résultats avec $KAuCl_4$: significativement moins sélectif vs. $\Delta E_{sélect}$ = 12,47 (état de la technique)

- sur mèche 90BN L*a*b* : 45,19 / 7,18 / 26,43
- sur mèche SA25.6 L*a*b* : 43,07 / 14,02 / 33,16

soit $\Delta E_{sélect}$ = 9,8 ($KAuCl_4$)

*Cas avec Mg*

Résultats avec chlorophylline de magnésium : significativement moins sélectif vs. $\Delta E_{sélect}$ = 12,47 (état de la technique)

- sur mèche 90BN L*a*b* : 47,96 / 5,61 / 27,68
- sur mèche SA25.6 L*a*b* : 43,77 / 11,92 / 33,43

SOit $\Delta E_{sélect}$ = 9,5

*Cas avec sels métalliques : V et Mo*

Résultats avec $H_5(PV_2Mo_{10}O_{40})$ : significativement moins sélectif vs. $\Delta E_{sélect}$ = 12,47 (état de la technique)

- sur mèche 90BN L*a*b* : 46.42/4.85/20.65
- sur mèche SA25.6 L*a*b* : 37.69/9.39/25.35

soit $\Delta E_{sélect}$ = 10.9 [$H_5(PV_2Mo_{10}O_{40})$]

*Cas avec sels métalliques : Ru*

Résultats avec $KRuO_4$: significativement moins sélectif vs. $\Delta E_{sélect}$ = 12,47 (état de la technique)

- sur mèche 90BN L*a*b* : 46,18/8,12/28,17
- sur mèche SA25.6 L*a*b* : 40,01/14,96/33,93

soit $\Delta E_{sélect}$ = 10.9 ($KRuO_4$)

*Protocole comparatif avec un autre type d'OrthodiPhénol : quercétine + bicarbonate + sel métallique* :

1. Phase de traitement avec les orthodiphénols/extraits :

Composition pour 100 g :

qsp 100 g eau
12,8 g éthanol
12,8 g propylène glycol
1 g de quercétine
$3.3.10^{-3}$ mol de dérivé métallique

Le traitement des fibres kératiniques est réalisé par application avec un rapport de bain 5g de composition pour 1 g de mèche sur cheveux permanentés 90% blancs. Le temps de pause après application est de 30 minutes sur plaque chauffante à 30 °C dans un papier aluminium.

2. Phase de révélation de la couleur :

Composition alcaline (pH 9,5) pour 100g :

2,6 g de bicarbonate d'ammonium
2 g de mono éthanol amine
Qsp 100 g eau

Le traitement des fibres kératiniques est ensuite réalisé par application sur cheveux avec un rapport de bain de 2,5 g de composition révélatrice pour 1 g de cheveux. Le temps de pause après application est de 10 minutes à température ambiante. Rinçage à l'eau, puis shampoing et séchage.

Résultats avec acétate de cuivre selon l'état de la technique :

- sur mèche 90BP L*a*b* : 49,90 / 8,14 / 36,82, $\Delta E^*_{montée}$ = 26.3, chromaticité C* = 37,7

Résultats avec le molybdène $Na_2MoO_4$ : significativement plus chromatique que l'état de la technique

- sur mèche 90BP L*a*b* : 44,86 / 15,88 / 41,36, $\Delta E^*_{montée}$ = 34.3, chromaticité C* = 44,3

Il apparaît donc que les colorations obtenues avec la composition selon l'invention ou le procédé selon l'invention comprenant un sel métallique différent de celui de l'état de la technique apportent de façon inattendue une chromaticité significativement plus forte, une plus faible sélectivité de coloration et/ou une plus forte montée de coloration que l'état de la technique.

## Revendications

1. Procédé de coloration est réalisé, en une ou plusieurs étapes, par application sur les fibres kératiniques d'une ou de plusieurs compositions cosmétiques contenant, pris ensemble ou séparément dans la ou les dites compositions, les ingrédients suivants :

   **a)** un ou plusieurs dérivé(s) d'orthodiphénol(s) différent(s) des dérivés à motif indoles ;
   **b)** un ou plusieurs dérivé(s) métallique(s) choisi(s) parmi les sels métalliques, complexes métalliques, oxydes métalliques, oxanions métalliques, leurs formes supportées, leurs hydrates et leur mélanges pour lesquels le ou les métal(aux) est (sont) choisi(s) parmi :

      **i)** l'or (Au) ;
      **ii)** le molybdène (Mo) ;
      **iii)** les oxydes d'argent (Ag) I et II, les sels d'Ag I et II choisis parmi les halogénures d'argent, le sulfate d'Ag, $\mathbf{[R^1\text{-}C(O)O]_nAg}$ avec n = 1 ou 2, $R^1$ représentant un groupe $(C_1\text{-}C_6)$alkyle ;
      **iv)** le tungstène (W),
      **v)** le vanadium (V),
      **vi)** le Ruthénium (Ru),
      **vii)** les métalloporphyrines de Mg II, les phtalocyanines de Mg II, les chlorophyllines de Mg II, les chlorophylles de Mg II,
      **ix)** le rhénium (Re),
      **x)** le titane (Ti),
      **xi)** le silicium (Si),
      **xii)** les oxydes d'étain
      **xiii)** le zirconium (Zr),
      **xiv)** le niobium (Nb),
      **xv)** l'indium (In),
      **xvi)** le sélénium (Se), et

   **c)** un ou plusieurs agent(s) alcalinisant(s) choisis parmi les alcanolamines et les (bi)carbonates ;

   étant entendu que le pH de la composition comprenant l'ingrédient **c)** est alcalin, soit supérieur à 7 et de préférence compris entre 8 et 12, particulièrement compris entre 8 et 10,5.

2. Procédé de coloration selon la revendication précédente dans lequel le ou les orthodiphénol(s) sont choisis parmi les dérivé(s) d'orthodiphénol(s) naturel(s).

3. Procédé de coloration selon la revendication 1 ou 2 dans lequel l'ingrédient i) est un orthodiphénol à cycle aromatique choisi parmi le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatiques comportant au moins deux groupes hydroxy portés par deux atomes adjacents contigus du cycle aromatique.

4. Procédé de coloration selon une quelconque des revendications précédentes dans lequel l'ingrédient **a)** est de formule (I) suivante ou l'un de ses oligomères, sous forme salifié ou non :

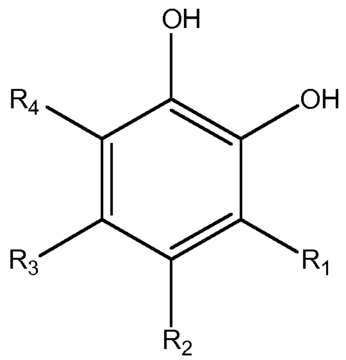

(I)

formule (I) dans laquelle les substituants :

• $R_1$ à $R_4$, identiques ou différents, représentent :

- un atome d'hydrogène,
- un atome halogène,
- un radical hydroxy,
- un radical carboxyle,
- un radical carboxylate d'alkyle ou alcoxycarbonyle,
- un radical amino éventuellement substitué,
- un radical alkyle linéaire ou ramifié éventuellement substitué,
- un radical alcényle linéaire ou ramifié éventuellement substitué,
- un radical cycloalkyle éventuellement substitué,
- un radical alcoxy,
- un radical alcoxyalkyle,
- un radical alcoxyaryle, le groupe aryle pouvant être éventuellement substitué,
- un radical aryle,
- un radical aryle substitué,
- un radical hétérocyclique, saturé ou non, porteur ou non d'une charge cationique ou anionique, éventuellement substitué et/ou éventuellement condensé avec un cycle aromatique, ledit cycle aromatique étant éventuellement substitué,
- un radical contenant un ou plusieurs atomes de silicium,

où deux des substituants portés par deux atome de carbone adjacents $R_1$ - $R_2$, $R_2$ - $R_3$ ou $R_3$ - $R_4$ forment conjointement un cycle saturé ou insaturé, aromatique ou non, substitué ou non, contenant éventuellement un ou plusieurs hétéroatomes et éventuellement condensé avec un ou plusieurs cycles saturés ou insaturés, éventuellement substitué, contenant éventuellement un ou plusieurs hétéroatomes.

**5.** Procédé de coloration selon la revendication précédente dans lequel le ou les orthodiphénol(s) sont choisis parmi :

- les flavonols,
- les anthocyanidines,
- les anthocyanines ou les anthocyanes ,
- les orthohydroxybenzoates,
- les flavones,
- les hydroxystilbènes,
- la 3,4-dihydroxyphénylalanine et ses dérivés,
- la 2,3-dihydroxyphénylalanine et ses dérivés,
- la 4,5-dihydroxyphénylalanine et ses dérivés,
- les dihydroxycinnamates,
- les orthopolyhydroxycoumarines,
- les orthopolyhydroxyisocoumarines,
- les orthopolyhydroxycoumarones,
- les orthopolyhydroxyisocoumarones,
- les orthopolyhydroxychalcones,
- les orthopolyhydroxychromones,
- les polyhydroxyquinones,
- les orthohydroxyxanthones,
- le 1,2 dihydroxybenzène et ses dérivés,
- le 1,2,4 trihydroxybenzène et ses dérivés,
- le 1,2,3 trihydroxybenzène et ses dérivés,
- le 2,4,5 trihydroxytoluène et ses dérivés,
- les proanthocyanidines,
- les proathocyanines,
- l'acide tannique,
- l'acide ellagique,
- et les mélanges des composés précédents.

**6.** Procédé de coloration selon une quelconque des revendications 2 à 5 dans lequel le ou les orthodiphénol(s) naturel(s) **a)** sont choisis parmi les extraits d'animaux, de bactéries, de champignons, d'algues, de plantes.

**7.** Procédé de coloration selon la revendication précédente dans lequel le ou les orthodiphénol(s) naturel(s) **a)** sont choisis parmi :

- les extraits de feuilles de thé, les extraits de feuilles de romarin et les extraits de feuilles de maté ;
- les extraits de fruits, tels que les extraits de raisin, les extraits fèves et/ou cabosses de cacaoyer ;
- les extraits de légumes, tels que les extraits de pelures d'oignon ;
- les extraits de bois d'arbres, tels que les extraits d'écorce de pin, les extraits de bois de campêche.

**8.** Procédé de coloration selon une quelconque des revendications précédentes dans lequel le ou les dérivé(s) métallique(s) **b)** sont choisis parmi :

**i)** le ou les dérivé(s) d'or choisi(s) parmi

a) des oxydes d'or I et III tel que $Au_2O_3$,
b) les hydroxydes d'Au I et III tels que $Au(OH)_3$, AuOH, Au(O)OH,
c) des sels d'or I particulièrement de formule AuHal avec Hal représentant un atome d'halogène tel que AuCl ou AuI,
d) des sels d'or III particulièrement choisi parmi les formules suivantes :

• **Au(Hal)**$_3$ avec Hal, identiques ou différents, tels que définis précédemment, comme $AuCl_3$, et $AuBr_3$,
• **ZAu(Hal)**$_4$, hydraté ou non, avec Z représentant un atome d'hydrogène, un métal alcalin tel que Li, Na, K ou un ammonium $NH_4^+$ et Hal, identiques ou différents, tels que définis précédemment, comme le $KAuCl_4$ ou $HAuCl_4$,
• **Au(R)**$_3$ avec R, identiques ou différents, représentant :

- un groupe ($C_1$-$C_6$)alkylcarbonyloxo, où le groupe alkyle est linéaire ou ramifié, tel que méthyle ou terbutyle tel que $Au(OAc)_3$,
- ou alors un ou deux des groupes R représentant un ligand L portant au moins un groupe électrodonneur tel que amino, phosphino, hydroxy, thiol, ou le ligand est un carbène « persistant » particulièrement de type « *Arduengo* » (Imidazol-2-ylidenes) ; préférentiellement le ligand est une phosphine telle que triphényl phosphine par exemple $(Ph_3P)AuOC(O)^tBu$,

e) des complexes d'Au tels que des métalloporphyrines d'Au I et III des phtalocyanines d'or I et III ou des chlorophyllines a ou b d'or I et III ;

particulièrement, le ou les dérivé(s) métallique(s) est (sont) choisi(s) parmi les oxydes et hydroxydes d'or I et III tels que $Au_2O_3$, $Au(OH)_3$, AuOH ;
**ii)** le ou les dérivé(s) d'argent choisi(s) parmi :

a) les oxydes d'argent I et II tels que $Ag_2O$ et AgO,
b) les sels d'Ag I particulièrement choisis parmi les formules suivantes :

• AgHal avec Hal représentant un atome d'halogène (F, Cl, Br, I) tel que AgCl, AgBr, AgI ;
• $Ag_xR^3_z$, avec $R^3$ identiques ou différents représentant :

▪ un groupe sulfate tels que $Ag_2SO_4$
▪ un groupe ($C_1$-$C_6$)alkylcarbonyloxo où le groupe alkyle est linéaire ou ramifié et peut éventuellement être substitué par un groupement hydroxyle, tel que l'acétate d'Ag, le propionate d'Ag, le lactate d'Ag
▪ $1 \leq z \leq 6$ et $1 \leq x \leq 4$ ;
▪ à l'exception du nitrate d'argent $Ag(NO_3)$ :

c) des métalloporphyrines d'Ag I ;
d) des phtalocyanines d'Ag I ;
e) des chlorophyllines a ou b d'Ag I ;

**iii)** le ou les dérivé(s) de molybdène (Mo) (II) à (VI) choisis parmi :

a) des oxydes de Mo (VI) tels que :

- le trioxide de Mo de formule **$MoO_3$** ;
- les oxydes de Mo (IV) à ligand *β*-dicétones **$MoO_2L_2$** avec L ligand, identiques ou différents, préférentiellement identiques représentent une *β*-dicétone de type **R-C(X)-C(R')-C(X)R"** avec R et R", identiques ou différents, représentent un groupe ($C_1$-$C_6$)alkyle, linéaire ou ramifié, et R' représente un atome d'hydrogène ou un groupe ($C_1$-$C_6$)alkyle, linéaire ou ramifié, X représente un atome d'oxygène, de soufre ou un groupe N(R) avec R représentant un atome d'hydrogène ou un groupe ($C_1$-$C_6$)alkyle, linéaire ou ramifié ;
- les complexes d'oxyde de Mo(VI) provenant du $MoO_3$ et de ligand hydroxyalkylacide carboxylique en $C_2$-$C_{10}$ ou de polyols en $C_2$-$C_{10}$ comprenant de 2 à 5 groupes hydroxy notamment l'éthylène glycol, glycérol ;
- le monohydrate, **$MoO_3 \cdot H_2O$** le dihydrate, **$MoO_3 \cdot 2H_2O$**, l'acide molybdique **($H_2MoO_4 \cdot H_2O$)** ;
- le dioxide de Molybdène ;
- dihalogénodioxomolybdène **$(Hal)_2MoO_2$** avec Hal identiques ou différents est tel que défini précédemment, particulièrement Hal sont identiques et représentent un atome de chlore ;
- les molybdènes bleus ou « molybdenum blues » mélange d'oxyde de Mo / hydroxydes de Mo(VI) et Mo(V) ; particulièrement $Mo^{6+}{}_3 Mo^{5+}{}_3 O_{18}H$, et les dérivés de condensation d'avec les ions phosphates ;
- les mélanges d'oxydes de Mo à valences différentes Mo(VI)-Mo(V) (les bronzes d'oxyde de Mo) ; plus particulièrement les bronzes binaires et les bronzes tertiaires, **$A_{0.33}MoO_3$** (A = Li, K, Rb, Cs, Tl), ; **$A_{0.3}MoO_3$** (A = K, Rb, Tl) ; **$A_{0.9}Mo_8O_{17}$** (A = Li, Na, K, Tl), et les bronzes à terre rares $La_2Mo_2O_7$;.

b) des oxanions de Mo choisi parmi les molybdates **$Z_2MoO_4$** avec Z, identiques ou différents, tels que définis précédemment ;
c) des polyoxométallates de formule : **$[XY_uMo_{12-u}O_{40}]^{(3+u)-}(Z)_{(3+u)}$** avec X et Y choisis parmi P, Si, V ; $0 \leq u \leq 6$, et Z est tel que défini précédemment ou Z représente un atome d'hydrogène, particulièrement le polyoxométallate est de formule $H_5PV_2Mo_{10}O_{40}$,
d) les halogénures de Mo binaires d'états d'oxydation (II) à (VI), hexacoordiné par 6 halogènes de Mo(V), (IV) et (III), les atomes de Mo étant lié par des liaisons halogène tel que l'halogénure de Mo(II) contenant des clusters $[Mo_6Hal'_8]^{4+}$ liés à des atomes d'halogène pour donner **$Mo_6Hal_{12}$** avec Hal, identiques ou différents, tels que défini précédemment ;
e) les tétrahalogénures de molybdène **$(Hal)_4Mo$** avec Hal, identiques ou différents, tels que définis précédemment, tel que $MoCl_4$;
f) les dérivés soufrés du Mo choisis parmi :

- les disulfures de Molybdenum [*1317-33-5*], sulfures de molybdenum(IV), $MoS_2$ les molybdates de formule **$(Z)_2MoS_4$**, avec Z, identiques ou différents, tels que défini précédemment ;
- les sesquisulfure de Mo ; les trisulfures de dimolybdène (III), $Mo_2S_3$;
- les sels de tétrasulfures **$Z\ MoS_{24}^-$** avec Z tel que défini précédemment,;

g) les dérivés d'Oxomolybdène (VI) choisis parmi :

- **$Mo(O)Hal_4$** avec Hal, identiques ou différents, sont tels que définis précédemment ;
- ,**$Mo(O)_2Hal_2$** avec Hal, identiques ou différents, sont tels que définis précédemment ;

h) les dérivés trihalogénooxomolybdène(V) et ces adduits avec des ligands organiques L tels que définis précédemment, L représente préférentiellement :

- un groupe bidendate R-C(X)-CR'R"-C(X)-R'" avec R et R"", identiques ou différents, représentant un groupe ($C_1$-$C_6$)alkyle, linéaire ou ramifié et R' et R", identiques ou différentes représentent un atome d'hydrogène ou un groupe ($C_1$-$C_6$)alkyle, linéaire ou ramifié, préférentiellement R' et R" représentent un atome d'hydrogène, X représente un atome d'oxygène, de soufre, ou un groupe N(R) avec R représentant un atome d'hydrogène ou groupe ($C_1$-$C_6$)alkyle, linéaire ou ramifié, tel que l'acétylacétone ;
- un groupe bidendate de type 2,2-bipyridyle ;

i) le dérivé d'Oxomolybdène de formule **$Mo(O)Hal_3{\cdot}2\ L$** avec L et Hal tels que définis précédemment, préférentiellement L représente un R"-O-R' avec R et R' tels que définis précédemment, tel que diéthyléther et un groupe hétéroaryle tel que pyridine ;
j) les molybdates, isopolymolybdates, et hétéropolymolybdates contenant un anion tétraédrique $[MoO_4]^{2-}$ tels que l'ammonium heptamolybdate (isopolymolybdate), $(NR'_4)_6Mo_7O_{24}$ hydraté, avec R' identiques ou différents sont tels que définis précédemment, particulièrement R' est un atome d'hydrogène ;
k) les molybdates de cations divalents notamment ceux solubles dans l'eau tels que les molybdates du $Mg^{2+}$ et les molybdates de cations trivalents notamment de formules **$A_2(MoO_4)_3$** ou **$A_2Mo_3O_{12}$**, avec A représentant un atome choisi parmi Al, Cr, Bi et Lanthanide ;
l) les hétéropolymolybdates à octaèdre **$[MoO_6]$** incorporant d'autres hétéroatomes que l'atome d'oxygène choisi parmi notamment S, N et P, Plus spécifiquement les hétéromolybdates sont de formule **$[X^+{}_nMo_{12}O_{40}]^{(8-n)-}$**, à hétéroatomes (X) tétracoordinés,
m) les complexes des Mo avec des ligands organosoufrés tels que les phosphorodithioates ou dithiophosphates et dithiocarbamates, **$[Mo_2O_3L_4]$** et **$[Mo_2O_2S_2L_2]$** avec L tel que défini précédemment ;
n) l'hexacarbonyl de molybdène $Mo(CO)_6$ ;
o) les pigments organiques dérivés de molybdate de métal alcalin et alcalino-terreux tel que dérivés du molybdate de sodium tels que dérivés de i) Diarylméthane ii) Triarylméthane ; iii) Xanthène; et
p) les molybdates de Ca et de Sr ;
particulièrement, le dérivé métallique est choisi parmi les composés de formule $Z_2MoO_4$,

**iv)** le ou les dérivé(s) métallique(s) de tungstène (W) choisi(s) parmi a) les oxydes de tungstène VI, b) les oxanions de tungstène préférentiellement les tungstates de métal alcalin **$Z_2WO_4$**, hydraté ou non, avec Z, identiques ou différents, tels que définis précédemment, c) les polyoxométallates tels que **$[XY_uW_{12-u}O_{40}]^{(4+u)-};(Z)_{(4+u)}$** avec X et Y choisis parmi P, Si, V ; $0 \leq u \leq 6$, particulièrement le polyoxométallate est de formule $H_4SiW_{12}O_{40}$ ;
préférentiellement, le dérivé métallique est choisi les tungstates de métal alcalin ;
**v)** le ou les dérivé(s) métallique(s) de vanadium (V) choisi(s) parmi a) les oxydes de vanadium tel que le $V_2O_5$, b) les oxanions de vanadium choisis parmi les vanadates et métavanadates tels que l'acéthylacétonate d'oxyde de vanadium $VO(acac)_2$, $VOSO_4$, ammonium vanadate et c) les polyoxométallates tels que **$[XV_uM_{12-u}O_{40}]^{q-};(Z)_q$** avec M = W, Mo ; X = P, Si ; $0 \leq u \leq 6$ et q=3+x si M=Mo ou q=4+x si M=W et Z est tel que défini précédemment, particulièrement le polyoxométallate est de formule $H_5PV_2Mo_{10}O_{40}$ ; préférentiellement, le dérivé métallique est choisi parmi ceux de formule **$[XV_uMo_{12-u}O_{40}]^{(3+u)};(Z)_{(3+u)}$** telle que définie précédemment et particulièrement $H_5PV_2Mo_{10}O_{40}$ ;
**vi)** le ou les dérivé(s) métallique(s) de ruthénium (Ru) choisi(s) parmi a) les oxydes de ruthénium, b) les oxanions de ruthénium tels que les perruthénates de métal alcalin, et c) les complexes de ruthénium tels que **$(Hal)_2RuL_4$** avec Hal identiques ou différents, tels que définis précédemment et L, identiques ou différents, sont des ligands tels que définis précédemment ; préférentiellement, le dérivé métallique est choisi parmi $RuCl_2(PPh_3)_4$, le perruthénate de potassium ;
**vii)** les métalloporphyrines de magnésium II, d) les phtalocyanines de magnésium II, e) les chlorophyllines de magnésium II, f) les chlorophylles de magnésium II,
**ix)** le ou les dérivé(s) métallique(s) de Rhénium (Re) choisi parmi $R'ReO_3$ avec R' représentant un atome d'hydrogène ou un groupe $(C_1\text{-}C_6)$alkyle, linéaire ou ramifié, tel que $CH_3ReO_3$ ou les complexes de Re tels que les phtalocyanines ;
**x)** le ou les dérivé(s) métallique(s) de titane (Ti) choisi(s) parmi : a) les sels de titane IV tels que le $Ti(SO_4)_2$, b) les oxydes de titane les oxydes de titane ainsi que leurs sels, leurs hydrates et leurs formes supportées notamment choisi(s) parmi l'hydroxide de Titane (III) $Ti(OH)_3$ et $TiO_3$, le trioxide de dititane $Ti_2O_3$, les trioxydes de titane de métal alcalinoterreux, les pentoxides de titane de métal alcalinoterreux, les titanates de formule générale **$M^{II}TiO_4$** dans laquelle $M^{II}$ représente un métal Mg, Zn, Mn ou Co, l'acide peroxytitanique et les peroxytitanates $H_4TiO_5$, le dioxyde de titane (II) $TiO_2$, le disulfure de titane $TiS_2$, et c) les complexes de Ti tels que les phtalocyanines, préférentiellement le ou les dérivé(s) métallique(s) est (sont) $TiO_2$ ;
**xi)** le ou les dérivé(s) métallique(s) de silicium (Si), plus particulièrement de l'oxyde de silicium tel que $SiO_2$ ;
**xii)** le dérivé métallique est (sont) des oxydes d'étain ainsi que leurs sels, leurs hydrates et leurs formes supportées tels que l'oxyde d'étain (II) Hydraté de type 5 $SnO{\cdot}2H_2O$ et l'oxyde d'étain (II), l'oxyde d'étain (IV) hydraté $SnO_2{\cdot}nH_2O$ et l'oxyde d'étain (IV) $SnO_2$, les sels de métal alcalin tel que le sodium et potassium d'hydroxyde d'étain de formule **$M_2\ [Sn(OH)_6]$** avec M représentant un métal alcalin, les hydroxydes d'étain de formule **$R_3SnOH$**, **$R_2SnOH_2$**, ou **$RSnOH_3$** avec, R qui représente un groupe hydrocarboné tel que $(C_1\text{-}C_6)$alkyle, linéaire ou ramifié, ou $(C_1\text{-}C_6)$alkoxy, linéaire ou ramifié, (di) $(C_1\text{-}C_6)$alkylamino, préférentiellement le ou les oxyde(s) métallique(s) est(sont) de l'oxyde d'étain (IV) $SnO_2$ ;

**xiii)** le ou les dérivé(s) métallique(s) est (sont) du zirconium (Zr) particulièrement choisi(s) parmi :

a) les oxyde de titane ainsi que leurs sels, leurs hydrates et leurs formes supportées tels que l'oxyde de Zirconium [*1314-23-4*], $ZrO_2$, l'hydrate d'oxyde de zirconium $[Zr_4(OH)_8{\cdot}16H_2O]_8$, le dihalogénure d' Hydroxide de zirconium comme le dichloride d'hydroxyde de Zirconium [*22196-48-1*], ainsi que les composés de formule $Zr(OH)_2Hal_2 \cdot 7H_2O$, avec Hal représentant un atome d'halogène tel que le chlore, les oxyhalogénure de zirconium zirconium tel que l'oxychlorure de zirconium,les oxides d'halogénure de zirconium tel que dichlorure d'oxyde de zirconium, $ZrOCl_2 \cdot 8H_2O$, $[Zr_4(OH)_8 \cdot 16H_2O]Cl_8 \cdot 12H_2O$ et le monohalogénure de zirconium tel que le monochlorure d'hydroxide de zirconium $[Zr_4(OH)_{12} \cdot 16H_2O]Cl_4$ ; et
b) et les complexes de Zr tels que les phtalocyanines, préférentiellement le ou les dérivés(s) métallique(s) est(sont), l'oxyde de zirconium (II) le $ZrO_2$ ;

**xiv)** le ou les dérivé(s) métallique(s) est (sont) du niobium (Nb) particulièrement choisi(s) parmi les oxydes de niobium ainsi que leurs sels, leurs hydrates et leurs formes supportées tels que le pentoxyde de niobium $Nb_2O_5$ [*1313-96-8*], le trioxyde de niobium de métal alcalin comme le trioxyde de niobium de lithium $LiNbO_3$ [12031-63-9] ou $KNbO_3$ [12030-85-2] et les oxyhalogénures de Niobium tel que l'oxychlorure de chlore [*13597-20-1*], $NbOCl_3$ ;
**xv)** le ou les dérivé(s) métallique(s) est (sont) le ou les dérivé(s) métallique(s) est (sont) de l'indium (In) ainsi que leurs sels, leurs hydrates et leurs formes supportées particulièrement choisi(s) parmi les oxyde d'indium tels que l'oxyde de Indium(III) de formule $In_2O_3$, l'oxyde d'Indium(II) [12136-26-4] de formule InO, et l'oxyde d'indium(I) [12030-22-7] de formule $In_2O$, l'hydroxyde d'Indium [56108-30-6], de formule $In(OH)_3$, préférentiellement l'oxyde d'indium (III) $In_2O_3$; et
**xvi)** le ou les dérivé(s) métallique(s) est (sont) du $SeO_2$

**9.** Procédé de coloration selon une quelconque des revendications précédentes dans lequel le ou les dérivé(s) métallique(s) b) est (sont) choisi(s) parmi **i)** l'or (Au), **ii)** l'argent (Ag), **iii)** le molybdène (Mo), **iv)** le tungstène (W), **v)** le vanadium (V), **vi)** le ruthénium (Ru), **xi)** le silicium (Si) ; particulièrement le ou les dérivé(s) métallique(s) b) est (sont) choisi(s) parmi les composés de formule $ZAu(Hal)_4$ tel que $KAuCl_4$ ; $Au(R)_3$ $Au_2O_3$ ; $Z_2MoO_4$ ; $Z_2WO_4$ ; $[XY_uMo_{12-u}O_{40}]^{q-}(Z)_q$ et $H_5(PV_2Mo_{10}O_{40})$, $(Hal)_2RuL_4$ tels que définis dans la revendication précédente ; chlorophylline de magnésium ; $KRuO_4$, $H_4SiW_{12}O_{40}$ et $Ag_2O$.

**10.** Procédé de coloration selon une quelconque des revendications précédentes dans lequel le ou les dérivé(s) métallique(s) b) est (sont) de l'or, particulièrement, le ou les dérivé(s) métallique(s) b) est (sont) de l'or (Au) à l'exception des sels d'or.

**11.** Procédé de coloration selon une quelconque des revendications précédentes dans lequel le ou les agent(s) alcalinisant(s) c) est (sont) choisi(s) parmi les alcanolamines et les (bi)carbonates alcalins ou alcalino-terreux.

**12.** Procédé de coloration selon une quelconque des revendications précédentes qui ne met pas en oeuvre d'agent oxydant chimique autre que l'oxygène de l'air.

**13.** Procédé de coloration selon une quelconque des revendications 1 à 11 qui met en oeuvre un ou plusieurs oxydant(s) chimique(s) tel(s) que :

a) l'ozone ;
b) les persels de métal alcalin ou d'ammonium quaternaire ;
c) les peracides organiques aliphatiques en $C_1$-$C_6$ et aromatiques en $C_6$-$C_{20}$ et leurs formes percarboxylates, tels que l'acide performique, l'acide peracétique, les dérivés d'acides perbenzoïques, l'acide trifluoroacétique, l'acide peroxyphtalique, l'acide peroxymaléique, l'acide peroxypropionique ;
d) les peroxydes organiques,
e) les anions oxydants tels que les nitrites, les nitrates, les hypochlorites, hypobromites, hypoiodites, les chlorites, bromites, iodites, les chlorates, bromates, iodates, les periodates, particulier l'oxydant est choisi parmi l'hypochlorite ou le periodate de métal alcalin tel que l'hypochlorite de sodium ou le périodate de sodium ;.
f) les radicaux stables N-Oxy (NO·), tels que le radical 2,2,6,6-tétra($C_1$-$C_6$)alkylpipéridino-oxy, 2,2,6,6-tétra($C_1$-$C_6$)alkylmorpholino-oxy les sels de Frémy nitrosodisulfonates, la N-oxyde morpholine ; particulièrement l'oxydant est choisi parmi le radical 2,2,6,6-tétraméthylpipéridinyloxy
g) les dérivés hypervalents de l'iode, tels que l'iodotriacétate, l'iodosobenzène, l'iodobenzènetriacétate, les dérives d'acide iodoperbenzoiques, les périodinanes, les alkyls et benzoylhypoiodites
h) les composés organiques suivants : N-halogénosuccinimides, l'acide trichloroisocyanurique, la N-hydroxyphtalimide, les nitrites d'alkyles
i) les peroxyde d'hydrogène ou système(s) générateur(s) de peroxyde d'hydrogène tel(s) que :

i-1) le peroxyde d'urée ;
i-2) les complexes polymériques pouvant libérer du peroxyde d'hydrogène tels que le polyvinylpyrrolidone/$H_2O_2$ ;
i-3) les oxydases produisant du peroxyde d'hydrogène en présence d'un substrat adéquat (par exemple le glucose dans le cas de glucose oxydase ou l'acide urique avec l'uricase) ;
i-4) les peroxydes de métaux générant dans l'eau du peroxyde d'hydrogène comme le peroxyde de calcium, peroxyde de magnésium;

i-v) les perborates ; ou
i-vi) les percarbonates.

**14.** Procédé selon une quelconque des revendications précédentes en une étape consistant à appliquer sur les fibres kératiniques une composition aqueuse comprenant **a)**, **b)**, et **c)** tels que définis dans une quelconque des revendications précédentes et éventuellement un ou plusieurs agent(s) oxydant(s) chimique(s) tel(s) que défini(s) dans la revendication précédente.

**15.** Procédé de coloration selon une quelconque des revendications 1 à 11 en deux étapes consistant dans la première étape à appliquer sur les fibres kératiniques une composition comprenant les ingrédients **a)** et **b)** tels que définis dans une quelconque des revendications 1 à 10 et éventuellement un ou plusieurs agent(s) oxydant(s) chimique(s) tel(s) que défini(s) dans la revendication 14, puis dans une deuxième étape consistant à appliquer une composition comprenant **c)** tel que défini dans les revendications 1, ou 11 particulièrement à un pH supérieur à 7 et de préférence compris entre 8 et 12.

**16.** Procédé de coloration selon une quelconque des revendications 1 à 11 et 13 réalisé en au moins deux étapes se terminant par le traitement des fibres kératiniques avec l'ingrédient **c)** tel que défini dans les revendications 1, ou 11 et peut être suivi d'étapes de post- traitement tel que d'un shampoouinage à l'aide de shampoing classique, d'un rinçage par exemple à l'eau et/ou du séchage des fibres kératiniques par traitement thermique.

**17.** Procédé de coloration selon une quelconque des revendications précédentes mettant en oeuvre une ou plusieurs photoirradiation(s) par une ou plusieurs onde(s) électromagnétique(s) de longueur d'onde comprise entre 10 nm dans le domaine de l'UV et 100 $\mu$m dans le domaine de l'infrarouge IR qui proviennent particulièrement de la photoirradiation artificielle notamment de lampes émettant dans l'UV, des lampes à incandescences, à halogène, fluorescentes, à mercure, à faible pression, des lampes à faible pression par exemple à sodium ou à néon, des lampes à forte pression par exemple à mercure, des lampes à halogénures, des lampes flash par exemple au xénon, des lampes à excimer fluorescent telle que celle à xénon, les Light Emitting Diodes ou LED de 50 à 1000mW, les lampes émettant la lumière noire ou lumière de Wood, et les lasers

**18.** Composition cosmétique pour la coloration des fibres kératiniques contenant :

**a)** un ou plusieurs dérivé(s) d'orthodiphénol(s), tel(s) que défini(s) dans une quelconque des revendications 1 à 7 ;
**b)** un ou plusieurs dérivé(s) métallique(s) tel(s) que défini(s) dans une quelconque des revendications 1, 8 à 10 ;
**c)** un ou plusieurs agent(s) alcalinisant(s) tel(s) que défini(s) dans une quelconque des revendications 1, ou 11; et éventuellement
**d)** un ou plusieurs agent(s) oxydant(s) chimique(s) tel(s) que défini(s) dans la revendication 13.

**19.** Composition selon la revendication précédente dans laquelle le ou les dérivé(s) métallique(s) b) est (sont) est (sont) de l'or (Au) tel que défini dans une quelconque des revendications 1, 8 à 10 et éventuellement un ou plusieurs agent(s) oxydant(s) chimique(s) tel(s) que défini(s) dans la revendication 13.

**20.** Utilisation pour la coloration des fibres kératiniques tels que les cheveux de l'association suivante :

**a)** un ou plusieurs dérivé(s) d'orthodiphénol(s), tel(s) que défini(s) dans une quelconque des revendications 1 à 7 ;
**b)** un ou plusieurs dérivé(s) métallique(s) tel(s) que défini(s) dans une quelconque des revendications 1, 8 à 10 ;
**c)** un ou plusieurs agent(s) alcalinisant(s) tel(s) que défini(s) dans une quelconque des revendications 1, ou 11 et éventuellement

**d)** un ou plusieurs agent(s) oxydant(s) chimique(s) tel(s) que défini(s) dans la revendication 13.

**21.** Utilisation pour la coloration des fibres kératiniques tels que les cheveux selon la revendication précédente **caractérisée en ce que** b) le ou les dérivé(s) métallique(s) est (sont) un ou plusieurs dérivé(s) d'or tel(s) que défini(s) dans une quelconque des revendications 1, 8 à 10.

**Patentansprüche**

**1.** Färbeverfahren, das in einem oder mehreren Schritten durch Aufbringen einer oder mehrerer kosmetischer Zusammensetzungen, die zusammengenommen oder separat in der Zusammensetzung bzw. den Zusammensetzungen die folgenden Bestandteile enthalten, auf die Keratinfasern durchgeführt wird:

a) ein oder mehrere ortho-Diphenolderivate, die von Derivaten mit Indol-Einheit verschieden sind;
b) ein oder mehrere Metallderivate, die aus Metallsalzen, Metallkomplexen, Metalloxiden, Metall-Oxoanionen, geträgerten Formen davon, Hydraten davon und Mischungen davon ausgewählt sind, für die das Metall bzw. die Metalle aus:

i) Gold (Au),
ii) Molybdän (Mo),
iii) Oxiden von Silber(Ag) (I) und Silber(Ag)(II), Salzen von Ag(I) und Ag(II), die aus Silberhalogeniden, Ag-Sulfat und $[R^1\text{-}C(O)O]_nAg$ mit n = 1 oder 2 ausgewählt sind, wobei $R^1$ für eine $(C_1\text{-}C_6)$Alkylgruppe steht,
iv) Wolfram (W),
v) Vanadium (V),
vi) Ruthenium (Ru),
vii) Mg(II)-Metalloporphyrinen, Mg(II)-Phthalocyaninen, Mg(II)-Chlorophyllinen und Mg(II)-Chlorophyllen,
ix) Rhenium (Re),
x) Titan (Ti),
xi) Silicium (Si),
xii) Zinnoxiden,
xiii) Zirconium (Zr),
xiv) Niob (Nb),
xv) Indium (In) und
xvi) Selen (Se)

ausgewählt ist bzw. sind; und
c) ein oder mehrere Alkalisierungsmittel, die aus Alkanolaminen und (Hydrogen)carbonaten ausgewählt sind;

mit der Maßgabe, dass der pH-Wert der Zusammensetzung, die den Bestandteil c) umfasst, alkalisch ist, d. h. größer als 7 ist und vorzugsweise zwischen 8 und 12 und insbesondere zwischen 8 und 10,5 liegt.

**2.** Färbeverfahren nach dem vorhergehenden Anspruch, bei dem man das ortho-Diphenol bzw. die ortho-Diphenole aus natürlichen ortho-Diphenolderivaten auswählt.

**3.** Färbeverfahren nach Anspruch 1 oder 2, bei dem es sich bei dem Bestandteil i) um ein ortho-Diphenol mit einem aromatischen Ring, der aus Benzol, Naphthalin, Tetrahydronaphthalin, Indan, Inden, Anthracen, Phenanthren, Isoindol, Indolin, Isoindolin, Benzofuran, Dihydrobenzofuran, Chroman, Isochroman, Chromen, Isochromen, Chinolin, Tetrahydrochinolin und Isochinolin ausgewählt ist, handelt, wobei der aromatische Ring mindestens zwei Hydroxylgruppen an zwei aneinandergrenzenden benachbarten Atomen des aromatischen Rings umfasst.

**4.** Färbeverfahren nach einem der vorhergehenden Ansprüche, bei dem der Bestandteil a) die folgende Formel (I) aufweist oder ein Oligomer davon ist, gegebenenfalls in versalzter Form:

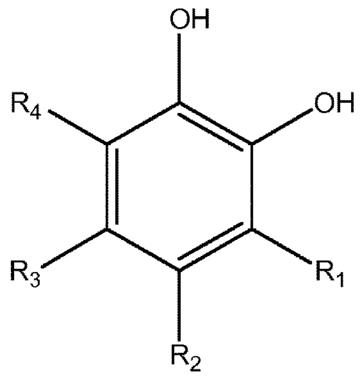

(I)

wobei in Formel (I) die Substituenten:

- $R_1$ bis $R_4$ gleich oder verschieden sind und für:

  - ein Wasserstoffatom,
  - ein Halogenatom,
  - einen Hydroxylrest,
  - einen Carboxylrest,
  - einen Alkylcarboxylat- oder Alkoxycarbonylrest,
  - einen gegebenenfalls substituierten Aminorest,
  - einen gegebenenfalls substituierten linearen oder verzweigten Alkylrest,
  - einen gegebenenfalls substituierten linearen oder verzweigten Alkenylrest,
  - einen gegebenenfalls substituierten Cycloalkylrest,
  - einen Alkoxyrest,
  - einen Alkoxyalkylrest,
  - einen Alkoxyarylrest, wobei die Arylgruppe gegebenenfalls substituiert sein kann,
  - einen Arylrest,
  - einen substituierten Arylrest,
  - einen gesättigten oder ungesättigten heterocyclischen Rest, der gegebenenfalls eine kationische oder anionische Ladung trägt, gegebenenfalls substituiert ist und/oder gegebenenfalls mit einem aromatischen Ring kondensiert ist, wobei der aromatische Ring gegebenenfalls substituiert ist,
  - einen Rest, der ein oder mehrere Siliciumatome enthält,

  stehen,

wobei zwei der Substituenten an zwei benachbarten Kohlenstoffatomen $R_1$-$R_2$, $R_2$-$R_3$ oder $R_3$-$R_4$ zusammen einen gesättigten oder ungesättigten, aromatischen oder nichtaromatischen, substituierten oder unsubstituierten Ring bilden, der gegebenenfalls ein oder mehrere Heteroatome enthält und gegebenenfalls mit einem oder mehreren gesättigten oder ungesättigten, gegebenenfalls substituierten Ringen, die gegebenenfalls ein oder mehrere Heteroatome enthalten, kondensiert ist.

**5.** Färbeverfahren nach dem vorhergehenden Anspruch, bei dem man das ortho-Diphenol bzw. die ortho-Diphenole aus:

- Flavonolen,
- Anthocyanidinen,
- Anthocyaninen oder Anthocyanen,
- ortho-Hydroxybenzoaten,
- Flavonen,
- Hydroxystilbenen,
- 3,4-Dihydroxyphenylalanin und Derivaten davon,
- 2,3-Dihydroxyphenylalanin und Derivaten davon,
- 4,5-Dihydroxyphenylalanin und Derivaten davon,
- Dihydroxycinnamaten,
- ortho-Polyhydroxycumarinen,

- ortho-Polyhydroxyisocumarinen,
- ortho-Polyhydroxycumaronen,
- ortho-Polyhydroxyisocumaronen,
- ortho-Polyhydroxychalconen,
- ortho-Polyhydroxychromonen,
- Polyhydroxychinonen,
- ortho-Hydroxyxanthonen,
- 1,2-Dihydroxybenzol und Derivaten davon,
- 1,2,4-Trihydroxybenzol und Derivaten davon,
- 1,2,3-Trihydroxybenzol und Derivaten davon,
- 2,4,5-Trihydroxytoluol und Derivaten davon,
- Proanthocyanidinen,
- Proanthocyaninen,
- Tanninsäure,
- Ellaginsäure
- und Mischungen der vorstehenden Verbindungen auswählt.

**6.** Färbeverfahren nach einem der Ansprüche 2 bis 5, bei dem man das natürliche ortho-Diphenol bzw. die natürlichen ortho-Diphenole a) aus Tier-, Bakterien-, Pilz-, Algen- und Pflanzenextrakten auswählt.

**7.** Färbeverfahren nach dem vorhergehenden Anspruch, bei dem man das natürliche ortho-Diphenol bzw. die natürlichen ortho-Diphenole a) aus:

- Teeblattextrakten, Rosmarinblattextrakten und Mateblattextrakten;
- Fruchtextrakten, wie Traubenextrakten oder Kakaobohnen- und/oder Kakaoschotenextrakten;
- Gemüseextrakten, wie Zwiebelschalenextrakten;
- Baumholzextrakten, wie Kieferrindenextrakten und Kampeschenholzextrakten;

auswählt.

**8.** Färbeverfahren nach einem der vorhergehenden Ansprüche, bei dem man das Metallderivat bzw. die Metallderivate b) aus:

i) dem Goldderivat bzw. den Goldderivaten, das bzw. die aus

a) Gold(I)- und Gold(III)-oxiden wie $Au_2O_3$,
b) Au(I)- und Au(III)-Hydroxiden wie $Au(OH)_3$, AuOH oder Au(O)OH,
c) Gold(I)-Salzen, insbesondere der Formel AuHal, wobei die Variablen Hal für ein Halogenatom stehen, wie AuCl oder AuI,
d) Gold(III)-Salzen, insbesondere ausgewählt aus den folgenden Formeln:

• $Au(Hal)_3$, wobei die Variablen Hal gleich oder verschieden sind und wie oben definiert sind, wie $AuCl_3$ und $AuBr_3$,
• gegebenenfalls hydratisiertem $ZAu(Hal)_4$, wobei Z für ein Wasserstoffatom, ein Alkalimetall wie Li, Na, K oder ein Ammonium $NH_4^+$ steht und die Variablen Hal gleich oder verschieden sind und wie oben definiert sind, wie $KAuCl_4$ oder $HAuCl_4$,
• $Au(R)_3$, wobei R gleich oder verschieden ist und für:

- eine ($C_1$-$C_6$) Alkylcarbonyloxogruppe mit linearer oder verzweigter Alkylgruppe wie Methyl oder tert-Butyl steht, wie $Au(OAc)_3$,
- oder auch eine oder zwei der R-Gruppen für einen Liganden L mit mindestens einer elektronenliefernden Gruppe wie Amino, Phosphino, Hydroxyl oder Thiol stehen oder es sich bei dem Liganden um ein "persistentes" Carben, insbesondere vom "Adruengo"-Typ (Imidazol-2-ylidene), handelt; es sich vorzugsweise bei dem Liganden um ein Phosphin wie Triphenylphosphin handelt, beispielsweise $(Ph_3P)AuOC(O)^tBu$,

e) Au-Komplexen wie Au(I)- und Au(III)-Metalloporphyrinen, Gold(I)- oder Gold(III)-Phthalocyaninen oder Gold(I)- oder Gold(III)-Chlorophyllinen;

ausgewählt ist bzw. sind;
das Metallderivat bzw. die Metallderivate insbesondere aus Gold(I)- oder Gold(III)-Oxiden und -Hydroxiden wie $Au_2O_3$, $Au(OH)_3$ oder AuOH ausgewählt ist bzw. sind;
ii) dem Silberderivat bzw. den Silberderivaten, das bzw. die aus

a) Silber(I)- und Silber(II)-oxiden wie $Ag_2O$ und AgO,
b) Ag(I)-Salzen, insbesondere ausgewählt aus den folgenden Formeln:

• AgHal, wobei Variaben Hal für ein Halogenatom (F, Cl, Br, I) stehen, wie AgCl, AgBr oder AgI;
• $Ag_xR^3_z$, wobei $R^3$ gleich oder verschieden ist und für Folgendes steht:

- eine Sulfonatgruppe, wie $Ag_2S0_4$;
- eine ($C_1$-$C_6$) Alkylcarbonyloxogruppe mit linearer oder verzweigter Alkylgruppe, die gegebenenfalls durch eine Hydroxylgruppe substituiert sein kann, steht, wie Ag-Acetat, Ag-Propionat und Ag-Lactat;
- $1 \leq z \leq 6$ und $1 \leq x \leq 4$;
- mit Ausnahme von Silbernitrat $Ag(NO_3)$ ;

c) Ag(I)-Metalloporphyrinen;
d) Ag(I)-Phthalocyaninen;
e) Ag(I)-Chlorophyllinen a oder b ausgewählt ist bzw. sind;

iii) dem Molybdän(Mo)(II)- bis (VI)-Derivat bzw. den Molybdän(Mo) (II)- bis (VI)-Derivaten, das bzw. die aus

a) Mo(VI)-Oxiden wie

- Mo-Trioxid der Formel $MoO_3$;
- Mo(IV)-Oxiden mit $\beta$-Diketon-Liganden $MoO_2L_2$, wobei die L-Liganden gleich oder verschieden, vorzugsweise gleich, sind und für ein $\beta$-Diketon vom R-C(X)-C(R')-C(X)R''-Typ stehen, wobei R und R'' gleich oder verschieden sind und für eine lineare oder verzweigte ($C_1$-$C_6$) - Alkylgruppe stehen und R' für ein Wasserstoffatom oder eine lineare oder verzweigte ($C_1$-$C_6$)Alkylgruppe steht und X für ein Sauerstoff- oder Schwefelatom oder eine N(R)-Gruppe steht, wobei R für ein Wasserstoffatom oder eine lineare oder verzweigte ($C_1$-$C_6$)Alkylgruppe steht;
- Mo(VI)-Oxid-Komplexen, die von $MoO_3$ und einem $C_2$-$C_{10}$-Hydroxyalkylcarbonsäure-Liganden oder $C_2$-$C_{10}$-Polyolen mit 2 bis 5 Hydroxylgruppen, insbesondere Ethylenglykol oder Glycerin, abstammen;

  - dem Monohydrat $MoO_3 \cdot H_2O$, dem Dihydrat $MoO_3 \cdot 2H_2O$ oder Molybdänsäure ($H_2MoO_4 \cdot H_2O$) ;

- Molybdändioxid;
- Dihalogendioxomolybdän $(Hal)_2MoO_2$, wobei die Variablen Hal gleich oder verschieden sind und wie oben definiert sind, insbesondere die Variablen Hal gleich sind und für ein Chloratom stehen;
- Molybdänblau-Verbindungen, Gemische von Mo-Oxid/Mo(VI)- und Mo(V)-Hydroxiden; insbesondere $Mo^{6+}{}_3Mo^{5+}{}_3O_{18}H$ und den Derivaten der Kondensation mit PhosphatIonen;
- Gemischen von Mo-Oxiden mit verschiedenen Wertigkeiten Mo(VI)-Mo(V) (Mo-Oxid-Bronzen); spezieller binären Bronzen und tertiären Bronzen, $A_{0,33}MoO_3$ (A = Li, K, Rb, Cs, Tl) ; $A_{0,3}MoO_3$ (A = K, Rb, Tl) ; $A_{0,9}Mo_8O_{17}$ (A = Li, Na, K, Tl), und Seltenerdmetallbronzen $La_2Mo_2O_7$;

b) Mo-Oxoanionen, ausgewählt aus Molybdaten $Z_2MoO_4$, wobei die Variablen Z gleich oder verschieden sind und wie oben definiert sind;
c) Polyoxometallaten der Formel: $[XY_uMo_{12-u}O_{40}]^{(3+u)-}$ $(Z)_{(3+u)}$, wobei X und Y aus P, Si und V ausgewählt sind; $0 \leq u \leq 6$ und Z wie oben definiert ist oder Z für ein Wasserstoffatom steht, wobei das Polyoxometallat insbesondere die Formel $H_5PV_2Mo_{10}O_{40}$ aufweist;
d) binären Halogeniden von Mo in den Oxidationsstufen (II) bis (VI), die durch 6 Halogene hexakoordiniert sind, von Mo(V), Mo(IV) und Mo(III), wobei die Mo-Atome über Halogenbindungen gebunden sind, wie Mo(II)-Halogenid mit $[Mo_6Hal'_8]^{4+}$-Clustern, die mit Halogenatomen gebunden sind, was $Mo_6Hal_{12}$ ergibt, wobei die Variablen Hal gleich oder verschieden sind und wie oben definiert sind;
e) Molybdäntetrahalogenide $(Hal)_4Mo$, wobei die Variablen Hal gleich oder verschieden sind und wie oben definiert sind, wie $MoCl_4$;

f) Mo-Schwefelderivaten, ausgewählt aus:

- Molybdändisulfiden [*1317-33-5*], Molybdän(IV)-Sulfiden, $MoS_2$, Molybdaten der Formel $(Z)_2MoS_4$, wobei die Variablen Z gleich oder verschieden sind und wie oben definiert sind;
- Mo-Sesquisulfid; Dimolybdän(III)-trisulfiden, $Mo_2S_3$;
- Tetrasulfidsalzen $ZMOS_{24}^-$,

g) Oxomolybdän(VI)-Derivaten, ausgewählt aus:

- $Mo(O)Hal_4$, wobei die Variablen Hal gleich oder verschieden sind und wie oben definiert sind;
- $Mo(O)_2Hal_2$, wobei die Variablen Hal gleich oder verschieden sind und wie oben definiert sind;

h) Trihalogenoxomolybdän(V)-Derivaten und Addukt en davon mit organischen Liganden L gemäß obiger Definition, wobei L vorzugsweise für Folgendes steht:

- eine zweizähnige Gruppe R-C(X)-CR'R''-C(X)-R''', wobei R und R'''' gleich oder verschieden sind und für eine lineare oder verzweigte $(C_1-C_6)$Alkylgruppe stehen, R' und R'' gleich oder verschieden sind und für ein Wasserstoffatom oder eine lineare oder verzweigte $(C_1-C_6)$Alkylgruppe stehen, wobei R' und R'' vorzugsweise für ein Wasserstoffatom stehen, und X für ein Sauerstoff- oder Schwefelatom oder eine N(R)-Gruppe steht, wobei R für ein Wasserstoffatom oder eine lineare oder verzweigte $(C_1-C_6)$Alkylgruppe steht, wie Acetylaceton;
- eine zweizähnige Gruppe vom 2,2-Bipyridyl-Typ;

i) dem Oxomolybdänderivat der Formel $Mo(O)Hal_3 \cdot 2$ L, wobei L und Hal wie oben definiert sind, vorzugsweise L für ein R"-O-R', wobei R und R' wie oben definiert sind, wie Diethylether, und eine Heteroarylgruppe, wie Pyridin, steht;
j) Molybdaten, Isopolymolybdaten und Heteropolymolybdaten mit einem tetraedrischen Anion $[MoO_4]^{2-}$, wie Ammoniumheptamolybdat (Ammoniumisopolymolybdat), $(NR'_4)_6Mo_7O_{24}$-Hydrat, wobei die Variablen R' gleich oder verschieden sind und wie oben definiert sind, R' insbesondere für ein Wasserstoffatom steht;
k) Molybdaten von zweiwertigen Kationen, insbesondere denjenigen, die in Wasser löslich sind, wie den Molybdaten von $Mg^{2+}$, und Molybdaten von dreiwertigen Kationen, insbesondere der Formeln $A_2(MoO_4)_3$ oder $A_2Mo_3O_{12}$, wobei A für ein Atom, das aus Al, Cr, Bi und Lanthanid ausgewählt ist, steht;
l) Heteropolymolybdaten mit einem $[MoO_6]$-Oktaeder mit anderen Heteroatomen als dem Sauerstoffatom, die insbesondere aus S, N und P ausgewählt sind; wobei die Heteromolybdate spezieller die Formel $[X^+_nMo_{12}O_{40}]^{(8-n)-}$ mit tetrakoordinierten Heteroatomen (X) aufweisen;
m) Mo-Komplexen mit Organoschwefel-Liganden wie Phosphorodithioaten oder Dithiophosphaten und Dithiocarbamaten, $[Mo_2O_3L_4]$ und $[Mo_2O_2S_2L_2]$, wobei L wie oben definiert ist;
n) Molybdänhexacarbonyl $Mo(CO)_6$;
o) organischen Pigmenten, die sich von Alkalimetall- und Erdalkalimetallmolybdaten ableiten, wie Derivate von Natriummolybdat, wie Derivate von i) Diarylmethan; ii) Triarylmethan; iii) Xanthen; und
p) Ca- und Sr-Molybdaten

ausgewählt ist bzw. sind;
wobei das Metallderivat insbesondere aus den Verbindungen der Formel $Z_2MoO_4$ ausgewählt ist;
iv) dem Wolfram(W)-Metallderivat bzw. den Wolfram(W)-Metallderivaten, das bzw. die aus a) Wolfram(VI)-oxiden, b) Wolfram-Oxoanionen, vorzugsweise hydratisierten oder nicht hydratisierten Alkalimetallwolframaten $Z_2WO_4$, wobei die Variablen Z gleich oder verschieden sind und wie oben definiert sind, c) Polyoxometallaten wie $[XY_uW_{12-u}O_{40}]^{(4+u)-}$; $(Z)_{(4+u)}$, wobei X und Y aus P, Si und V ausgewählt sind und $0 \leq u \leq 6$; wobei das Polyoxometallat insbesondere die Formel $H_4SiW_{12}O_{40}$ aufweist; ausgewählt ist bzw. sind;
wobei das Metallderivat vorzugsweise aus Alkalimetallwolframaten ausgewählt ist;
v) dem Vanadium(V)-Metallderivat bzw. den Vanadium(V)-Metallderivaten, das bzw. die aus a) Vanadiumoxiden wie $V_2O_5$, b) Vanadium-Oxoanionen, ausgewählt aus Vanadaten und Metavanadaten, wie Vanadiumoxidacetylacetonat $VO(acac)_2$, $VOSO_4$ oder Ammoniumvanadat, und c) Polyoxometallaten wie $[XV_uM_{12-u}O_{40}]^{q-}$; $(Z)_q$, wobei M = W oder Mo, X = P oder Si, $0 \leq u \leq 6$, q=3+x, wenn M=Mo, oder q=4+x, wenn M=W, und Z wie oben definiert ist; wobei das Polyoxometallat insbesondere die Formel $H_5PV_2Mo_{10}O_{40}$ aufweist; ausgewählt ist bzw. sind; wobei das Metallderivat vorzugsweise aus denjenigen der Formel $[XV_uMo_{12-u}O_{40}]^{(3+u)}$; $(Z)_{(3+u)}$ gemäß obiger Definition und insbesondere $H_5PV_2Mo_{10}O_{40}$ ausgewählt ist;
vi) dem Ruthenium(Ru)-Metallderivat bzw. den Ruthenium(Ru)-Metalld erivaten, das bzw. die aus a) Rutheniumoxiden, b) Ruthenium-Oxoanionen wie Alkalimetallperruthenaten und c) Rutheniumkomplexen wie $(Hal)_2RuL_4$, wobei die Variablen Hal gleich oder verschieden sind und wie oben definiert sind und die Variablen L gleich oder verschieden sind und Liganden gemäß obiger Definition sind, ausgewählt ist bzw. sind; wobei das Metallderivat vorzugsweise aus $RuCl_2(PPh_3)_4$ und Kaliumperruthenat ausgewählt ist;

vii) Magnesium(II)-metalloporphyrinen, d) Magnesium(II)-phthalocyaninen, e) Magnesium-(II)-chlorophyllinen und f) Magnesium(II)-chlorophyllen,

ix) dem Rhenium(Re)-Metallderivat bzw. den Rhenium(Re)-Metallderivaten, das bzw. die aus $R'ReO_3$, wobei R' für ein Wasserstoffatom oder eine lineare oder verzweigte $(C_1$-$C_6)$Alkylgruppe steht, wie $CH_3ReO_3$, oder Re-Komplexen, wie Phthalocyaninen, ausgewählt ist bzw. sind;

x) dem Titan(Ti)-Metallderivat bzw. den Titan(Ti)-Metallderivaten, das bzw. die aus a) Titan(IV)-Salzen wie $Ti(SO_4)_2$, b) Titanoxiden sowie Salzen davon, Hydraten davon und geträgerten Formen davon, insbesondere ausgewählt aus Titan(III)-hydroxid $Ti(OH)_3$ und $TiO_3$, Dititantrioxid $Ti_2O_3$, Erdalkalimetalltitantrioxiden, Erdalkalimetalltitanpentoxiden, Titanaten der allgemeinen Formel $M^{II}TiO_4$, wobei $M^{II}$ für ein Mg-, Zn-, Mn- oder Co-Metall steht, Peroxytitansäure und Peroxytitanaten $H_4TiO_5$, Titan(II)-dioxid $TiO_2$ oder Titandisulfid $TiS_2$, und c) Ti-Komplexen wie Phthalocyaninen ausgewählt ist bzw. sind; wobei es sich bei den Metallderivat bzw. dem Metallderivaten vorzugsweise um $TiO_2$ handelt;

xi) dem Silicium(Si)-Metallderivat bzw. den Silicium(Si)-Metallderivaten, spezieller Siliciumoxid wie $SiO_2$;

xii) das Metallderivat ist (sind) Zinnoxide sowie Salze davon, Hydrate davon und geträgerte Formen davon wie Zinn(II)-oxidhydrat vom Typ $5SnO \cdot 2H_2O$ und Zinn(II)-oxid, Zinn(IV)-oxidhydrat $SnO_2 \cdot nH_2O$ und Zinn (IV)-oxid $SnO_2$, Alkalimetallsalze wie Natrium- und Kaliumzinnhydroxid der Formel $M_2[Sn(OH)_6]$, wobei M für ein Alkalimetall steht, Zinnhydroxide der Formel $R_3SnOH$, $R_2SnOH_2$ oder $RSnOH_3$, wobei R für eine Kohlenwasserstoffgruppe, wie lineares oder verzweigtes $(C_1$-$C_6)$Alkyl oder lineares oder verzweigtes $(C_1$-$C_6)$ Alkoxy oder (Di)$(C_1$-$C_6)$alkylamino steht; wobei es sich bei dem Metalloxid bzw. den Metalloxiden vorzugsweise um Zinn(IV)-oxid $SnO_2$ handelt;

xiii) das Metallderivat bzw. die Metallderivate ist (sind) von Zirconium (Zr), insbesondere ausgewählt aus:

a) Titanoxid sowie Salzen davon, Hydraten davon und geträgerten Formen davon wie Zirconiumoxid [*1314-23-4*], $ZrO_2$, Zirconiumoxidhydrat $[Zr_4(OH)_8 \cdot 16H_2O]_8$, Zirconiumhydroxiddihalogenid wie Zirconiumhydroxiddichlorid [*22196-48-1*] sowie den Verbindungen der Formel $Zr(OH)_2Hal_2 \cdot 7H_2O$, wobei Hal für ein Halogenatom wie Chlor steht, Zirconiumoxidhalogeniden wie Zirconiumoxidchlorid, Zirconiumhalogenidoxiden wie Zirconiumoxiddichlorid, $ZrOCl_2 \cdot 8H_2O$, $[Zr_4(OH)_8 \cdot 16H_2O]Cl_8 \cdot 12H_2O$ und Zirconiummonohalogenid, wie Zirconiumhydroxidmonochlorid $[Zr_4(OH)_{12} \cdot 16H_2O]Cl_4$; und

b) Zr-Komplexen wie Phthalocyaninen, wobei es sich bei dem Metallderivat bzw. den Metallderivaten vorzugsweise um Zirconium(II)-oxid $ZrO_2$ handelt;

xiv) das Metallderivat bzw. die Metallderivate ist (sind) von Niob (Nb), insbesondere ausgewählt aus Nioboxiden sowie Salzen davon, Hydraten davon und geträgerten Formen davon wie Niobpentoxid $Nb_2O_5$ [*1313-96-8*], Alkalimetallniobtrioxid wie Lithiumniobtrioxid $LiNbO_3$ [12031-63-9] oder $KNbO_3$ [12030-85-2] und Nioboxidhalogeniden wie Niobiumoxidchlorid [*13597-20-1*], $NbOCl_3$;

xv) das Metallderivat bzw. die Metallderivate ist (sind) das Metallderivat bzw. die Metallderivate ist (sind) von Indium (In), sowie Salze davon, Hydrate davon und geträgerten Formen davon, insbesondere ausgewählt aus Indiumoxiden wie Indium(III)-oxid der Formel $In_2O_3$, Indium(II)-oxid [12136-26-4] der Formel InO und Indium(I)-oxid [12030-22-7] der Formel $In_2O$, Indiumhydroxid [56108-30-6] der Formel $In(OH)_3$, vorzugsweise Indium (III) -oxid $In_2O_3$; und

xvi) das Metallderivat bzw. die Metallderivate ist bzw. sind $SeO_2$;

auswählt.

**9.** Färbeverfahren nach einem der vorhergehenden Ansprüche, wobei das Metallderivat bzw. die Metallderivate b) aus i) Gold (Au), ii) Silber (Ag), iii) Molybdän (Mo), iv) Wolfram (W), v) Vanadium (V), vi) Ruthenium (Ru), xi) Silicium (Si) ausgewählt ist bzw. sind; insbesondere das Metallderivat bzw. die Metallderivate b) aus den Verbindungen der Formel $ZAu(Hal)_4$ wie $KAuCl_4$; $Au(R)_3$ $Au_2O_3$; $Z_2MoO_4$; $Z_2WO_4$; $[XY_uMo_{12-u}O_{40}]^{q-}(Z)_q$ und $H_5(PV_2Mo_{10}O_{40})$, $(Hal)_2RuL_4$ gemäß dem vorhergehenden Anspruch angegebenen Definition; Magnesiumchlorophyllin; $KRuO_4$, $H_4SiW_{12}O_{40}$ und $Ag_2O$ ausgewählt ist bzw. sind.

**10.** Färbeverfahren nach einem der vorhergehenden Ansprüche, wobei das Metallderivat bzw. die Metallderivate b) von Gold ist (sind); wobei das Metallderivat bzw. die Metallderivate b) insbesondere von Gold (Au) mit Ausnahme von Goldsalzen ist (sind).

**11.** Färbeverfahren nach einem der vorhergehenden Ansprüche, bei dem man das Alkalisierungsmittel bzw. die Alkalisierungsmittel c) aus Alkanolaminen und Alkali- oder Erdalkali(hydrogen)carbonaten auswählt.

**12.** Färbeverfahren nach einem der vorhergehenden Ansprüche, bei dem kein chemisches Oxidationsmittel außer Luftsauerstoff eingesetzt wird.

**13.** Färbeverfahren nach einem der Ansprüche 1 bis 11, bei dem man ein oder mehrere chemische Oxidationsmittel einsetzt wie:

a) Ozon;
b) Alkalimetall- oder quaternäre Ammoniumpersalze;
c) organische aliphatische $C_1$-$C_6$-Persäuren und aromatische $C_6$-$C_{20}$-Persäuren und deren Percarboxylat-Formen, wie Perameisensäure, Peressigsäure, Perbenzoesäurederivate, Trifluoressigsäure, Peroxyphthalsäure, Peroxymaleinsäure oder Peroxypropionsäure;
d) organische Peroxide,
e) oxidierende Anionen, wie Nitrite, Nitrate, Hypochlorite, Hypobromite, Hypoiodite, Chlorite, Bromite, Iodite, Chlorate, Bromate, Iodate, Periodate, wobei das Oxidationsmittel insbesondere aus Alkalimetallhypochlorit oder -periodat wie Natriumhypochlorit oder Natriumperiodat ausgewählt wird;
f) stabile N-Oxy(NO·)-Radikale, wie das 2,2,6,6-Tetra($C_1$-$C_6$)alkylpiperidino-oxy-Radikal, das 2,2,6,6-Tetra($C_1$-$C_6$)alkylmorpholino-oxy-Radikal, Fremy-Nitrosodisulfonatsalze oder Morpholin-N-oxid; wobei das Oxidationsmittel insbesondere aus dem 2,2,6,6-Tetramethylpiperidinyloxy-Radikal ausgewählt wird;
g) hypervalente Iodderivate, wie Iodtriacetat, Iodosobenzol, Iodbenzoltriacetat, Iodperbenzoesäurederivate, Periodinane und Alkyl- und Benzoylhypoiodite;
h) die folgenden organischen Verbindungen: N-Halogensuccinimide, Trichlorisocyanursäure, N-Hydroxyphthalimid und Alkylnitrite;
i) Wasserstoffperoxid oder ein oder mehrere Systeme, die Wasserstoffperoxid erzeugen, wie

i-1) Harnstoffperoxid;
i-2) Polymerkomplexe, die Wasserstoffperoxid freisetzen können, wie Polyvinylpyrrolidon/$H_2O_2$ ;
i-3) Oxidase n, die in Gegenwart eines geeigneten Substrats (beispielsweise Glucose im Fall von Glucoseoxidase oder Harnsäure mit Uricase) Wasserstoffperoxid produzieren;
i-4) Metallperoxide, die in Wasser Wasserstoffperoxid erzeugen, wie Calciumperoxid oder Magnesiumperoxid;

i-v) Perborate; oder
i-vi) Percarbonate.

**14.** Verfahren nach einem der vorhergehenden Ansprüche in einem Schritt, der darin besteht, dass man auf die Keratinfasern eine wässrige Zusammensetzung, die a), b) und c) gemäß einem der vorhergehenden Ansprüche und gegebenenfalls ein oder mehrere chemische Oxidationsmittel gemäß dem vorhergehenden Anspruch umfasst, aufbringt.

**15.** Färbeverfahren nach einem der Ansprüche 1 bis 11 in zwei Schritten, das darin besteht, dass man im ersten Schritt auf die Keratinfasern eine Zusammensetzung, die die Bestandteile a) und b) gemäß einem der Ansprüche 1 bis 10 und gegebenenfalls ein oder mehrere chemische Oxidationsmittel gemäß Anspruch 14 umfasst, aufbringt und dann in einem zweiten Schritt eine Zusammensetzung, die c) gemäß den Ansprüchen 1 oder 11 umfasst, insbesondere bei einem pH-Wert von mehr als 7 und vorzugsweise zwischen 8 und 12, aufbringt.

**16.** Färbeverfahren nach einem der Ansprüche 1 bis 11 und 13, das in mindestens zwei Schritten durchgeführt wird, die mit der Behandlung der Keratinfasern mit dem Bestandteil c) gemäß den Ansprüchen 1 oder 11 endet und auf die Nachbehandlungsschritte wie eine Shampoonierung mit einem herkömmlichen Shampoo, eine Spülung, beispielsweise mit Wasser, und/oder Trocknung der Keratinfasern durch Wärmebehandlung folgen können.

**17.** Färbeverfahren nach einem der vorhergehenden Ansprüche unter Einsatz eines oder mehrerer Lichtbestrahlungen mit einer oder mehreren elektromagnetischen Wellen mit einer Wellenlänge zwischen 10 nm im UV-Bereich und 100 $\mu$m im Infrarot-IR-Bereich, die insbesondere aus der künstlichen Lichtbestrahlung, insbesondere Lampen, die im UV-Bereich emittieren, Glühlampen, Halogenlampen, Fluoreszenzlampen, Quecksilberlampen, Niederdrucklampen, beispielsweise Natrium- oder Neonlampen, Hochdrucklampen, beispielsweise Quecksilberlampen, Halogenidlampen, Blitzlampen, beispielsweise Xenon-Blitzlampen, Lampen mit fluoreszierendem Excimer wie Xenonlampen mit fluoreszierendem Excimer, Leuchtdioden oder LED mit einer Leistung von 50 bis 1000 mW, Lampen, die schwarzes Licht oder Wood-Licht emittieren, und Lasern, stammen.

**18.** Kosmetische Zusammensetzung zur Färbung von Keratinfasern, enthaltend:

a) ein oder mehrere ortho-Diphenolderivate gemäß einem der Ansprüche 1 bis 7;
b) ein oder mehrere Metallderivate gemäß einem der Ansprüche 1, 8 bis 10;
c) ein oder mehrere Alkalisierungsmittel gemäß einem der Ansprüche 1 oder 11; und gegebenenfalls
d) ein oder mehrere chemische Oxidationsmittel gemäß Anspruch 13.

**19.** Zusammensetzung nach dem vorhergehenden Anspruch, wobei das Metallderivat bzw. die Metallderivate b) von Gold (Au) gemäß einem der Ansprüche 1, 8 bis 10 und gegebenenfalls ein oder mehrere chemische Oxidationsmittel gemäß Anspruch 13 ist (sind) ist (sind) .

**20.** Verwendung der folgenden Kombination zur Färbung von Keratinfasern wie dem Haar:

a) einem oder mehreren ortho-Diphenolderivaten gemäß einem der Ansprüche 1 bis 7;
b) einem oder mehreren Metallderivaten gemäß einem der Ansprüche 1, 8 bis 10;
c) einem oder mehreren Alkalisierungsmitteln gemäß einem der Ansprüche 1 oder 11; und gegebenenfalls
d) einen oder mehreren chemischen Oxidationsmitteln gemäß Anspruch 13.

**21.** Verbindung zur Färbung von Keratinfasern wie dem Haar nach dem vorhergehenden Anspruch, **dadurch gekennzeichnet, dass** b) das Metallderivat bzw. die Metallderivate ein oder mehrere Derivate von Gold gemäß einem der Ansprüche 1, 8 bis 10 ist (sind).

## Claims

**1.** Dyeing method carried out, in one or more stages, by application, to the keratinous fibres, of one or more cosmetic compositions comprising, taken together or separately in the said composition (s), the following ingredients:

**a)** one or more ortho-diphenol derivative(s) other than derivatives having indole units;
**b)** one or more metal derivative (s) chosen from metal salts, metal complexes, metal oxides, metal oxoanions, their supported forms, their hydrates and their mixtures for which the metal or metals is (are) chosen from:

**i)** gold (Au),
**ii)** molybdenum (Mo),
**iii)** silver (Ag)(I) and (II) oxides, Ag(I) and (II) salts chosen from silver halides, Ag sulfate, $[\mathbf{R^1}\text{-}\mathbf{C(O)O}]_n\mathbf{Ag}$ with n = 1 or 2, $R^1$ representing a $(C_1\text{-}C_6)$alkyl group,
**iv)** tungsten (W),
**v)** vanadium (V),
**vi)** ruthenium (Ru),
**vii)** Mg(II) metalloporphyrins, Mg(II) phthalocyanines, Mg(II) chlorophyllins or Mg(II) chlorophylls,
**ix)** rhenium (Re),
**x)** titanium (Ti),
**xi)** silicon (Si),
**xii)** tin oxides,
**xiii)** zirconium (Zr),
**xiv)** niobium (Nb),
**xv)** indium (In),
**xvi)** selenium (Se), and

**c)** one or more basifying agent(s) chosen from alkamolamines and (bi)carbonates;

it being understood that the pH of the composition comprising the ingredient **c)** is alkaline, i.e. greater than 7 and preferably between 8 and 12, particularly between 8 and 10.5.

2. Dyeing method according to the preceding claim, in which the ortho-diphenol(s) is (are) chosen from natural ortho-diphenol derivative(s).

3. Dyeing method according to Claim 1 or 2, in which the ingredient a) is an ortho-diphenol comprising an aromatic ring chosen from benzene, naphthalene, tetrahydronaphthalene, indane, indene, anthracene, phenanthrene, isoindole, indoline, isoindoline, benzofuran, dihydrobenzofuran, chroman, isochroman, chromene, isochromene, quinoline, tetrahydroquinoline and isoquinoline, the said aromatic ring comprising at least two hydroxyl groups carried by two contiguous adjacent atoms of the aromatic ring.

4. Dyeing method according to any one of the preceding claims, in which the ingredient **a)** is of following formula (I) or one of its oligomers, in salified or non-salified form:

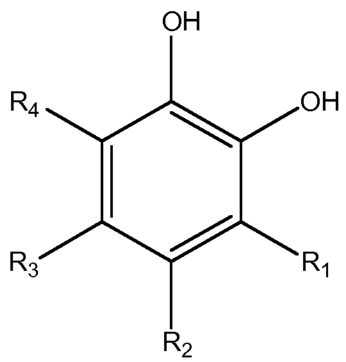

(I)

in which formula (I) the substituents:

• $R_1$ to $R_4$, which are identical or different, represent:

- a hydrogen atom,
- a halogen atom,
- a hydroxyl radical,
- a carboxyl radical,
- an alkyl carboxylate or alkoxycarbonyl radical,
- an optionally substituted amino radical,
- an optionally substituted and linear or branched alkyl radical,
- an optionally substituted and linear or branched alkenyl radical,
- an optionally substituted cycloalkyl radical,
- an alkoxy radical,
- an alkoxyalkyl radical,
- an alkoxyaryl radical, it being possible for the aryl group to be optionally substituted,
- an aryl radical,
- a substituted aryl radical,
- a saturated or unsaturated heterocyclic radical, carrying or not carrying a cationic or anionic charge, optionally substituted and/or optionally fused with an aromatic ring, the said aromatic ring being optionally substituted,
- a radical comprising one or more silicon atoms,

where two of the substituents carried by two adjacent carbon atoms $R_1$ - $R_2$, $R_2$ - $R_3$ or $R_3$ - $R_4$ together form a saturated or unsaturated, aromatic or non-aromatic and substituted or unsubstituted ring, optionally comprising one or more heteroatoms and optionally fused with one or more saturated or unsaturated and optionally substituted rings optionally comprising one or more heteroatoms.

5. Dyeing method according to the preceding claim, in which the ortho-diphenol(s) is (are) chosen from:

- flavonols,
- anthocyanidins,
- anthocyanins or anthocyans,

- ortho-hydroxybenzoates,
- flavones,
- hydroxystilbenes,
- 3,4-dihydroxyphenylalanine and derivatives thereof,
- 2,3-dihydroxyphenylalanine and derivatives thereof,
- 4,5-dihydroxyphenylalanine and derivatives thereof,
- dihydroxycinnamates,
- ortho-polyhydroxycoumarins,
- ortho-polyhydroxyisocoumarins,
- ortho-polyhydroxycoumarones,
- ortho-polyhydroxyisocoumarones,
- ortho-polyhydroxychalcones,
- ortho-polyhydroxychromones,
- polyhydroxyquinones,
- orthohydroxyxanthones,
- 1,2-dihydroxybenzene and derivatives thereof,
- 1,2,4-trihydroxybenzene and derivatives thereof,
- 1,2,3-trihydroxybenzene and derivatives thereof,
- 2,4,5-trihydroxytoluene and derivatives thereof,
- proanthocyanidins,
- proanthocyanins,
- tannic acid,
- ellagic acid,
- and the mixtures of the preceding compounds.

**6.** Dyeing method according to any one of Claims 2 to 5, in which the natural ortho-diphenol(s) **a)** is (are) chosen from extracts of animals, bacteria, fungi, algae or plants.

**7.** Dyeing method according to the preceding claim, in which the natural ortho-diphenol(s) **a)** is (are) chosen from:

- extracts of tea leaves, extracts of rosemary leaves and extracts of mate leaves;
- extracts of fruit, such as extracts of grape or extracts of cocoa beans and/or pods;
- extracts of vegetables, such as extracts of onion peel;
- extracts of tree wood, such as extracts of pine bark or extracts of logwood.

**8.** Dyeing method according to any one of the preceding claims, in which the metal derivative(s) **b)** is (are) chosen from:

**i)** the gold derivative(s) chosen from:

a) gold(I) and (III) oxides, such as $Au_2O_3$,
b) Au(I) and (III) hydroxides, such as $Au(OH)_3$, AuOH or Au(O)OH,
c) gold (I) salts, particularly of formula AuHal with Hal representing a halogen atom, such as AuCl or AuI,
d) gold(III) salts chosen in particular from the following formulae:

• **$Au(Hal)_3$** with Hal, which are identical or different, as defined above, such as $AuCl_3$ and $AuBr_3$,
• hydrated or non-hydrated **$ZAu(Hal)_4$,** with Z representing a hydrogen atom, an alkali metal, such as Li, Na or K, or an ammonium $NH_4^+$, and Hal, which are identical or different, as defined above, such as $KAuCl_4$ or $HAuCl_4$,
• **Au(R)**$_3$ with R, which are identical or different, representing:

- a ($C_1$-$C_6$)alkylcarbonyloxo group, where the alkyl group is linear or branched, such as methyl or tert-butyl, such as $Au(OAc)_3$,
- or alternatively one or two of the R groups represent a ligand L carrying at least one electron-donating group, such as amino, phosphino, hydroxyl or thiol, or the ligand is a "persistent" carbene, particularly of "*Arduengo*" type (imidazol-2-ylidenes); preferentially, the ligand is a phosphine, such as triphenylphosphine, for example $(Ph_3P)AuOC(O)^tBu$,

e) Au complexes, such as Au(I) and (III) metalloporphyrins, gold(I) and (III) phthalocyanines or gold(I) and

(III) chlorophyllins a or b;

in particular, the metal derivative(s) is (are) chosen from gold(I) and (III) oxides and hydroxides, such as $Au_2O_3$, $Au(OH)_3$ or AuOH;

**ii)** the silver derivative(s) chosen from:

a) silver(I) and (II) oxides, such as $Ag_2O$ and AgO;
b) Ag(I) salts chosen in particular from the following formulae:

• AgHal, with Hal representing a halogen atom (F, Cl, Br, I), such as AgCl, AgBr or AgI;
• $Ag_xR^3_z$, with $R^3$, which are identical or different, representing:

- a sulfate group, such as $Ag_2SO_4$;
- a ($C_1$-$C_6$)alkylcarbonyloxo group, where the alkyl group is linear or branched and can optionally be substituted by a hydroxyl group, such as Ag acetate, Ag propionate or Ag lactate;
- $1 \leq z \leq 6$ and $1 \leq x \leq 4$;
- with the exception of silver nitrate Ag ($NO_3$) ;

c) Ag(I) metalloporphyrins;
d) Ag(I) phthalocyanines;
e) Ag(I) chlorophyllins a or b;

**iii)** the molybdenum (Mo) (II) to (VI) derivative (s) chosen from:

a) Mo(VI) oxides, such as:

- Mo trioxide of formula $\mathbf{MoO_3}$;
- Mo(IV) oxides having *β*-diketone ligands $\mathbf{MoO_2L_2}$ with the L ligands, which are identical or different, preferentially identical, representing a *β*-diketone of **R-C(X)-C(R')-C(X)R"** type with R and R", which are identical or different, representing a linear or branched ($C_1$-$C_6$) alkyl group, R' representing a hydrogen atom or a linear or branched ($C_1$-$C_6$) alkyl group and X representing an oxygen or sulfur atom or an N(R) group with R representing a hydrogen atom or a linear or branched ($C_1$-$C_6$)alkyl group;
- Mo(VI) oxide complexes originating from $MoO_3$ and from a $C_2$-$C_{10}$ hydroxyalkylcarboxylic acid ligand or from $C_2$-$C_{10}$ polyols comprising from 2 to 5 hydroxyl groups, in particular ethylene glycol or glycerol;
- the monohydrate $\mathbf{MoO_3 \cdot H_2O}$, the dihydrate $\mathbf{MoO_3} \cdot \mathbf{2H_2O}$ or molybdic acid ($\mathbf{H_2MoO_4} \cdot \mathbf{H_2O}$);
- molybdenum dioxide;
- dihalodioxomolybdenum $\mathbf{(Hal)_2MoO_2}$ with Hal, which are identical or different, as defined previously; in particular, Hal are identical and represent a chlorine atom;
- molybdenum blues, Mo oxide/Mo(VI) hydroxide and Mo(V) hydroxide mixture; in particular $Mo^{6+}{}_3$ $Mo^{5+}{}_3$ $O_{18}H$, and the derivatives from condensation with phosphate ions;
- mixtures of Mo oxides where the molybdenum has different valences Mo(VI)-Mo(V) (Mo oxide bronzes), more particularly, the binary bronzes and the tertiary bronzes $\mathbf{A_{0.33}MoO_3}$ (A = Li, K, Rb, Cs, Tl); $\mathbf{A_{0.3}MoO_3}$ (A = K, Rb, Tl); $\mathbf{A_{0.9}Mo_8O_{17}}$ (A = Li, Na, K, Tl), and the rare earth metal bronzes $La_2Mo_2O_7$;

b) Mo oxoanions chosen from the molybdates $\mathbf{Z_2MoO_4}$ with Z, which are identical or different, as defined above;
c) polyoxometallates of formula: $\mathbf{[XY_uMo_{12-u}O_{40}]^{(3+u)-}}$ $\mathbf{(Z)_{(3+u)}}$ with X and Y chosen from P, Si, V; $0 \leq u \leq 6$, and Z as defined above or Z representing a hydrogen atom; in particular, the polyoxometallate is of formula $H_5PV_2Mo_{10}O_{40}$;
d) binary Mo halides of oxidation states (II) to (VI), hexacoordinated with 6 halogens of Mo(V), (IV) and (III), the Mo atoms being bonded via halogen bonds, such as Mo(II) halide containing $[Mo_6Hal_8]^{4+}$ clusters bonded to halogen atoms to give $\mathbf{Mo_6Hal_{12}}$ with Hal, which are identical or different, as defined above;
e) molybdenum tetrahalides $\mathbf{(Hal)_4Mo}$ with Hal, which are identical or different, as defined above, such as $MoCl_4$;
f) Mo sulfur derivatives chosen from:

- molybdenum disulfides [*1317-33-5*], molybdenum(IV) sulfides, $MoS_2$, the molybdates of formula $\mathbf{(Z)_2MoS_4}$, with Z, which are identical or different, as defined above;

- Mo sesquisulfides; dimolybdenum(III) trisulfides, $Mo_2S_3$;
- tetrasulfide salts **Z $MoS_{24}^-$** with Z as defined above;

g) oxomolybdenum(VI) derivatives chosen from:

- **Mo(O)$Hal_4$** with Hal, which are identical or different, being as defined above;
- **$Mo(O)_2Hal_2$** with Hal, which are identical or different, being as defined above;

h) trihalooxomolybdenum(V) derivatives and these adducts with organic ligands L as defined above, L preferentially representing:

- a bidentate group R-C(X)-CR'R''-C(X)-R''' with R and R'''', which are identical or different, representing a linear or branched ($C_1$-$C_6$) alkyl group, R' and R'', which are identical or different, representing a hydrogen atom or a linear or branched ($C_1$-$C_6$)alkyl group, R' and R'' preferentially representing a hydrogen atom, and X representing an oxygen or sulfur atom or an N(R) group, with R representing a hydrogen atom or a linear or branched ($C_1$-$C_6$)alkyl group, such as acetylacetone;
- a bidentate group of 2,2-bipyridyl type;

i) the oxomolybdenum derivative of formula **Mo(O)$Hal_3$·2 L** with L and Hal as defined above; preferentially, L represents an R''-O-R' with R and R' as defined above, such as diethyl ether, and a heteroaryl group, such as pyridine;
j) molybdates, isopolymolybdates and heteropolymolybdates comprising a tetrahedral anion $[MoO_4]^{2-}$, such as ammonium heptamolybdate (isopolymolybdate), $(NR'_4)_6Mo_7O_{24}$ hydrate, with R', which are identical or different, being as defined above; in particular, R' is a hydrogen atom;
k) molybdates of divalent cations, especially those which are water-soluble, such as the molybdates of $Mg^{2+}$, and molybdates of trivalent cations, especially of formula **$A_2(MoO_4)_3$** or **$A_2MO_3O_{12}$**, with A representing an atom chosen from Al, Cr, Bi and lanthanide;
l) heteropolymolybdates having an octahedron [**$MoO_6$**] incorporating heteroatoms other than the oxygen atom, chosen especially from S, N and P; more specifically, the heteromolybdates are of formula **$[X^+_nMo_{12}O_{40}]^{(8-n)-}$**, comprising tetracoordinated heteroatoms (X);
m) Mo complexes with organosulfur ligands, such as phosphorodithioates or dithiophosphates and dithiocarbamates, **$[Mo_2O_3L_4]$** and **$[Mo_2O_2S_2L_2]$**, with L as defined above;
n) molybdenum hexacarbonyl, $Mo(CO)_6$;
o) organic pigments derived from alkali metal and alkaline earth metal molybdates, such as sodium molybdate derivatives, such as derivatives of i) diarylmethane; ii) triarylmethane; iii) xanthene; and
p) Ca and Sr molybdates;
in particular, the metal derivative is chosen from the compounds of formula $Z_2MoO_4$,

**iv)** the tungsten (W) metal derivative(s) chosen from a) tungsten(VI) oxides, b) tungsten oxoanions, preferentially the hydrated or non-hydrated alkali metal tungstates **$Z_2WO_4$,** with Z, which are identical or different, as defined above, c) polyoxometallates, such as **$[XY_uW_{12-u}O_{40}]^{(4+u)}$-$(Z)_{(4+u)}$**, with X and Y chosen from P, Si or V and $0 \leq u \leq 6$; in particular, the polyoxometallate is of formula $H_4SiW_{12}O_{40}$;
preferentially, the metal derivative is chosen from alkali metal tungstates;
**v)** the vanadium (V) metal derivative(s) chosen from a) vanadium oxides, such as $V_2O_5$, b) vanadium oxoanions chosen from vanadates and metavanadates, such as vanadium oxide acetylacetonate $VO(acac)_2$, $VOSO_4$ or ammonium vanadate, and c) polyoxometallates, such as **$[XV_uM_{12-u}O_{40}]^{q-}(Z)_q$,** with M = W or Mo, X = P or Si, $0 \leq u \leq 6$, q=3+x if M=Mo or q=4+x if M=W, and Z as defined above; in particular, the polyoxometallate is of formula $H_5PV_2Mo_{10}O_{40}$; preferentially, the metal derivative is chosen from those of formula **$[XV_uMo_{12-u}O_{40}]^{(3+u)}(Z)_{(3+u)}$** as defined above and in particular $H_5PV_2Mo_{10}O_{40}$ ;
**vi)** the ruthenium (Ru) metal derivative(s) chosen from a) ruthenium oxides, b) ruthenium oxoanions, such as alkali metal perruthenates, and c) ruthenium complexes, such as **$(Hal)_2RuL_4$,** with Hal, which are identical or different, as defined above and L, which are identical or different, being ligands as defined above; preferentially, the metal derivative is chosen from $RuCl_2(PPh_3)_4$, potassium perruthenate;
**vii)** magnesium(II) metalloporphyrins, d) magnesium(II) phthalocyanines, e) magnesium(II) chlorophyllins or f) magnesium(II) chlorophylls ;
**ix)** the rhenium (Re) metal derivative(s) chosen from R'$ReO_3$, with R' representing a hydrogen atom or a linear or branched ($C_1$-$C_6$)alkyl group, such as $CH_3ReO_3$, or Re complexes, such as phthalocyanines;
**x)** the titanium (Ti) metal derivative(s) chosen from: a) titanium(IV) salts, such as $Ti(SO_4)_2$, b) titanium oxides

and their salts, their hydrates and their supported forms, chosen in particular from titanium(III) hydroxide $Ti(OH)_3$ and $TiO_3$, dititanium trioxide $Ti_2O_3$, alkaline earth metal titanium trioxides, alkaline earth metal titanium pentoxides, titanates of general formula **$M^{II}TiO_4$,** in which $M^{II}$ represents a metal Mg, Zn, Mn or Co, peroxytitanic acid and peroxytitanates $H_4TiO_5$, titanium(II) dioxide $TiO_2$ or titanium disulfide $TiS_2$, and c) Ti complexes, such as phthalocyanines; preferentially, the metal derivative(s) is (are) $TiO_{2;}$

**xi)** the silicon (Si) metal derivative(s), more particularly silicon oxide, such as $SiO_{2;}$

**xii)** the metal derivative is (are) tin oxides and their salts, their hydrates and their supported forms, such as tin(II) oxide hydrate of $5SnO \cdot 2H_2O$ type and tin(II) oxide, tin(IV) oxide hydrate $SnO_2 \cdot nH_2O$ and tin(IV) oxide $SnO_2$, alkali metal salts, such as sodium and potassium tin hydroxide of formula **$M_2$ [$Sn(OH)_6$],** with M representing an alkali metal, the tin hydroxides of formula **$R_3SnOH$, $R_2SnOH_2$** or **$RSnOH_3$,** with R representing a hydrocarbon group, such as linear or branched ($C_1$-$C_6$)alkyl, or linear or branched ($C_1$-$C_6$)alkoxy, or (di) ($C_1$-$C_6$)alkylamino; preferentially, the metal oxide(s) is (are) tin(IV) oxide $SnO_{2;}$

**xiii)** the metal derivative(s) is (are) of zirconium (Zr), chosen in particular from:

a) zirconium oxides and their salts, their hydrates and their supported forms, such as zirconium oxide *[1314-23-4],* $ZrO_2$, zirconium oxide hydrate $[Zr_4(OH)_8 \cdot 16H_2O]_8$, zirconium hydroxide dihalide, such as zirconium hydroxide dichloride [*22196-48-1*], and also the compounds of formula $Zr(OH)_2Hal_2 \cdot 7H_2O$, with Hal representing a halogen atom, such as chlorine, zirconium oxyhalides, such as zirconium oxychloride, zirconium halide oxides, such as zirconium oxide dichloride, $ZrOCl_2 \cdot 8H_2O$, $[Zr_4(OH)_8 \cdot 16H_2O]Cl_8 \cdot 12H_2O$, and zirconium monohalide, such as zirconium hydroxide monochloride $[Zr_4(OH)_{12} \cdot 16H_2O]Cl_4$; and

b) Zr complexes, such as phthalocyanines; preferentially, the metal derivative(s) is (are) zirconium(II) oxide $ZrO_2$;

**xiv)** the metal derivative(s) is (are) of niobium (Nb), chosen in particular from niobium oxides and their salts, their hydrates and their supported forms, such as niobium pentoxide $Nb_2O_5$ [*1313-96-8*], alkali metal niobium trioxide, such as lithium niobium trioxide $LiNbO_3$ [12031-63-9] or $KNbO_3$ [12030-85-2], and niobium oxyhalides, such as niobium oxychloride *[13597-20-1],* $NbOCl_3$;

**xv)** the metal derivative (s) is (are) the metal derivative(s) is (are) of indium (In) and their salts, their hydrates and their supported forms, chosen in particular from indium oxides, such as indium(III) oxide of formula $In_2O_3$, indium(II) oxide [12136-26-4] of formula InO and indium(I) oxide [12030-22-7] of formula $In_2O$, indium hydroxide [56108-30-6] of formula $In(OH)_3$, preferentially indium(III) oxide $In_2O_{3;}$

**xvi)** The metal derivative(s) is (are) $SeO_2$.

**9.** Dyeing method according to any one of the preceding claims, in which the metal derivative(s) b) is (are) chosen from **i)** gold (Au), **ii)** silver (Ag), **iii)** molybdenum (Mo), **iv)** tungsten (W), **v)** vanadium (V), **vi)** ruthenium (Ru) and **xi)** silicon (Si); in particular, the metal derivative(s) b) is (are) chosen from the compounds of formula $ZAu(Hal)_4$, such as $KAuCl_4$; $Au(R)_3$ $Au_2O_3$; $Z_2MoO_4$; $Z_2WO_4$; $[XY_uMo_{12-u}O_{40}]^{q-}(Z)_q$ and $H_5(PV_2Mo_{10}O_{40})$, $(Hal)_2RuL_4$ as defined in the preceding claim; magnesium chlorophyllin; $KRuO_4$, $H_4SiW_{12}O_{40}$ and $Ag_2O$.

**10.** Dyeing method according to any one of the preceding claims, in which the metal derivative(s) b) is (are) of gold; in particular, the metal derivative(s) b) is (are) of gold (Au), with the exception of gold salts.

**11.** Dyeing method according to any one of the preceding claims, in which the basifying agent(s) **c)** is (are) chosen from alkanolamines and alkali metal or alkaline earth metal (bi)carbonates.

**12.** Dyeing method according to any one of the preceding claims, which does not employ a chemical oxidizing agent other than atmospheric oxygen.

**13.** Dyeing method according to any one of Claims 1 to 11, which employs one or more chemical oxidizing agent(s), such as:

a) ozone;

b) alkali metal or quaternary ammonium persalts;

c) aliphatic $C_1$-$C_6$ and aromatic $C_6$-$C_{20}$ organic peracids and their percarboxylate forms, such as performic acid, peracetic acid, perbenzoic acid derivatives, pertrifluoroacetic acid, peroxyphthalic acid, peroxymaleic acid or peroxypropionic acid;

d) organic peroxides;

e) oxidizing anions, such as nitrites, nitrates, hypochlorites, hypobromites, hypoiodites, chlorites, bromites,

iodites, chlorates, bromates, iodates or periodates; the oxidizing agent is chosen in particular from an alkali metal hypochlorite or periodate, such as sodium hypochlorite or sodium periodate;
f) stable N-oxy (NO·) radicals, such as the 2,2,6,6-tetra($C_1$-$C_6$)alkylpiperidino-oxy radical, 2,2,6,6-tetra($C_1$-$C_6$)alkylmorpholino-oxy radical, Fremy nitrosodisulfonate salts or morpholine N-oxide; in particular, the oxidizing agent is chosen from the 2,2,6,6-tetramethylpiperidinyloxy radical;
g) hypervalent iodine derivatives, such as iodotriacetate, iodosobenzene, iodobenzenetriacetate, iodoperbenzoic acid derivatives, periodinanes, and alkyl and benzoyl hypoiodites;
h) the following organic compounds: N-halosuccinimides, trichloroisocyanuric acid, N-hydroxyphthalimide and alkyl nitrites;
i) hydrogen peroxide or system(s) which generate hydrogen peroxide,
such as:

i-1) urea hydrogen peroxide;
i-2) polymer complexes which can release hydrogen peroxide, such as polyvinylpyrrolidone/$H_2O_2$;
i-3) oxidases which produce hydrogen peroxide in the presence of a suitable substrate (for example glucose in the case of glucose oxidase or uric acid with uricase);
i-4) metal peroxides which generate hydrogen peroxide in water, such as calcium peroxide or magnesium peroxide;
i-5) perborates; or
i-6) percarbonates.

**14.** Method according to any one of the preceding claims, in one stage, which consists in applying, to the keratinous fibres, an aqueous composition comprising **a), b)** and **c)** as defined in any one of the preceding claims and optionally one or more chemical oxidizing agent(s) as defined in the preceding claim.

**15.** Dyeing method according to any one of Claims 1 to 11, in two stages, which consists, in the first stage, in applying, to the keratinous fibres, a composition comprising the ingredients **a)** and **b)** as defined in any one of Claims 1 to 10 and optionally one or more chemical oxidizing agent(s) as defined in Claim 14 and then, in a second stage, in applying a composition comprising **c)** as defined in Claims 1 or 11, in particular at a pH of greater than 7 and preferably of between 8 and 12.

**16.** Dyeing method according to any one of Claims 1 to 11 and 13 carried out in at least two stages which terminates in the treatment of the keratinous fibres with the ingredient **c)** as defined in Claims 1 or 11 and can be followed by post-treatment stages, such as a shampooing using a conventional shampoo, a rinsing operation, for example with water, and/or the drying of the keratinous fibres by heat treatment.

**17.** Dyeing method according to any one of the preceding claims employing one or more photoirradiation(s) by one or more electromagnetic wave(s) with a wavelength of between 10 nm in the UV region and 100 $\mu$m in the infrared IR region which originate in particular from artificial photoirradiation, in particular from lamps emitting in the UV region, incandescent, halogen, fluorescent, mercury or low-pressure lamps, low-pressure lamps, for example sodium or neon lamps, high-pressure lamps, for example mercury lamps, halide lamps, flash lamps, for example xenon flash lamps, fluorescent excimer lamps, such as xenon fluorescent excimer lamps, Light Emitting Diodes or LEDs of 50 to 1000 mW, lamps emitting black light or Wood's light, and lasers.

**18.** Cosmetic composition for the dyeing of keratinous fibres, comprising:

**a)** one or more ortho-diphenol derivative(s) as defined in any one of Claims 1 to 7;
**b)** one or more metal derivative (s) as defined in any one of Claims 1 and 8 to 10;
**c)** one or more basifying agent (s) as defined in either one of Claims 1 or 11; and optionally
**d)** one or more chemical oxidizing agent(s) as defined in Claim 13.

**19.** Composition according to the preceding claim, in which the metal derivative(s) b) is (are) of gold (Au) as defined in any one of Claims 1 and 8 to 10 and optionally one or more chemical oxidizing agent(s) as defined in Claim 13.

**20.** Use, for the dyeing of keratinous fibres, such as the hair, of the following combination:

**a)** one or more ortho-diphenol derivative(s) as defined in any one of Claims 1 to 7;
**b)** one or more metal derivative (s) as defined in any one of Claims 1 and 8 to 10;

**c)** one or more basifying agent (s) as defined in either one of Claims 1 or 11; and optionally
**d)** one or more chemical oxidizing agent(s) as defined in Claim 13.

**21.** Use, for the dyeing of keratinous fibres, such as the hair, according to the preceding claim, **characterized in that** b) the metal derivative(s) is (are) one or more gold derivative (s) as defined in any one of Claims 1 and 8 to 10.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2814943
- FR 2814945
- FR 2814946
- FR 2814947
- EP 0664114 A
- US 3931249 A
- WO 1995000625 A
- US 4412934 E
- US 5008093 A
- US 3376110 A
- US 5183901 A
- FR 2586913


### Littérature non-brevet citée dans la description


- Metal complex dyes. Ullmann's Encyclopedia of Industrial Chemistry. 2005, 1-42
- **H. ZOLLINGER.** Color Chemistry. Wiley-VCH, 2003, 123-160
- **H. ZOLLINGER.** Color Chemistry. Wiley-VCH, 2003, 140
- Phtalocyanines. Ullmann's Encyclopedia of Industrial Chemistry. 2005, 1-34
- Ullmann's Encyclopedia. Wiley-VCH Verlag GmbH & Co, 2005, 1-42
- Ullmann's Encyclopedia. Wiley-VCH Verlag GmbH & Co, 2005, 27
- Molybdenium compounds. **EDWARD I. STIEFFEL.** Kirk-Othmer Encyclopedia of Chemical Technology Copyright©. John Wiley & Sons, Inc, 2001, 871-895
- Molybdenum and Molybdenum Compounds. Ullmann's Encyclopedia. WILEY-VCH Verlag GmbH & Co. KGaA, 2000
- **C. B. KNOBLER et al.** *J. Chem. Soc. Dalton Trans.,* 1983, 1299
- **F. A. SCHRODER ; J. SCHERLE ; Z. NATURFORSCH.** *B: Anorg. Chem. Org. Chem.,* 1973, vol. 28B, 46
- **C. B. KNOBLER ; B.R. PENFOLD ; G. T. WILKINS.** *J. Chem. Soc. Dalton Trans.,* 1980, vol. 248
- **V. K. RUDENKO.** *Koord. Khim.,* 1979, vol. 5, 307
- *Sov. J. Coord. Chem. (Engl. Transl.),* 1979, vol. 5, 231
- **M. GREENBLAT.** *Chem. Rev.,* 1988, vol. 88, 31
- **T. MA ; K. INOUE ; E. ABE ; J. YU ; X. WANG ; B. ZHANG.** *J Electroanal. Chem.,* 2002, vol. 537, 31
- Ullmann's Encyclopedia. Wiley-VCH Verlag GmbH & Co, KgA, 2005, 1-42
- Ullmann's Encyclopedia. Wiley-VCH Verlag GmbH & Co, KgA, 2005, 27
- Titanium, Titanium Alloys, and Titanium Compounds. Ullmann's encyclopedia. Wiley-VCH Verlag GmbH & Co. KGaA, 2005, 1-33
- Tin, Tin Alloys, and Tin Compounds. Ullmann's encyclopedia. Wiley-VCH Verlag GmbH & Co. KGaA, 2005, 27-29
- Zirconium and Zirconium Compounds. Ullmann's encyclopedia. Wiley-VCH Verlag GmbH & Co. KGaA, 2005, 15-18
- Niobium and Niobium Compounds. Ullmann's encyclopedia. Wiley-VCH Verlag GmbH & Co. KGaA, 2005, 5-6
- Indium and Indium Compounds. Ullmann's encyclopedia. Wiley-VCH Verlag GmbH & Co. KGaA, 2005, 7
- Ultraviolet and Visible Spectroscopy. Ullmann's Encyclopedia. Wiley-VCH Verlag GmbH & Co. KGaA, 2008
- Lamps. Ullmann's Encyclopedia. Wiley-VCH Verlag GmbH & Co. KGaA, 2005
- Photochemistry. Ullmann's Encyclopedia. Wiley-VCH Verlag GmbH & Co. KGaA, 2005